# EUROPÄISCHE PATENTANMELDUNG

(11) **EP 4 295 879 A1**
(43) Veröffentlichungstag der Anmeldung: **27.12.2023**
(21) Anmeldenummer: 22180071.7
(22) Anmeldetag: 21.06.2022
(51) Int. Cl.: A61M 15/08, A61M 11/00, A61M 15/00, A61M 5/00, A24F 40/05, A24F 40/10, A24F 40/48

(54) **NASENAPPLIKATOR**

(71) Anmelder: HATCHMORE Labs GmbH, 82031 Grünwald (DE)
(72) Erfinder: Sinner, Klaus, 80801 München (DE); Souren, Francois Henri Mathieu Marie, 81541 München (DE)
(74) Vertreter: Bobbert & Partner Patentanwälte PartmbB

(57) **Zusammenfassung**

Die vorliegende Erfindung betrifft einen Nasenapplikator (100) zum nasalen Verabreichen wenigstens einer medizinischen Substanz (S), insbesondere eines Schmerzmittels, mit einem Gehäuse (2), aufweisend oder verbunden jeweils mit: einem Substanzreservoir (R) zum Vorhalten einer Menge der Substanz (S); einer Abrufvorrichtung (1) zum Betätigen durch den Benutzer mit dem Ziel, eine nächste Applikationsdosis (Dₙ) der Substanz (S) abzurufen; einem Abgabedosierer (3) zum Abgeben von Applikationsdosen (D₀, D₁, D₂, ..., Dₙ₋₁, Dₙ, Dₙ₊₁, ...) auf eine Betätigung der Abrufvorrichtung (1) hin zu jeweils einem Abgabezeitpunkt (tA₀, tA₁, tA₂, ..., tAₙ₋₁, tAₙ, tAₙ₊₁, ...); einer Verbindungsstelle (4a) für einen Nasenaufsatz (4), oder einen Nasenaufsatz (4) oder ein Nasenstück; und einer Ausbringvorrichtung (17) zum Ausbringen einer Applikationsdosis der Substanz (S) über die Verbindungsstelle (4a) oder den Nasenaufsatz (4) oder das Nasenstück und/oder aus dem Nasenapplikator (100) hinaus.

## Beschreibung

Die vorliegende Erfindung betrifft eine Medikamentenabgabevorrichtung, insbesondere einen Nasenapplikator, gemäß Anspruch 1. Sie betrifft ferner ein System wie hierin offenbart sowie ein System gemäß Anspruch 13.

Aus der Praxis sind Nasenapplikatoren bekannt, mittels welcher sich ein Patient ein Medikament oder einen Wirkstoff nasal applizieren kann.

Es ist Aufgabe der vorliegenden Erfindung, einen weiteren Nasenapplikator vorzuschlagen.

Die erfindungsgemäße Aufgabe wird durch den Nasenapplikator mit den Merkmalen des Anspruchs 1 gelöst, ferner durch die hierin offenbarten Systeme, welche ebenfalls Teil der Erfindung sind, beispielsweise mit dem System mit den Merkmalen des Anspruchs 13.

Erfindungsgemäß wird eine Medikamentenabgabevorrichtung (für die nasale, inhalative, intravenöse, orale, bukkale, sublinguale, subkutan, intramuskulär, usw. Abgabe), insbesondere ein Nasenapplikator zum Verabreichen bzw. nasalen Verabreichen wenigstens einer Substanz, z. B. einer medizinischen oder nicht-medizinischen Substanz, z. B. ein medizinischer oder nicht-medizinischer Wirkstoff, vorgeschlagen. Die medizinische Substanz kann insbesondere ein Schmerzmittel oder ein Impfstoff oder Phagen sein. Hierzu weist die Medikamentenabgabevorrichtung bzw. der Nasenapplikator ein Gehäuse auf.

Die Medikamentenabgabevorrichtung weist optional eine Verbindungsstelle für ein Substanzreservoir zum Vorhalten einer Menge der Substanz auf, oder sie weist ein Substanzreservoir zum Vorhalten einer Menge der Substanz auf oder ist mit einer/einem solchen verbunden.

Die Medikamentenabgabevorrichtung ist mit einer Abrufvorrichtung zum Betätigen durch den Benutzer/Patienten verbunden oder weist eine solche auf. Die Abrufvorrichtung ist programmiert oder anderweitig vorbereitet, damit der Benutzer der Medikamentenabgabevorrichtung mittels Betätigens der Abrufvorrichtung eine nächste Applikationsdosis der Substanz abrufen kann. Zweck oder Ziel der Abrufvorrichtung ist somit, eine Applikationsdosis auslösen zu können.

Die Medikamentenabgabevorrichtung weist weiter einen Abgabedosierer auf oder ist mit einem solchen verbunden. Der Abgabedosierer ist vorgesehen zum Abgeben von Applikationsdosen auf eine Betätigung der Abrufvorrichtung hin. Die Abgabe erfolgt, wenn sie erfolgt, jeweils zu einem sogenannten Abgabezeitpunkt.

Die Medikamentenabgabevorrichtung weist optional, z. B. alternativ oder ergänzend zur Verbindungsstelle für ein Substanzreservoir und/oder zur nachstehend genannten Verbindungsstelle für eine unter Druck stehende Gaskartusche, eine Verbindungsstelle für einen Nasenaufsatz, einen Nasenaufsatz oder ein Nasenstück auf oder ist mit einer Verbindungsstelle für einen Nasenaufsatz, einem Nasenaufsatz oder einem Nasenstück verbunden.

Weiter weist die Medikamentenabgabevorrichtung optional, z. B. alternativ oder ergänzend zu wenigstens einer der vorstehend genannten Verbindungsstellen eine Verbindungsstelle für eine unter Druck stehende Gaskartusche auf, oder ist mit einer unter Druck stehenden Gaskartusche verbunden. Die Gaskartusche kann optional zum Austreiben von Substanz z. B. aus der Dosierkammer oder dem nachstehend diskutierten Nasenaufsatz-Reservoir dienen. Die Gaskartusche kann mit Gas, Luft, Edelgas oder dergleichen gefüllt sein. In ihr kann ein Druck oberhalb des Atmosphärendrucks herrschen.

Schließlich weist die Medikamentenabgabevorrichtung eine Ausbringvorrichtung zum aktiven Ausbringen einer Applikationsdosis der Substanz über die Verbindungsstelle für den Nasenaufsatz oder den Nasenaufsatz oder das Nasenstück und/oder aus dem Nasenapplikator hinaus, auf oder ist mit einer solchen verbunden.

Ein erfindungsgemäßes System umfasst optional einen oder mehrere erfindungsgemäße Nasenapplikatoren sowie ein oder mehrere Peripheriegeräte, wobei einer oder mehrere der Nasenapplikatoren, insbesondere mittels seiner Steuervorrichtung, mit einem oder mehreren der Peripheriegeräte in Signal- oder Kommunikationsverbindung stehen oder hierzu vorbereitet oder programmiert sind.

Ein weiteres erfindungsgemäßes System umfasst optional einen oder mehrere erfindungsgemäße Nasenapplikatoren und einen oder mehrere der hierin offenbarten Einweg-Nasenaufsätze als Nasenaufsätze. Die Einweg-Nasenaufsätze können optional mit Substanz bestückt sein.

Erfindungsgemäß wird ferner ein System vorgeschlagen, welches wenigstens einen erfindungsgemäßen Nasenapplikator und weiter wenigstens eine Einweg-Schutzhülle zum Aufsetzen auf den Nasenaufsatz aufweist.

Wenn hierin von einem Benutzer die Rede ist, so umfasst dies den Patienten, der sich selbst mediziert, den Pfleger, die Ärztin, Angehörige von Patienten, und mehr, der z. B. auf Geheiß des Patienten die Medikamentenabgabevorrichtung wie hierin beschrieben bei der Medikation des Patienten verwendet.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert", "vorgesehen" oder "konfiguriert" die Rede ist, so ist auch offenbart, diese Begriffe gegeneinander auszutauschen.

Wenn hierin von "programmiert", "vorgesehen" oder "konfiguriert" zum Ausführen eines Schritts oder einer Handlung die Rede ist, so ist auch offenbart, dass dieser Schritt oder diese Handlung optional automatisch vorgenommen wird, z. B. von der Steuervorrichtung.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Erfindungsgemäße Ausführungsformen können eines oder mehrere der oben und/oder im Folgenden genannten Merkmale in jeder technisch möglichen Kombination aufweisen.

Wann immer hierin eine Eignung, ein Zweck, ein Schritt oder ein Verfahrensschritt genannt ist, umfasst die vorliegende Erfindung auch eine entsprechende Programmierung oder Konfiguration einer zum Erzielen oder Ausführen geeigneten Vorrichtung oder eines Abschnitts hiervon.

Auch wenn die vorliegende Erfindung nicht auf einen Nasenapplikator beschränkt ist, sondern Medikamentenabgabevorrichtungen als solche betrifft, wird im Folgenden schwerpunktmäßig auf Nasenapplikatoren eingegangen. Zum Nasenapplikator gemachte Ausführen, Erläuterungen und Vorteile treffen ungeschmälert auch auf Medikamentenabgabevorrichtungen zu, und umgekehrt. In manchen Ausführungsformen können die Begriffe "Medikamentenabgabevorrichtung" und "Nasenapplikator" synonym sein. In einigen Ausführungsformen sind diese Begriffe gegeneinander austauschbar. Was hierin für die "Medikamentenabgabevorrichtung" ausgeführt ist, gilt somit auch für den "Nasenapplikator", und umgekehrt.

Der Applikator kann optional für verschiedene Applikationsformen z. B. sublingual, inhalativ und sogar intravenös verwendbar sein. Die hierin offenbarte Verbindungsstelle kann ausgestaltet sein, um mit verschiedensten Anschlüssen verbunden zu werden, durch welche eine Flüssigkeit befördert werden kann, wie z. B einem Impaction Baffle oder Stufenbaffle oder Impaktionsbaffle, welche die Flüssigkeit in kleine Tröpfchen aufbricht, einem Zweistrahlzerstäuber (impinging jet), einer porösen Membran, einer Lochplatte (Rayleigh breakup Prinzip), einem Infusionsschlauch, einer Wirbelkammer, einer Nasenbrille, anderweitigen Nasenadaptern zur Erzeugung kleiner Tröpfchen, usw. Sie kann hiermit jeweils verbunden sein.

In manchen Ausführungsformen weist der Nasenapplikator weiter eine Dosierkammer auf. Die Dosierkammer dient zum vorübergehenden Aufnehmen der Applikationsdosis der im Substanzreservoir vorgehaltenen Substanz oder angesaugter Luft, etwa der Umgebung entnommen. Die Dosierkammer kann eine Luftkammer sein.

In einigen Ausführungsformen weist der Nasenapplikator ergänzend eine Bestückungsvorrichtung zum Bestücken der Dosierkammer mit der Applikationsdosis der Substanz aus dem Substanzreservoir oder mit Luft, beispielsweise durch Ansaugen, auf.

In manchen Ausführungsformen ist die Bestückungsvorrichtung eine Pumpe.

In einigen Ausführungsformen ist die Bestückungsvorrichtung eine Membran, die über die Zeit Flüssigkeit in die Dosierkammer diffundieren lässt.

In anderen Ausführungsformen ist die Bestückungsvorrichtung ein Tropfer, alternativ ein Faltenbalg, ein Balg oder eine Manschette, ein Bolussystem, oder dergleichen.

In einigen Ausführungsformen kann die Konzentration selbst angemischt werden, um unterschiedliche Dosierungen zu simulieren. Mit anderen Worten: In einer Dosierkammer von konstanter und/oder variabler Größe (z. B. 100 µL) werden unterschiedliche Mischungen von Trägerflüssigkeit und Wirkstoff bereitgestellt, um die gewünschte Konzentration, also z. B. 50 µg/100 µL oder 60 µg/100 µL, etc., zu erhalten. Hierzu, und optional ergänzend oder alternativ zu anderen Zwecken, kann eine ergänzend zum Substanzreservoir eine zweite Fluidkammer vorgesehen sein, welche ein zweites Fluid (vorzugsweise eine Flüssigkeit) welches sich von der Substanz des Reservoirs unterscheidet, aufweist oder zu seiner Aufnahme vorgesehen ist, etwa ein Fluid zum Verdünnen der Substanz mit dem Ziel, eine gewünschte Konzentration einzustellen.

Eine Pumpe, oder weitere Pumpe, zum Ausbringen des zweiten Fluids aus der zweiten Fluidkammer kann, wie auch jede andere jegliche Bestückungsvorrichtung wie z. B. eine Spritze, vorgesehen sein.

Die zweite Fluidkammer, oder eine weitere, dritte, kann ein Trocknungsmittel aufweisen oder hierfür vorgesehen sein.

Anstelle oder ergänzend zum Trocknungsmittel kann ein Antidot für den oder einen Wirkstoff der Substanz vorgesehen sein, ein Neutralisierungsmittel, um die pharmakologische Wirkung zu mindern oder zu blockieren.

In einigen Ausführungsformen ist die Ausbringvorrichtung angeordnet zum, insbesondere aktiven, Ausbringen von der in der Dosierkammer, im Substanzreservoir oder im Nasenaufsatz-Reservoir vorliegenden Applikationsdosis der Substanz über die Verbindungsstelle, den Nasenaufsatz, den Einweg-Nasenaufsatz oder das Nasenstück und/oder aus dem Nasenapplikator hinaus.

In manchen Ausführungsformen ist die Ausbringvorrichtung angeordnet zum, insbesondere aktiven, Ausbringen von in der Dosierkammer vorliegendem Gas oder komprimiertem Gas über die Verbindungsstelle, den Nasenaufsatz, den Einweg-Nasenaufsatz oder das Nasenstück und/oder aus dem Nasenapplikator hinaus.

In einigen Ausführungsformen ist vorgesehen, statt einer Gaskartusche einen Flüssigkeitsbehälter mit z. B. Kochsalzlösung, insbesondere zum hierin für die Gaskartusche offenbarten Zweck, oder eine Verbindungsstelle hierfür, vorzugsehen.

In bestimmten Ausführungsformen ist der Nasenaufsatz ein Einweg-Nasenaufsatz.

In manchen Ausführungsformen kann bei einem motorgetriebenen direkten Kolbenantrieb, also bei einem System, bei welchem die Wirbelkammer direkt mit einer Spritze und/oder Zylinderampullenspritze (Karpule), die hier als Reservoir und Dosierkammer fungiert, gekoppelt ist - z. B. mittels eines Motors oder auf andere Weise - die benötigte Auslösegeschwindigkeit, -kraft und/oder Druck erzeugt werden, indem Druck auf die Druckplatte der Spritze aufgebracht wird. Die Abgabemenge kann mittels eines Durchflusssensors ermittelt und gesteuert werden. Der Durchflusssensor ist vorgesehen, um in diesen Ausführungsformen die Bewegung der Ausbringvorrichtung zu beenden, beispielsweise um den optionalen Motor anzuhalten, wenn die gewünschte Abgabemenge erreicht wurde.

In manchen Ausführungsformen kann optional ein Tonsignal abgegeben werden, während und/oder solange ein Fluid ausgebracht wird. Alternativ oder ergänzend kann in einigen Ausführungsformen ein Tonsignal nach abgeschlossener Applikation erfolgen. Von der vorliegenden Erfindung sind sowohl verschiedene Tonsignale als auch verschiedene Tonarten und -höhen umfasst.

In manchen Ausführungsformen erfolgt die Dosiseinstellung und/oder die Auslösung manuell, oder als eine Kombination aus manuell und automatisch.

In manchen Ausführungsformen weist die Ausbringvorrichtung und/oder die Bestückungsvorrichtung einen Energiespeicher für mechanische oder elektrische Energie oder Federenergie auf.

In bestimmten Ausführungsformen weist die Ausbringvorrichtung und/oder die Bestückungsvorrichtung einen Energiespeicher für hydraulische und/oder pneumatische Energie auf.

In einigen Ausführungsformen ist die gespeicherte Energie durch den menschlichen Körper oder durch die Schwerkraft erzeugte Energie. Die Speicherung der Energie erfolgt in diesen Ausführungsformen mittels Änderung der Dichte oder mittels Umwandlung dieser Energie in eine andere Energieform.

In manchen Ausführungsformen weisen die Ausbringvorrichtung und/oder die Bestückungsvorrichtung einen Motor, vorzugsweise einen Elektromotor, auf.

In einigen Ausführungsformen kann der Motor auch mit Druckluft (pneumatisch, hydraulisch) oder per mechanischer Energie, beispielsweise Federenergie und/oder menschlich aufgebrachter Energie angetrieben werden. Der Motor kann auch eine Pumpe sein.

In manchen Ausführungsformen weist die Ausbringvorrichtung und/oder die Bestückungsvorrichtung eine Spindel auf.

Die Spindel kann elektrisch, pneumatisch, hydraulisch, mechanisch oder mittels Federenergie angetrieben sein.

Die Spindel kann unterschiedlich ausgelegt sein, z. B. als Linearsystem (Linearachse) mit, Zahnriemen- und Spindelantrieb für eine Linearbewegung, als Kugelbuchsen, als Gewindespindel (Kugelgewinde oder Trapezgewindegetriebe), als Wellenführung, als Nutwellen, als zylindrische, Flansch-Rotations-, Kurz-Hub-, Lang-Hub-Drehmomentkugelbuchsen für eine Drehmomentübertragung, als Drehmomentwellen mit und ohne Nut, als Schienenführung, als Elektro-, Pneumatik-, oder Hydraulikzylinder, als Teleskopzylinder (z. B. mehrstufiger Hydraulikkolben) oder als einfach oder doppelt wirkender Zylinder.

Die Spindel kann in manchen Ausführungsformen glatt sein und z. B. mittels zwei Paar schräg gelagerter und gegenüberliegender Kugellager angetrieben werden. Es kann ein Rollringgetriebe (Uhing-Getriebe) vorgesehen sein.

In bestimmten Ausführungsformen weist der Nasenapplikator kein Federelement und/oder keinen Speicher für mechanische Energie oder Federenergie auf.

In bestimmten Ausführungsformen weist die Bestückungsvorrichtung und/oder die Ausbringvorrichtung kein Federelement und/oder keinen Speicher für mechanische Energie oder Federenergie auf.

In einigen Ausführungsformen ist der Motor mit der Spindel verbunden.

In diesen Ausführungsformen ist der Motor mit der Spindel direkt verbunden. Alternativ sind Motor und Spindel indirekt verbunden, d. h. es kann eine Übersetzung oder Untersetzung zwischengeschaltet sein.

Motor und Spindel können gemeinsam als Getriebe bezeichnet werden. Es kann in manchen Ausführungsformen ein festes Getriebe sein, d. h. Drehzahlverhältnis und Drehmomentwandlung sind nicht veränderbar. In anderen Ausführungsformen kann das Getriebe ein Verstellgetriebe (gestuft und/oder stufenlos) sein. Das Getriebe kann in weiteren Ausführungsformen ein Schaltgetriebe zur Drehzahl- und/oder Drehmomentabstufung und/oder Drehrichtungsumkehr sein.

Innerhalb des Getriebes kann die Über- bzw. Untersetzung formschlüssig oder kraftschlüssig sein wie z. B. bei einem Zahnrad- und/oder Reibradgetriebe und/oder Umlaufrädergetriebe (alle drei Rädergetriebe), einem Ketten- und/oder Riemengetriebe (beides Zugmittelgetriebe), einem Planetengetriebe, einem Druckmittelgetriebe (z. B. wie beim hydraulischen Getriebe einer Autobremse), einem Gelenkgetriebe (z. B. wie beim Schubkurbelgetriebe eines Verbrennungsmotors - Kurbelwelle und Pleuel), einem Kurvengetriebe (z. B. wie beim Nockengetriebe einer Ventilsteuerung), einem Schrauben- und Schneckengetriebe (z. B. wie bei einem Schraubstock oder Wagenheber), einem Schubkettengetriebe, einem Walzkörpergetriebe, einem Leistungsteilungsgetriebe (z. B. einem Differentialgetriebe), einem Lamellenkettengetriebe, einem Rollringgetriebe, einem Koppelgetriebe (z. B. Kurbelgetriebe, Malteser-Kreuz-Getriebe) oder einem Getriebe mit einem elastischen Übertragungselement (z. B. Gleitkeilgetriebe).

In einigen Ausführungsformen weist der Nasenaufsatz des erfindungsgemäßen Nasenapplikators ein Nasenaufsatz-Reservoir zum Aufnehmen oder Vorhalten einer Menge der Substanz auf oder ist ein solches Nasenaufsatz-Reservoir.

In manchen Ausführungsformen ist die Ausbringvorrichtung angeordnet zum Ausbringen von der in der Dosierkammer oder im Substanzreservoir oder in dem Nasenaufsatz vorliegenden Applikationsdosis der Substanz über die Verbindungsstelle, den Nasenaufsatz oder das Nasenstück und/oder aus dem Nasenapplikator hinaus.

In manchen Ausführungsformen enthält das Substanzreservoir oder das Nasenaufsatz-Reservoir eine Substanz, vorzugsweise einen Impfstoff oder Phagen.

In einigen Ausführungsformen ist der Impfstoff ein DNA-, mRNA- oder Vektorimpfstoff, und/oder ein SARS-CoV-2- oder Influenza-Impfstoff.

In manchen Ausführungsformen ist der Impfstoff ein replizierender Impfstoff oder enthält Phagen.

In einigen Ausführungsformen ist der Impfstoff ein Lebendimpfstoff oder ein Totimpfstoff.

In manchen Ausführungsformen weist der Nasenapplikator weiter einen Mechanismus zum Verändern des Fassungsvolumens der Dosierkammer für die Applikationsdosis der Substanz auf. Das Verändern des Fassungsvolumens kann manuell oder automatisch erfolgen.

Eine solche Veränderung des Fassungsvolumens kann mittels Veränderns des Hubwegs der Bestückungs- bzw. Ausbringvorrichtung erfolgen. Beispiele hierfür sind: Verlängern/Verkürzen des Verfahrwegs der Bestückungs- bzw. Ausbringvorrichtung, Verdrehung einer schiefen Ebene, mittels einer Kurve z. B. eine Nocke, Nut, Herzkurve, usw., ein verstellbarer Endstop/Anschlag, verstellbare Pins als Wegbegrenzer, eine Veränderung des Dosierkammerdurchmessers, einer Veränderung der Bestückungs- bzw. Ausbringvorrichtungsgeometrie (z. B. rund zu oval), eine Veränderung der Dosierkammergeometrie (z. B. rund zu oval) und/oder nach außen oder nach innen laufende Teleskopkolben.

In manchen Ausführungsformen kann vorgesehen sein, das Fassungsvolumen der Dosierkammer zu verändern, indem vorgesehen ist, den Kolben zunächst mittels Drehung der Spindel in eine erste Richtung (etwa gegen den Uhrzeigersinn) zu bewegen und die Dosierkammer entsprechend zu vergrößern, um Substanz aus dem Reservoir anzusaugen. Zum Ausbringen der so in die Dosierkammer eingebrachten Applikationsdosis wird die Spindel anschließend gegenläufig rotiert.

In bestimmten Ausführungsformen kann vorgesehen sein, das Befüllen und das Entleeren der Dosierkammer mittels eines Schubkurbelantriebs zu bewirken.

Der Schubkurbelantriebs kann eine Exzenterwelle und eine Schubstange, die auf oder an der Exzenterwelle relativ zu dieser drehbar, aufweisen oder hieraus bestehen.

Der Schubkurbelantriebs dient dazu, eine Drehbewegung in eine oszillierende Schubbewegung, oder umgekehrt, umzuformen. Bezogen auf die hier relevante Ausgestaltung der Bestückungs- bzw. Ausbringvorrichtungsvorrichtung kann sich hierbei ein Kolbenmotor ergeben mit einem Kolben, der sich im Zylinder geradlinig hin und her bewegt.

Die Steuervorrichtung kann programmiert sein, um mittels Drehung des Motorantriebs in einer ersten Richtung sowohl die Dosierkammer zu befüllen, als auch durch ein weiteres Drehen des Motorantriebs in derselben Richtung auch wieder zu entleeren mit dem Ziel, die nächste Applikationsdosis auszubringen. Die Menge der hierbei ausgebrachten Dosis kann durch Drehen um einen entsprechenden Winkelwert festgelegt sein oder werden.

Die Steuervorrichtung kann alternativ programmiert sein, um mittels Drehung des Motorantriebs in einer ersten Richtung die Dosierkammer zu befüllen, durch ein Drehen des Motorantriebs in der zweiten, der ersten entgegengesetzten Richtung wieder zu entleeren mit dem Ziel, die nächste Applikationsdosis auszubringen. Die Menge der hierbei ausgebrachten Dosis kann wiederum durch Drehen um einen entsprechenden Winkelwert festgelegt sein oder werden, was das Einstellen der Dosis variabel macht. Der Schubkurbelantrieb kann hierbei vorteilhaft zum Einsatz kommen.

In einigen Ausführungsformen ist eine Verkleinerung der Dosierkammer von der vorliegenden Erfindung umfasst, indem nicht der Hubweg des Ausbringmechanismus verändert, sondern die Verbindungsseite verfahren wird.

Nimmt die Dosierkammer z. B. maximal ein Volumen von 150 µL auf, so kann sich folgendes Verfahren anbieten, zu welchem die Steuervorrichtung ebenfalls programmiert sein kann: Man füllt mit dem Ansaughub die Dosierkammer mit 150 µL, befüllt sie also vollkommen. Die abzugebende Applikationsdosis beträgt z. B. aber nur 50 µL welche durch Umkehr des Wegs, den z. B. der Kolben zurücklegt, abgegeben wird, was den Ausbringhub ausmacht. Die in der Dosierkammer verbleibende Restmenge beträgt in diesem Beispiel somit 100 µL. Beträgt die wiederum nächste Applikationsdosis beispielsweise 75 µL, so muss das Gerät ausnahmsweise keinen Ansaughub durchführen, sondern macht gleich den Ausbringhub. Die wiederum verbleibende Restmenge, die in der Dosierkammer auch nach dieser letzten Applikation noch immer vorliegt, beträgt 25 µL. Beträgt die nachfolgende Applikationsdosis z. B. 100 µL, so erfordert diese wieder einen Ansaughub von 75 µL. Die Dosierkammer kann alternativ jedoch wieder auf 150 µL befüllt werden.

Weiter alternativ ist von der vorliegenden Erfindung umfasst, dass eine konstant große Dosierkammer (z. B. 150 µL) stets nur mit dem gewünschten Ausbringvolumen befüllt wird (z. B. mittels Mikropumpe, beispielsweise mit 100µL. In diesen Ausführungsformen wird die Auslösevorrichtung in Richtung Verbindungsstelle bewegt, um zuerst Luft zu komprimieren. Ab einem vorbestimmten Druck öffnet das Ventil und die Luft wird zusammen mit der Flüssigkeit durch die Wirbelkammer gedrückt. Alternativ wird die komprimierte Luft über ein Entlüftungsventil abgeleitet.

In manchen Ausführungsformen ist der Mechanismus zum Verändern des Fassungsvolumens der Dosierkammer für die Applikationsdosis der Substanz Teil des Abgabedosierers.

Wenn hierin von einem Motor die Rede ist, so kann dieser als Wankelmotor ausgestaltet sein.

In einigen Ausführungsformen weist der Nasenapplikator weiter eine elektronische Steuervorrichtung auf.

In bestimmten Ausführungsformen ist die elektronische Steuervorrichtung programmiert, um zum Applizieren einer vorbestimmten Dosismenge x (z. B. 150 µL als Applikationsdosis eine Menge, die größer als x (z. B. 2x oder hier: 300 µL) ist, aus dem Substanzreservoir und/oder aus der Dosierkammer zu fördern. Mittels dieser Förderung über das therapeutische Maß hinaus kann sich ein Primen oder Entlüften der Leitung(en), entlang welcher die Applikationsdosis ausgebracht wird, vorteilhaft erübrigen.

In manchen Ausführungsformen ist die elektronische Steuervorrichtung programmiert, um die Ausbringvorrichtung mit einer Vielzahl von Geschwindigkeiten, Kräften, Drücken, Beschleunigungen und/oder Flussraten zu verfahren und/oder in einer Vielzahl von Geschwindigkeiten, Kräften, Drücken, Beschleunigungen und/oder Flussraten Substanz ausbringen zu lassen. So kann beispielsweise vorgesehen sein, mit einer ersten, insbesondere vorab festgelegter, Geschwindigkeit auszubringen, mit einer zweiten, insbesondere vorab festgelegten, Geschwindigkeit auszubringen, welche sich von der ersten Geschwindigkeit unterscheidet, und optional mit noch weiteren Geschwindigkeiten. Diese Ausbringgeschwindigkeiten können in Abhängigkeit von der auszubringenden Menge und/oder dem auszubringenden Volumen an Substanz und/oder deren Beschaffenheit festgelegt sein. In manchen Ausführungsformen weist der Nasenapplikator eine zweite Fluidkammer auf, aufweisend ein Trocknungsmittel oder ein Mittel zum Neutralisieren der Substanz.

In manchen Ausführungsformen weisen die Ausbringvorrichtung und die Bestückungsvorrichtung identische Bauelemente auf oder bestehen aus diesen.

In einigen Ausführungsformen ist die elektronische Steuervorrichtung programmiert, um auf den Mechanismus zum Verändern des Fassungsvolumens der Dosierkammer einzuwirken.

In manchen Ausführungsformen weist die Bestückungsvorrichtung zum Bestücken der Dosierkammer mit der Applikationsdosis der Substanz wenigstens ein erstes Einweg-Ventil oder Rückschlagventil auf und/oder die Dosierkammer ist durch wenigstens ein erstes Einweg-Ventil oder Rückschlagventil begrenzt.

In einigen Ausführungsformen weist die Bestückungsvorrichtung optional wenigstens ein Ventil auf, welches bei einem vorbestimmten Druckwert (z. B. 4 bar) öffnet. In anderen Worten wird die Dosierkammer mittels der Ausbringvorrichtung unter Druck gesetzt, sobald sich diese bewegt, steigt der Druck in der Dosierkammer auf den vorbestimmten Druckwert (z. B. 4 bar), öffnet das Ventil und bleibt so lange offen bis die Dosierkammer leer ist. Somit kann das Einweg-Ventil auch die Funktion eines Druckzuschaltventils aufweisen.

Alternativ oder ergänzend kann vorgesehen sein, mittels eines Druckschaltsensors oder Drucksensors ein Ventil zu öffnen bzw. zu schließen. Diese Lösung deckt sowohl die Funktion eines Rückschlagventils sowie die Funktion eines Druckzuschaltventils (alternativ: Druckschaltventil, Druckfolgeventil oder Zuschaltventil) eines anderweitigen Ventils, einer Drossel, einer Sperre für Volumenfluss, usw. ab.

In manchen Ausführungsformen ist das Ventil mechanisch schaltbar, in anderen elektrisch oder elektromagnetisch, in wieder anderen pneumatisch oder hydraulisch.

Ein mechanisch schaltbares Ventil ist beispielsweise ein Rückschlagventil mit Federelement. Weitere Ausführungsformen eines mechanisch geschalteten Ventils sind beispielsweise Teller- oder Kugelrückschlagventil, Rückschlagklappe oder Rückstauklappe.

In einigen Ausführungsformen ist das Ventil ein schaltbares Dreiwegeventil. Auch dieses kann mechanisch, elektrisch oder elektromagnetisch, pneumatisch oder hydraulisch schaltbar sein.

In manchen Ausführungsformen ist das Einweg-Ventil in der Auslöse- bzw. Bestückungsvorrichtung integriert oder teilintegriert.

In einigen Ausführungsformen wird das Einweg-Ventil ersetzt, indem der Stopfen im Substanzreservoir blockiert wird, nachdem die Dosierkammer gefüllt ist.

Das Einwegventil kann in die Wirbelkammer integriert sein, also z. B. jenseits der Verbindungsstelle, oder im Nasenaufsatz, usw.

Die Wirbelkammer kann geformt sein, die Funktion eines Ventils zu übernehmen. Die Wirbelkammer kann ein solches Ventil aufweisen oder durch dieses zu einer Seite begrenzt oder in ihrer Erstreckung definiert sein.

In manchen Ausführungsformen weist die Bestückungsvorrichtung zum Bestücken der Dosierkammer mit der Applikationsdosis der Substanz wenigstens ein zweites Ventil, z. B. ein beliebiges, wie hierin diskutiertes oder anderes Ventil, insbesondere ein Einweg-Ventil oder Rückschlagventil, auf. Alternativ oder ergänzend ist die Dosierkammer durch wenigstens ein solches, z. B. ein zweites Einweg-Ventil oder Rückschlagventil, begrenzt.

Das zweite Rückschlagventil weist dabei vorzugsweise eine bezogen auf die Dosierkammer entgegengesetzte Öffnungsrichtung auf als das erste Rückschlagventil.

In bestimmten Ausführungsformen können die Funktion des ersten Ventils und die Funktion des zweiten Ventils in einem Bauteil zusammen vorgesehen sein.

In einigen Ausführungsformen weist der Nasenapplikator weiter eine Sperrvorrichtung zum zeitweisen Sperren des Abgabedosierers während einer zu oder nach dem jeweiligen Abgabezeitpunkt der abgegebenen Applikationsdosis erneut beginnenden Sperrdauer. Die Sperrdauer hat einen Sperrdauerbeginn und ein Sperrdauerende.

In manchen Ausführungsformen weist der Nasenapplikator weiter eine Dosiserfassungsvorrichtung zum Erfassen der Höhe der mittels des Abgabedosierers zum Abgabezeitpunkt jeweils abgegebenen Applikationsdosis auf.

In einigen Ausführungsformen weist der Nasenapplikator weiter einen Datenspeicher zum Speichern zumindest der Abgabezeitpunkte einer oder mehrerer bereits abgegebener Applikationsdosen auf. Alternativ oder ergänzend werden die Höhen dieser Applikationsdosen ebenfalls gespeichert.

In bestimmten Ausführungsformen weist die Dosierkammer eine Kammer zur Aufnahme der Substanz oder der dosierten Menge der Substanz auf oder besteht aus dieser. Die Dosierkammer ist in diesen Ausführungsformen insbesondere von variabler Größe oder variablem Fassungsvolumen.

In anderen Ausführungsformen ist die Dosierkammer von nichtvariabler Größe und/oder von stets gleichem Fassungsvolumen.

In bestimmten Ausführungsformen ist der Abgabedosierer konfiguriert und angeordnet, um die abzugebende Menge an Substanz als Spray und/oder zerstäubt abzugeben.

In bestimmten Ausführungsformen weist der Nasenapplikator keinen Energiespeicher und/oder keine Mittel zur Abgabe einer vorbestimmten, insbesondere mechanischen, Energiemenge an den Energiespeicher auf.

In bestimmten Ausführungsformen weist der Nasenapplikator keine Mittel zur Freigabe der vorbestimmten Energiemenge aus dem Energiespeicher auf die Dosierkammer auf, um damit die darin befindliche Flüssigkeit einem vorbestimmten Druckanstieg von einem niedrigen Druck auf einen höheren Druck auszusetzen und eine Abgabe der Flüssigkeit aus der Dosierkammer einzuleiten.

In manchen Ausführungsformen kann die Medikamentenabgabevorrichtung ferner mit einer Sperrvorrichtung verbunden sein oder eine solche aufweisen.

Die Sperrvorrichtung ist in einigen Ausführungsformen konfiguriert zum zeitweisen Sperren des Abgabedosierers während einer, insbesondere vordefinierten, Sperrdauer. Die Sperrdauer beginnt beispielsweise nach Abgabe einer, mancher oder jeder Applikationsdosis. Es kann somit vorgesehen sein, zu oder nach dem jeweiligen Abgabezeitpunkt der abgegebenen Applikationsdosis erneut eine Sperrdauer beginnen zu lassen. Die Sperrdauer ist definiert durch ihren jeweiligen Sperrdauerbeginn und ihr jeweiliges Sperrdauerende. Von der Sperrvorrichtung angesetzte Sperrdauern können unterschiedliche Dauern aufweisen oder jeweils die gleiche Dauer haben.

Außerdem weist die Medikamentenabgabevorrichtung in einigen Ausführungsformen eine Dosiserfassungsvorrichtung auf oder ist mit einer solchen verbunden. Die Dosiserfassungsvorrichtung ist programmiert zum Erfassen der Höhe (z. B. als Menge, in Gewichtseinheit, in Volumeneinheit, in Stoffeinheit wie Mol, usw.) der Applikationsdosis, die mittels des Abgabedosierers zum jeweiligen Abgabezeitpunkt jeweils abgegeben wird. Sie kann auch programmiert sein zum Erfassen der Höhe einer hierin als Ausgangsdosis bezeichneten Applikationsdosis.

Ferner ist die Medikamentenabgabevorrichtung in einigen Ausführungsformen mit einer elektronischen Steuervorrichtung verbunden oder weist eine solche auf.

Die Medikamentenabgabevorrichtung kann einen Datenspeicher zum Speichern aufweisen oder hiermit verbunden sein. Er kann zumindest die Abgabezeitpunkte einer oder mehrerer bereits abgegebener Applikationsdosen, welche also z. B. vor dem nächsten Abgabezeitpunkt oder in der Vergangenheit liegen und/oder der Höhen dieser Applikationsdosen speichern und/oder aufweisen.

Die Medikamentenabgabevorrichtung kann eine Erfassungsvorrichtung aufweisen, oder hiermit verbunden sein, welche programmiert ist, die Betätigung der Abrufvorrichtung durch den Benutzer zu erfassen. Die Erfassungsvorrichtung kann zumindest eine zu einem Betätigungszeitpunkt erfolgte oder erfolgende Betätigung der Abrufvorrichtung durch den Benutzer als Bestätigungsverhalten erfassen und ist vorzugweise entsprechend ausgestaltet, programmiert und/oder hierzu vorgesehen. Das Betätigungsverhalten hat zum Ziel, eine nächste oder künftige Applikationsdosis auszulösen, abgeben oder applizieren zu lassen und/oder um die Umstände hierfür zu überprüfen, beispielsweise, ob ein vorausgehender Abgabezeitpunkt bereits eine ausreichend lange Zeitdauer zurückliegt. Das Betätigungsverhalten kann die Betätigung der Abrufvorrichtung sein oder hieraus durch Auswerten erfasst werden.

Die elektronische Steuervorrichtung kann programmiert sein, um im Datenspeicher gespeicherte Daten auszulesen. Sie ist ferner programmiert, um das Betätigungsverhalten des Benutzers, das mittels der Erfassungsvorrichtung erfasst wurde, auszuwerten.

Das Ergebnis der Auswertung des Betätigungsverhaltens des Benutzers mittels der elektronischen Steuervorrichtung kann in einigen Ausführungsformen Einfluss auf die Vorbestimmung der Höhe der nächsten oder künftigen Applikationsdosis haben.

Die elektronische Steuervorrichtung kann programmiert sein, um die Zeit, welche seit dem Abgabezeitpunkt einer oder mehrerer bereits abgegebenen Applikationsdosen bis zur Betätigung der Abrufvorrichtung vergangen sind, zu ermitteln und, vor allem quantitativ, beim Vorbestimmen zu berücksichtigen.

Die elektronische Steuervorrichtung kann programmiert sein, um die Zeit, welche seit dem Abgabezeitpunkt einer oder mehrerer der bereits abgegebenen Applikationsdosen bis zur Betätigung der Abrufvorrichtung vergangen sind, zu ermitteln und, vor allem quantitativ, zu berücksichtigen beim Vorbestimmen, zu einem Vorbestimmungszeitpunkt, der Höhe der nächsten oder der weiteren Applikationsdosis, welche an den Benutzer für den Fall, dass dieser außerhalb einer möglichweise bei der Betätigung der Abrufvorrichtung noch andauernden Sperrdauer die Abrufvorrichtung betätigt, zu einem nächsten Abgabezeitpunkt abgebbar ist.

In manchen Ausführungsformen sind der Nasenapplikator und/oder eine oder mehrere Vorrichtungen hiervon programmiert zum Vorbestimmen der Höhe der nächsten oder der weiteren Applikationsdosis bereits zu einem Vorbestimmungszeitpunkt, welcher während einer Sperrdauer liegt.

In einigen Ausführungsformen sind der Nasenapplikator und/oder seine Vorrichtungen nicht programmiert, um eine Zeitdauer oder einen Zeitpunkt seitens des Nutzers eingeben zu können, nach welcher/zu welchem die Wirkung oder eine vorbestimmte Wirkung der verabreichten Wirkstoffmenge beendet, abgebaut oder unterschritten sein soll.

In manchen Ausführungsformen sind der Nasenapplikator und/oder seine Vorrichtungen nicht programmiert zum Kompensieren oder Verändern der vorbestimmten Höhe der nächsten oder der weiteren Applikationsdosis mittels eines gespeicherten und/oder abgerufenen Kompensationsfaktors für die Höhe.

In einigen Ausführungsformen ist der Nasenapplikator nicht ausgestaltet oder konfiguriert, um eine Applikationsdosis zu verdampfen oder zu vaporisieren.

In einigen Ausführungsformen ist die Substanz im Reservoir dauerhaft druckbeaufschlagt, etwa mittels einer Feder, welche angeordnet ist, um auf das Innenvolumen des Reservoirs komprimierend einzuwirken.

Die Dosierkammer kann ausgestaltet sein, um bei ihrem Bestücken mit der auszubringenden, nächsten oder weiteren Applikationsdosis in das zum Fassen dieser Dosis erforderliche Volumen durch eine entsprechende Bewegung mittels z. B. des Motors, der Spindel und/oder der Bestückungsvorrichtung gebracht zu werden. Sie kann anschließend, zum Ausbringen der Dosis, verringert werden.

Die Dosierkammer hat in manchen Ausführungsformen eine variable Größe oder ein variables Fassungsvolumen. Diese/dieses wird vorzugsweise automatisch vor oder zu ihrer Bestückung mit der nächsten oder weiteren Dosis mittels Motors, Spindel und/oder Bestückungsvorrichtung eingestellt.

Die Steuervorrichtung ist in einigen Ausführungsformen programmiert, um mittels Motors, Spindel und/oder Bestückungsvorrichtung, das Volumen der Dosierkammer zu ihrem Befüllen zunächst zu vergrößern und anschließend zu ihrem Entleeren wieder zu verringern. Hierzu drehen Motor und/oder Spindel vorzugsweise sowohl in einer ersten als auch später in einer zweiten, der ersten entgegengesetzten Richtung.

In manchen Ausführungsformen ist die Dosierkammer begrenzt durch Innenwandabschnitte eines Kolbens, eines beweglichen Stempels und/oder durch zwei Ventile. In einigen Ausführungsformen wird die Dosierkammer hierdurch ausgebildet.

In einigen Ausführungsformen sind eines oder mehrere der Ventile, welche die Dosierkammer begrenzen, Rückschlagventile oder andere mechanisch betätigte Ventile.

In manchen Ausführungsformen sind die Ventile, welche die Dosierkammer begrenzen, ortsfest angeordnet, was ihre Präzision erhöhen kann. Ortsfest bedeutet hier, dass sie nicht mit z. B. der Bewegung des Kolbens verfahrbar sind.

In bestimmten Ausführungsformen sind die Ventile, welche die Dosierkammer begrenzen, an einem gemeinsamen Ende der Dosierkammer angeordnet oder dieser zugeordnet, z. B. beide in einem oberen Bereich, oder beide dem Nasenaufsatz zugeordnet.

In einigen Ausführungsformen ist die Steuervorrichtung programmiert, um die Bestückungsvorrichtung nach Betätigung der Abrufvorrichtung mit dem Ziel, die nächste oder weitere Applikationsdosis zu verabreichen, sowohl in eine erste Richtung als anschließend auch in eine zweite, der ersten Richtung entgegengesetzte Richtung zu verfahren.

In manchen Ausführungsformen weist der Nasenapplikator keine Kolbenpumpe auf.

In einigen Ausführungsformen weist der Nasenapplikator keine Vorrichtung zum Begrenzen des maximal möglichen Hubwegs einer Kolbenpumpe auf.

In manchen Ausführungsformen weist der Nasenapplikator keine elektrische Ventilbetätigungsvorrichtung auf.

Weiter kann sie programmiert sein, um, z. B. während des Gebrauchs des Nasenapplikators und/oder vorzugsweise ohne Zutun einer medizinischen Kraft, des Herstellers oder weiterer Personen, zu einem Vorbestimmungszeitpunkt (oder Bestimmungszeitpunkt), vorzugsweise aus einer Vielzahl von möglichen Höhen, die - vor Behandlungsbeginn optional noch nicht bekannte, z. B. zumindest innerhalb von vorgegebenen Grenzen, variable - Höhe der nächsten Applikationsdosis, z. B. aus einer Vielzahl von möglichen oder festlegbaren Applikationsdosen, vorzubestimmen oder zu bestimmen, etwa auszuwählen, zu errechnen oder festzulegen, welche als nächstes, z. B. zu einem nächsten Abgabezeitpunkt, an den Benutzer abgebbar oder für eine Abgabe an diese abgebbar ist, sollte dieser außerhalb einer Sperrdauer, falls eine solche gesetzt ist, die Abrufvorrichtung betätigen. Die Höhe der nächsten Applikationsdosis kann vorab unbekannt sein. Die Höhe der nächsten Applikationsdosis kann vorab nicht gespeichert sein, also einem Speicher nicht entnommen werden, sie muss vielmehr bestimmt oder vorbestimmt werden. In manchen Ausführungsformen ist die Höhe der nächsten Applikationsdosis unbekannt, bevor die Steuervorrichtung sie nicht vorbestimmt oder bestimmt hat, etwa durch ihr Berechnen. Sie kann somit bis zum Vorbestimmungszeitpunkt unbekannt sein. Sie kann variabel sein.

Die elektronische Steuervorrichtung kann programmiert sein, die Höhe der nächsten Applikationsdosis erst nach einem vorhergehenden Betätigen der Abrufvorrichtung vorzubestimmen.

Die elektronische Steuervorrichtung kann programmiert sein, die Höhe der nächsten Applikationsdosis nicht vor wenigstens erstmaliges Betätigen der Abrufvorrichtung vorzubestimmen.

Die elektronische Steuervorrichtung kann programmiert sein, die Höhe der jeweils nächsten Applikationsdosis für jede Applikationsdosis, oder zumindest für mehrere Applikationsdosen, zu bestimmen.

Die elektronische Steuervorrichtung kann programmiert sein, die mittels der Dosiererfassungsvorrichtung erfassten Höhen von abgegebenen Applikationsdosen im Datenspeicher zu speichern.

Ein solcher Vorbestimmungszeitpunkt liegt zu oder nach dem Betätigungszeitpunkt. Der Schritt des Vorbestimmens erfolgt unter Berücksichtigung von aus dem Datenspeicher ausgelesenen Daten. Die ausgelesenen Daten umfassen hierbei vorzugsweise zumindest einerseits den Abgabezeitpunkt einer oder mehrerer der bereits abgegebenen Applikationsdosen, oder die zwischen dem oder den mehreren Abgabezeitpunkten der bereits abgegebenen Applikationsdosen und dem zu oder nach dem nächsten Betätigungszeitpunkt liegenden Abgabezeitpunkt verstrichenen Zeitdauer. Alternativ oder ergänzend umfassen die ausgelesenen Daten vorzugweise andererseits die Höhe einer oder mehrerer der insbesondere mittels der Medikamentenabgabevorrichtung bereits abgegebenen Applikationsdosen.

Die Höhe der nächsten Applikationsdosis unterscheidet sich in einigen Ausführungsformen von der Höhe wenigstens einer, mehrerer oder aller bislang vorbestimmten, früheren Applikationsdosen.

Die ausgelesenen Daten umfassen in bestimmten Ausführungsformen keine Daten, die auf einer Eingabe des Patienten mit Blick auf sein Befinden eingeben sind, oder beruhen nicht ausschließlich auf diesen, allenfalls auch. Solche Daten können patientenindividuelle Steuerungsdaten sein und/oder Daten, welche die Beschwerdedauer, Beschwerdestärke und/oder Beschwerdehäufigkeit abbilden.

In einigen Ausführungsformen ist der Abgabedosierer nicht die Dosiererfassungsvorrichtung. Während der Abgabedosierer die Applikationsdosis an den Patienten abgibt, erfasst die Dosiserfassungsvorrichtung die Höhe dessen, was tatsächlich abgegeben wurde.

In manchen Ausführungsformen legt die Dosiererfassungsvorrichtung die Höhe der nächsten Applikationsdosis nicht fest.

In einigen Ausführungsformen weist der Nasenapplikator kein Display auf, welches angesteuert werden würde, um eine verbleibende Anzahl an Applikationsdosen und/oder eine Anzahl bereits abgegebener Applikationsdosen anzuzeigen.

In manchen Ausführungsformen weist der Nasenapplikator keine Vielzahl von Vials, insbesondere identischen Vials, auf.

In einigen Ausführungsformen ergeht nach Ablauf einer vorbestimmten Höchstzeitdauer kein Erinnerungssignal, z. B. an den Nutzer gerichtet, zur erneuten Betätigung der Abrufvorrichtung.

In manchen Ausführungsformen umfasst der Datenspeicher weiter pharmakologische, pharmakodynamische, pharmakometrische und/oder pharmakokinetische Daten, insbesondere die Substanz betreffend, die appliziert wird oder werden soll. Diese Daten können Modelle, insbesondere PK-Modelle, oder PK-Kurven, sein oder umfassen. Diese Daten können Formeln sein und/oder basierend auf ihnen erzeugt werden, z. B. on-line, von der elektronischen Steuervorrichtung und/oder in *real time* (Echtzeit), oder erzeugt worden sein. Sie können patientenindividuell erfasst bzw. zusammengestellt worden sein. Alternativ oder ergänzend können auch andere, der Substanz zugeordnete, Daten vom Datenspeicher umfasst oder dort hinterlegt sein.

Zu den Daten können beispielsweise die Dosishalbwertzeit und/oder die Eliminations- oder terminale Halbwertszeit der Substanz zählen.

Die Dosishalbwertszeit ist die benötigte Zeit, bis die Konzentration (z. B. im Plasma, im Blut, am Wirkort (ZNS, zentrales Nervensystem), im Fettgewebe, in der Leber, etc.) auf den halben Wert (50%) der verabreichten Dosis gesenkt ist.

Die Eliminations- oder terminale Halbwertszeit ist die benötigte Zeit, bis die bei Pseudo-Equilibrium vorherrschende Konzentration um auf den halben Wert (50%) absinkt.

Die elektronische Steuervorrichtung ist optional programmiert, um zusätzlich auch solche Daten aus dem Datenspeicher auszulesen.

Ferner erfolgt hierbei das Vorbestimmen der nächsten oder weiteren Applikationsdosis der Substanz unter zusätzlicher Berücksichtigung der zusätzlich ausgelesenen Daten. Dabei kann der Vorbestimmungszeitpunkt, wie optional auch der Betätigungszeitpunkt, dem Abgabezeitpunkt der nächsten Applikationsdosis entsprechen, er kann aber auch vor dem Abgabezeitpunkt liegen.

Das Sperrdauerende der Sperrdauer kann dem Zeitpunkt der maximalen Konzentration (dieser Zeitpunkt ist hierin auch bezeichnet als: tₘₐₓ) zwischen Abgabe zweier aufeinander folgenden Applikationsdosen der Substanz entsprechen oder hiervon abgeleitet sein.

Die Sperrdauer kann in bestimmten Ausführungsformen vom Konzentrationsmaximum abhängen und entsprechend vom Arzt oder anderen berechtigten Personen oder automatisch von der Medikamentenabgabevorrichtung, alternativ von einer definierten Konzentration, alternativ von der Zeit bis diese definierte Konzentration unterschritten wird.

In einigen Ausführungsformen ist die elektronische Steuervorrichtung weiter programmiert zum Veranlassen eines Abgebens der nächsten oder der weiteren Applikationsdosis in der vorbestimmten Höhe zum entsprechenden Abgabezeitpunkt mittels des Abgabedosierers. Die elektronische Steuervorrichtung ist ferner programmiert, um nachfolgend die Höhe und/oder eine daraus resultierende Konzentration der Applikationsdosis, beispielsweise als Mengenangabe oder Volumenangabe usw. der Substanz, der abgegebenen nächsten Applikationsdosis im Datenspeicher zu speichern oder speichern zu lassen. Weiter ist die Steuervorrichtung vorzugsweise programmiert, um beispielsweise zeitgleich oder nachfolgend zur Abgabe der Applikationsdosis die Sperrvorrichtung zum zeitweisen Sperren des Abgabedosierers für eine bestimmte Sperrdauer zu veranlassen.

Die Sperrdauer beginnt beispielsweise ab oder nach dem Abgabezeitpunkt dieser nächsten Applikationsdosis und ist definiert durch einen Sperrdauerbeginn und/oder ein Sperrdauerende, was auch auf beliebige andere hierin genannte Sperrdauern zutreffen kann.

Der Abgabezeitpunkt der abgegebenen, nächsten Applikationsdosis kann im Datenspeicher gespeichert werden, die Steuervorrichtung kann hierzu programmiert sein.

In manchen Ausführungsformen ist die elektronische Steuervorrichtung weiter programmiert, um die Abfolge der weiteren vorstehend genannten Schritte auch im Zusammenhang mit einer oder mehreren der auf die nächste Applikationsdosis folgenden Abgaben von weiteren Applikationsdosen, die zeitlich nach der nächsten Applikationsdosis liegen, auszuführen oder zu veranlassen.

In einigen Ausführungsformen ist die elektronische Steuervorrichtung programmiert, aus dem Datenspeicher Daten auszulesen, welche z. B. PK-Kurven und/oder PK-Modelle umfassen, und welche einen zeitlichen Verlauf der Konzentration der Substanz im Körper, insbesondere im Blut, des Patienten abbilden. Basierend auf diesen Kurven oder Modellen kann die elektronische Steuervorrichtung ein nächstes Maximum der Konzentration errechnen. Das nächste Maximum kann das Maximum sein, das die Konzentration zwischen unmittelbar aufeinanderfolgenden Abgabezeitpunkten einnimmt. Es kann das Ergebnis kumulierter Applikationsdosen sein.

In bestimmten Ausführungsformen wird dieses Maximum der Konzentration errechnet und zur Festlegung der Sperrdauer herangezogen. Beim Festlegen der Sperrdauer kann der Zeitpunkt, zu welchem dieses Maximum erzielt wird, berücksichtigt werden.

In manchen Ausführungsformen ist die elektronische Steuervorrichtung weiter programmiert, die nächste Applikationsdosis derart zu bestimmen, dass das nächste Maximum der Konzentration einen vorgegebenen Maximalwert oder ein vorgegebenes Maximum nicht übersteigen wird. Die Bestimmung der Applikationsdosis erfolgt in diesen Ausführungsformen basierend auf im Datenspeicher gespeicherten Daten, insbesondere PK-Kurven oder Modelle, und einem im Datenspeicher vorgehaltenen, therapeutischen Maximum. Das therapeutische Maximum kann das Maximum einer einzelnen Applikationsdosis sein oder das Maximum einer Summe von mehreren Applikationen oder das Maximum einer Summe von mehreren Applikationen über eine bestimmte Zeit bzw. innerhalb eines bestimmten Zeitintervalls.

In einigen Ausführungsformen umfasst das Auswerten des erfassten Betätigungsverhaltens des Benutzers das Feststellen des Betätigungszeitpunkts, zu welchem der Benutzer die nächste oder die weitere Applikationsdosis abrufen möchte. Alternativ umfasst das Auswerten des Benutzungsverhaltens die Zeitspanne, die zwischen diesem Betätigungszeitpunkt und dem Sperrdauerende der zuletzt abgelaufenen Sperrdauer liegt.

Hierbei umfasst das Vorbestimmen das Berücksichtigen des festgestellten nächsten Betätigungszeitpunkts oder das Berücksichtigen der festgesellten Zeitspanne.

In manchen Ausführungsformen weist die Medikamentenabgabevorrichtung oder der Nasenapplikator weiter eine Feedbackvorrichtung auf. Sie dient dem Benutzer, ein Feedback z. B. zu seinem Wohlbefinden, seinem Schmerzzustand oder dergleichen abzugeben. Die Feedbackvorrichtung, welche ein Schalter, eine Touchfläche, usw. sein kann, kann z. B. vorgesehen sein, um vom Benutzer dann betätigt zu werden, wenn, oder solange er schmerzfrei ist oder auf andere Weise eine Aussage über die Wirkung der Substanz in seinem Körper machen kann. So kann verabredet sein, dass der Benutzer die Feedbackvorrichtung erstmals dann betätigt, z. B. drückt, wenn der Schmerz nach der dem Benutzer zuletzt applizierten Applikationsdosis erstmals als erträglich empfunden wird. Weiter kann verabredet sein, dass der Benutzer die Feedbackvorrichtung ab dem Zeitpunkt nicht mehr betätigt, zu dem er erstmals wieder (aufgrund nachlassender Konzentration der Substanz im Körper) Schmerzen empfindet.

Die Feedbackvorrichtung kann mit der Abrufvorrichtung identisch sein, z. B. mit einer eigenen Schalterauswertung, etwa indem sie länger, kürzer, öfter oder auf andere Weise anders zu betätigen ist als die Abrufvorrichtung, alternativ kann sie eine eigene Vorrichtung zum Betätigen durch den Benutzer sein.

Hierbei ist die Erfassungsvorrichtung programmiert zum Erfassen einer erfolgten Betätigung der Feedbackvorrichtung durch den Benutzer zu wenigstens einem, vorzugsweise ersten, Feedbackzeitpunkt.

Ferner ist die Steuervorrichtung hierbei programmiert zum Ermitteln der im Körper oder im Blut des Benutzers vorliegenden Konzentration der Substanz zu dem wenigstens einen Feedbackzeitpunkt.

Ein Ermitteln kann hierin stets ein Errechnen, Aus- und/oder Ablesen sein.

Ergänzend ist die Steuervorrichtung programmiert zum Festsetzen einer Zielkonzentration oder ihres Maximalwerts auf einen Wert jeweils unterhalb, oberhalb oder gleich der ermittelten Konzentration.

Immer wenn hierin von einer "Zielkonzentration" die Rede ist, kann dies die Konzentration sein, die einen gewünschten therapeutischen Effekt bewirkt.

In einigen Ausführungsformen ist die Erfassungsvorrichtung weiter programmiert zum Erfassen von wenigstens einer Betätigung der Abrufvorrichtung durch den Benutzer zu unterschiedlichen Betätigungszeitpunkten, insbesondere nach der Sperrdauer. Alternativ oder ergänzend ist die Erfassungsvorrichtung programmiert zum Erfassen eines, Feedbackzeitpunkts, vorzugsweise eines letzten Feedbackzeitpunkts, ab welchem weitere Betätigungen der Feedbackvorrichtung durch den Benutzer zumindest bis zum Abgabezeitpunkt der nächsten Applikationsdosis ausbleiben.

Optional ist von der vorliegenden Erfindung auch das Speichern dieser Feedbackzeitpunkte umfasst.

Die Steuervorrichtung kann weiter programmiert sein zum Ermitteln von Konzentrationen der Substanz im Körper, insbesondere im Blut, etwa anhand der ausgelesenen Daten wie z. B. PK-Kurven, PK-Modellen, usw. Hierbei ist beispielsweise sowohl die Konzentration zum, vorzugsweise ersten, Feedbackzeitpunkt als auch die Konzentration zum, vorzugsweise ersten, Betätigungszeitpunkt (z. B. seit Ende der letzten Sperrdauer) oder zum letzten Feedbackzeitpunkt relevant. Diese Konzentrationen können den jeweiligen Zeitpunkten zugeordnet sein oder werden.

Ferner kann die Steuervorrichtung weiter programmiert sein zum Ermitteln oder Auswählen eines zeitlichen Konzentrationsverlaufs aus einer Gruppe von zeitlichen Konzentrationsverläufen. Beim Ermitteln des zeitlichen Konzentrationsverlaufs wird sowohl die Konzentration zum, vorzugsweise ersten, Feedbackzeitpunkt als auch die Konzentration zum, vorzugsweise ersten Betätigungszeitpunkt oder dem letzten Feedbackzeitpunkt berücksichtigt.

Wenn hierin von einer "Gruppe von zeitlichen Konzentrationsverläufen" die Rede ist, so ist dies eine Gruppe von Konzentrationsverläufen, die sich auf dieselbe Dosis (z. B. 50 µg Fentanyl) beziehen oder die hiervon abgeleitet werden. Insbesondere umfassen die Konzentrationsverläufe solche, die den zeitlichen Verlauf der Konzentration für den Mittelwert aller mittels dieser Dosis untersuchten Patienten eines Kollektivs widerspiegeln, oder den Mittelwert plus/minus einer oder mehrerer Standardabweichungen. Eine Vielzahl solcher Verläufe oder Daten können im Datenspeicher gespeichert sein. Aus ihr kann die Gruppe der betrachteten zeitlichen Verläufe ausgewählt sein.

Ferner kann die Steuervorrichtung programmiert sein zum Berücksichtigen des so ermittelten zeitlichen individuellen Konzentrationsverlaufs beim Vorbestimmen der Höhe einer oder mehrerer der nachfolgenden Applikationsdosen, insbesondere zum Berücksichtigen der ermittelten individuellen PK-Kurve.

In manchen Ausführungsformen des Nasenapplikators ist die Steuervorrichtung weiter programmiert, um beim Vorbestimmen der Höhe der nächsten Applikationsdosis die festgesetzte Zielkonzentration oder ihren Maximalwert zu berücksichtigen. Alternativ oder ergänzend kann vorgesehen sein, dass beim Vorbestimmen der ermittelte zeitliche individuelle Konzentrationsverlauf, insbesondere die ermittelte individuelle PK-Kurve, berücksichtigt wird.

In einigen Ausführungsformen ist die Steuervorrichtung weiter programmiert, um nach der Abgabe einer Ausgangsdosis oder Initialdosis zu einem früheren Zeitpunkt, zu jeweils einem ersten (oder hier als "initial" bezeichneten) Zeitpunkt der Betätigung der Abrufvorrichtung durch den Benutzer (vorzugsweise nach Ablauf einer sich an die Abgabe der Ausgangsdosis anschließende Sperrdauer, in welcher dem Benutzer auf Betätigen der Abrufvorrichtung hin keine Applikationsdosis appliziert wird), eine, diesem Betätigungszeitpunkt zugeordnete Konzentration zu ermitteln, und zwar für jeden der (im Datenspeicher hinterlegten) Konzentrationsverläufe der/einer Gruppe zu ermitteln oder hiervon abzuleiten. Dieser erste Betätigungszeitpunkt liegt zeitlich nach dem Abgabezeitpunkt der Ausgangsdosis. Die Steuervorrichtung ist weiter programmiert, um die so ermittelten Konzentrationen jeweils als geschätzte Zielkonzentration des zugehörigen zeitlichen Konzentrationsverlaufs festzulegen. Hat es, vereinfacht gesprochen, eine Ausgangsdosis gegeben, und hat der Benutzer nach Ablauf einer auf diese folgende Sperrdauer eine weitere Applikationsdosis abrufen wollen, so werden dem Zeitpunkt, zu welchem der Benutzer versucht hat, die weitere Applikationsdosis abzurufen, für jeden der betrachteten zeitlichen Konzentrationsverlauf eine Konzentration zugeordnet. Da die so zugeordnete Konzentration rechnerisch ermittelt wurde und das Ergebnis einer Hypothese ist, welche noch einer Überprüfung und Bestätigung bedarf, wird die so ermittelte Konzentration hierin als geschätzte Zielkonzentration bezeichnet. Hat die Gruppe von betrachteten zeitlichen Verläufen drei Elemente (Verläufe), so erhält man drei geschätzte Zielkonzentrationswerte. Man stellt, anders ausgedrückt, drei Hypothesen auf. Sie besagen, wie hoch die Konzentration im Körper sein müsste, würde der erste, der zweite oder der dritte Konzentrationsverlauf auf der Gruppe für den betrachteten Benutzer zutreffen.

Eine Ausgangsdosis kann hierin auch als erste oder vorausgehende Applikationsdosis bezeichnet werden, welche zu einem Abgabezeitpunkt, der entsprechend der erste Abgabezeitpunkt sein kann, abgegeben wurde. Die Ausgangsdosis kann die tatsächlich allererste Applikation mittels der Medikamentenabgabevorrichtung sein.

Die Steuervorrichtung kann ferner zum Applizieren der auf die Ausgangsdosis folgenden ersten Applikationsdosis als Reaktion auf die Betätigung der Abrufvorrichtung zum ersten Betätigungszeitpunkt programmiert sein.

Weiter kann die Steuervorrichtung zum Ermitteln eines Zeitpunkts, zu welchem nach dem Applizieren dieser ersten Applikationsdosis die Konzentration im Körper, insbesondere im Blut, jeweils erneut auf die geschätzte Zielkonzentration des zugehörigen zeitlichen Konzentrationsverlaufs abgesunken sein wird oder sein sollte. Dieser Zeitpunkt wird hierin auch als berechneter Zielkonzentrationszeitpunkt bezeichnet. Um ihn jeweils (also für jeden Konzentrationsverlauf aus der Gruppe) zu berechnen, werden jeweils erneut dieselben zeitlichen Konzentrationsverläufe der Gruppe herangezogen, für die jeweils bereits, ein berechneter Zielkonzentrationszeitpunkt festgelegt ist. Anzumerken ist, dass Unterschiede zwischen der Höhe der Ausgangsdosis und der nachfolgenden, ersten Applikationsdosis hierbei berücksichtigt werden. Betrug die Ausgangsdosis beispielsweise 50 µg Fentanyl, so haben die drei ausgewählten zeitlichen Konzentrationsverläufe der Gruppe angegeben, wie diese 50 µg Fentanyl im Körper nachzuweisen sein könnten. Ein erster der drei Konzentrationsverläufe könnte z. B. annehmen, dass der Verlauf sich wie in einem Kollektiv durchschnittlich beobachtet verhalten wird. Ein zweiter kann z. B. annehmen, dass sich der Verlauf wie der erste Verlauf um plus eine Standardabweichung, plus 10%, usw. verhält. Ein dritter kann z. B. annehmen, dass der Verlauf sich wie der erste Verlauf um minus eine Standardabweichung, minus 10%, usw. verhält. Beträgt die nachfolgende, erste Applikationsdosis nun aber beispielsweise nicht ebenfalls 50 µg, sondern nur 30 µg Fentanyl, so werden jeweils dieselben drei ausgewählten zeitlichen Konzentrationsverläufe der Gruppe angenommen. Sie sind nur nicht mehr auf 50 µg, sondern auf 30 µg Fentanyl berechnet.

Die Steuervorrichtung kann ferner zum direkten oder indirekten Erfassen oder Feststellen des zweiten Betätigungszeitpunkts zum Abrufen einer zweiten oder auf die nachfolgenden, erste Applikationsdosis ihrerseits nachfolgende, z. B. zweite, Applikationsdosis programmiert sein.

Ergänzend kann die Steuervorrichtung programmiert sein, um einen zeitlichen Konzentrationsverlauf aus der Gruppe als individuellen zeitlichen Konzentrationsverlauf, insbesondere als individuelle PK-Kurve zu ermitteln. Hierbei wird die jeweilige Differenz zwischen dem berechneten Zielkonzentrationszeitpunkt eines jeden zeitlichen Konzentrationsverlaufs aus der Gruppe und dem zweiten Betätigungszeitpunkt berücksichtigt. Als individueller zeitlicher Konzentrationsverlauf kommt insbesondere jener zeitliche Konzentrationsverlauf in Frage, welcher die geringste Differenz zwischen seinem berechneten Zielkonzentrationszeitpunkt und dem zweiten Betätigungszeitpunkt aufweist.

Die Steuervorrichtung kann ferner programmiert sein, um beim Vorbestimmen der Höhe einer oder mehrerer der nachfolgenden Applikationsdosen den ermittelten zeitlichen individuellen Konzentrationsverlauf zu berücksichtigen, was insbesondere eine individuelle PK-Kurve oder ein individuelles PK-Modell sein kann.

In manchen Ausführungsformen ist die Steuervorrichtung weiter programmiert zum Ermitteln der im Körper oder im Blut des Benutzers vorliegenden Konzentration der Substanz. Das Ermitteln erfolgt zu wenigstens einem betrachteten Betätigungszeitpunkt oder zu wenigstens einem betrachteten Vorbestimmungszeitpunkt. Ferner ist die Steuervorrichtung programmiert, diese Konzentration als die Zielkonzentration festzusetzen.

Hierbei ist die Steuervorrichtung weiter programmiert, um am nächsten Vorbestimmungszeitpunkt die Höhe der/einer nächsten Applikationsdosis derart festzusetzen, dass die Konzentration im Körper oder im Blut des Benutzers erst nach Ablauf einer vorbestimmten Zeitdauer erneut auf die Zielkonzentration absinken wird. Diese Zeitdauer kann einstellbar sein, beispielsweise durch den behandelnden Arzt, und beginnt mit dem Abgabezeitpunkt der nächsten Applikationsdosis.

Hierzu kann analog wie oben beschrieben der unveränderliche zeitliche individuelle Konzentrationsverlauf, also die PK-Kurve des Benutzers zugrunde gelegt und/oder berücksichtigt werden.

In einigen Ausführungsformen ist die elektronische Steuervorrichtung weiter programmiert, um im Schritt des Vorbestimmens der Höhe der nächsten Applikationsdosis aus dem Datenspeicher ausgelesene Daten zu berücksichtigen. Diese Daten umfassen sowohl den Abgabezeitpunkt einer oder mehrerer der bereits abgegebenen Applikationsdosen einerseits als auch die Höhe einer oder mehrerer der bereits abgegebenen Applikationsdosen andererseits. Es ist von der vorliegenden Erfindung umfasst, dass hierbei alternativ zum Abgabezeitpunkt die zwischen dem oder den mehreren Abgabezeitpunkten der bereits abgegebenen Applikationsdosen und dem nach dem nächsten Betätigungszeitpunkt liegenden Abgabezeitpunkt verstrichenen Zeitdauer betrachtet werden kann.

In manchen Ausführungsformen umfasst das Auswerten des Betätigungsverhaltens des Benutzers, welches mittels der Erfassungsvorrichtung erfasst wird, das Erfassen von Betätigungen der Abrufvorrichtung durch den Benutzer zu Betätigungszeitpunkten innerhalb einer Sperrdauer. Dies kann in einer beliebigen, einer bestimmten, d. h, in der ersten und/oder in der zweiten usw., oder in jeder Sperrdauer erfolgen. In bestimmten Ausführungsformen ist optional auch das Speichern dieses Betätigungsverhaltens in oder auf einer hierzu geeigneten Vorrichtung, z. B. dem hierin genannten Datenspeicher, vorgesehen.

Die Steuervorrichtung ist weiter programmiert, um eine Konzentration der Substanz im Körper, insbesondere im Blut, zu ermitteln, die den jeweiligen Betätigungszeitpunkten innerhalb einer der Sperrdauern zugeordnet ist oder sein kann.

Die Steuervorrichtung ist weiter programmiert, um eine Zielkonzentration oder ihrem Mindestwert auf einen Wert jeweils oberhalb, unterhalb oder gleich der zugeordneten Konzentration festzusetzen.

In bestimmten Ausführungsformen kann das oben ausgeführte Vorgehen mehrfach wiederholt, d. h. in Schleife, ausgeführt werden. Die so angenäherte Zielkonzentration, oder der so angenäherte Mindestwert kann optional nach einer oder jeder Sperrdauer auf Null (0) zurückgesetzt werden.

In einigen Ausführungsformen ist die Erfassungsvorrichtung des erfindungsgemäßen Nasenapplikators weiter programmiert, um wenigstens eine Betätigung der Abrufvorrichtung durch den Benutzer zu wenigstens einem Betätigungszeitpunkt nach der Sperrdauer zu erfassen und optional zu speichern.

Hierbei ist die Steuervorrichtung weiter programmiert zum Ermitteln einer Konzentration im Körper, insbesondere im Blut, welche dem Betätigungszeitpunkt nach der Sperrdauer jeweils zugeordnet ist.

Weiter ist die Steuervorrichtung programmiert zum Ermitteln eines zeitlichen Konzentrationsverlaufs der Gruppe. Hierbei wird sowohl wenigstens ein, vorzugsweise der letzte, Betätigungszeitpunkt innerhalb der (beliebigen) Sperrdauer als auch wenigstens ein, vorzugsweise der erste, Betätigungszeitpunktes nach der Sperrdauer berücksichtigt.

Die Steuervorrichtung ist ferner programmiert, um beim Vorbestimmen der Höhe einer oder mehrerer der nachfolgenden Applikationsdosen den ermittelten zeitlichen individuellen Konzentrationsverlauf zu berücksichtigen, insbesondere die ermittelte individuelle PK-Kurve.

In manchen Ausführungsformen des Nasenapplikators ist die Steuervorrichtung weiter programmiert, um beim Vorbestimmen der Höhe der nächsten Applikationsdosis die festgesetzte Zielkonzentration oder ihren Mindestwert und/oder den ermittelten zeitlichen individuellen Konzentrationsverlauf, insbesondere die ermittelte individuelle PK-Kurve, zu berücksichtigen.

In einigen Ausführungsformen umfasst das Auswerten des Betätigungsverhaltens des Benutzers, das mittels der Erfassungsvorrichtung erfasst wird, das Auswerten von Betätigungen der Abrufvorrichtung durch den Benutzer zu Betätigungszeitpunkten innerhalb einer der Sperrdauern. Dies kann in einer beliebigen, einer bestimmten, d. h, in der ersten und/oder in der zweiten usw., oder in jeder Sperrdauer erfolgen. In bestimmten Ausführungsformen ist optional auch das Speichern dieses Betätigungsverhaltens in oder auf einer hierzu geeigneten Vorrichtung vorgesehen.

Weiter kann die Steuervorrichtung programmiert sein zum Ermitteln einer Zunahme der Zeitabstände zwischen aufeinanderfolgenden Betätigungszeitpunkten innerhalb einer der (betrachteten) Sperrdauern, alternativ des Ausbleibens weiterer Betätigungszeitpunkte innerhalb der betrachteten Sperrdauer. Ergänzend kann die Steuervorrichtung programmiert sein, um einen Zeitpunkt festzulegen, ab welchem das Maß der Zunahme oder das Ausbleiben vorbestimmten Kriterien genügt.

Als Kriterien kann beispielsweise festgelegt sein, dass, sobald z. B. 50%-75% von der Sperrdauer T_vainₙ verstrichen sind, man davon ausgehend darf, die Betätigungszeitpunkte auf vorbestimmte Weise wesentlich weiter auseinander liegen als zuvor, und/oder falls für eine, z. B. vorbestimmte, Zeitdauer keine Betätigung mehr erfolgt ist, davon ausgegangen wird, dass die Wirkung des therapeutischen Effektes spürbar/bemerkbar geworden ist und/oder die Zielkonzentration erreicht wurde.

Für manche Ausführungsformen kann festgelegt sein, dass, wenn rechnerisch z. B. 80% oder ein anderer, vorbestimmter Anteil der errechneten Maximalkonzentration erreicht wurde und es immer noch vergebliche Abrufversuche gibt, also der gewünschte therapeutische Effekt noch nicht erreicht wurde, das Ende der laufenden Sperrdauer vorverlegt wird, da die restlichen z. B. 20% der Konzentration nicht ausreichen werden, um den bei derzeitig angelegten Regime den Benutzer auf die Zielkonzentration (C_{ED}) zu bringen.

Für einige Ausführungsformen kann festgelegt sein, dass, wenn rechnerisch z. B. 80% der errechneten Maximalkonzentration erreicht wurde und es immer noch vergebliche Abrufversuche gibt, also der gewünschte therapeutische Effekt noch nicht erreicht wurde, der nächste Vorbestimmungszeitpunkt vorverlegt wird im Wissen, dass wahrscheinlich zum Ende der Sperrdauer ein Abrufversuch des Benutzers stattfinden wird.

Wenn diese Zeitintervalle größer werden, bedeutet dies, dass der gewünschte therapeutische Effekt, vielleicht langsam, aber auf jeden Fall anfängt einzusetzen. Somit kann im Fall, dass nach dem Ende der Sperrdauer eine Betätigung durch den Benutzer stattfindet, die Dosis kleiner ausfallen bzw. berechnet werden, wie wenn kein therapeutischer Effekt vorliegen würde.

Ferner kann die Steuervorrichtung programmiert sein zum Ermitteln einer dem Zeitpunkt zugeordneten Konzentration der Substanz im Körper, insbesondere im Blut.

Die Steuervorrichtung kann weiter programmiert sein, um die Zielkonzentration oder ihren Mindestwert auf einen Wert jeweils oberhalb, unterhalb oder gleich der zugeordneten Konzentration festzusetzen.

In manchen Ausführungsformen ist die elektronische Steuervorrichtung weiter programmiert zum Auswerten von wenigstens einer Betätigung der Abrufvorrichtung durch den Benutzer zu wenigstens einem Betätigungszeitpunkt, welcher nach der Sperrdauer liegt. Optional ist auch das Speichern der Betätigung in oder auf einer hierzu geeigneten Vorrichtung von der vorliegenden Erfindung umfasst.

Hierbei ist die Steuervorrichtung weiter programmiert, um eine Konzentration im Körper, insbesondere im Blut, zu ermitteln für den oder die Betätigungszeitpunkte nach der Sperrdauer.

Die Steuervorrichtung kann hierbei weiter programmiert sein, um einen zeitlichen Konzentrationsverlauf der/einer Gruppe unter Berücksichtigung sowohl eines, vorzugsweise des letzten, Betätigungszeitpunktes innerhalb der Sperrdauer als auch eines, vorzugsweise des ersten, Betätigungszeitpunktes nach der Sperrdauer zu ermitteln.

Ergänzend kann die Steuervorrichtung programmiert sein, beim Vorbestimmen der Höhe einer oder mehrerer der nachfolgenden Applikationsdosen den ermittelten zeitlichen individuellen Konzentrationsverlauf, insbesondere die ermittelte individuelle PK-Kurve zu berücksichtigen.

In einigen Ausführungsformen ist die Steuervorrichtung des Nasenapplikators weiter programmiert, um beim Vorbestimmen der Höhe der nächsten Applikationsdosis die festgesetzte Zielkonzentration oder ihren Mindestwert zu berücksichtigen. Alternativ oder ergänzend kann die Steuervorrichtung den ermittelten zeitlichen individuellen Konzentrationsverlauf berücksichtigen, insbesondere die ermittelte individuelle PK-Kurve.

In manchen Ausführungsformen ist die Steuervorrichtung weiter zum Begrenzen der Höhe der nächsten Applikationsdosis und/oder zum Begrenzen der daraus resultierenden Konzentration programmiert.

Alternativ oder ergänzend ist die Steuervorrichtung zum Begrenzen der kumulierten Höhe aller innerhalb einer vorbestimmten Zeitdauer abgegebenen Applikationsdosen und/oder zum Begrenzen der daraus resultierenden Konzentrationen programmiert.

Wiederum alternativ oder ergänzend ist die Steuervorrichtung zum Begrenzen der kumulierten Höhe aller abgegebenen Applikationsdosen und/oder zum Begrenzen der daraus resultierenden Konzentrationen programmiert.

Hierzu greift die Steuervorrichtung optional auf im Datenspeicher gespeicherte Daten zurück, die die Substanz betreffen.

In einigen Ausführungsformen ist der erfindungsgemäße Nasenapplikator geeignet zur nasalen Verabreichung einer Applikationsdosis von Substanzen, welche insbesondere medizinische Wirkstoffe sind oder aufweisen.

In anderen Ausführungsformen betrifft die Erfindungen eine Medikamentenabgabevorrichtung, welche kein Nasenapplikator ist. Was hierin zum Nasenapplikator ausgeführt ist, gilt daher uneingeschränkt auch für eine Medikamentenabgabevorrichtung als einen Nasenapplikator, insbesondere wenn diese tragbar und vom Benutzer, z. B. einem Patienten, allein betätig bar ist und üblicherweise auch ohne fremde Hilfe oder besonderes Wissen betätigt wird.

Insbesondere ist eine Medikamentenabgabevorrichtung für andere Verabreichungsarten geeignet, beispielsweise für weitere transmukosale Verabreichungen, insbesondere über die Mundschleimhaut, beispielsweise in der Backentasche (bukkal) oder unter der Zunge (sublingual), für intravenöse oder auch für inhalative Verabreichungen.

In manchen Ausführungsformen ist oder umfasst der Abgabedosierer ein(en) Partikel- oder Tröpfchengenerator, beispielsweise eine Wirbelkammer, ein(en) Sprühkopf, eine Düse, eine Kanüle, ein(en) Tropfer, eine Lochplatte, ein(en) Baffle, eine poröse Membran oder dergleichen.

In einigen Ausführungsformen umfasst der Abgabedosierer einen Energiebeaufschlagungsmechanismus und/oder einen Fördermechanismus. Dieser kann mechanisch und/oder elektronisch ausgestaltet sein, z. B. als Mikropumpe, Kolbenpumpe, Rotationspumpe, Peristaltikpumpe, Kreiselpumpe oder dergleichen.

In manchen Ausführungsformen umfasst der Abgabedosierer eine analoge, insbesondere stufenlose, und/oder eine digitale (0 oder 1) Steuerung zur Dosierung. Diese kann Teil der elektronischen Steuervorrichtung des Nasenapplikators sein. Insbesondere entspricht eine Applikationsdosis oder Abgabemenge der Substanz einer Menge im Bereich zwischen 1 µL bis 300 µL pro Betätigung.

In einigen Ausführungsformen kann eine hohe Reproduzierbarkeit und Genauigkeit bei der Abgabe der Menge durch eine benutzerunabhängige Betätigung (alternativ: Auslösung) durch immer gleiche Auslöseparameter wie z. B. Auslösegeschwindigkeit, Auslösekraft, Beschleunigung, Flussrate, Druck, Rotation, Hub, etc. gewährleistet sein.

In manchen Ausführungsformen umfasst der Nasenapplikator ein Dosierventil.

In bestimmten Ausführungsformen umfasst der Nasenapplikator einen, insbesondere abnehmbaren, Elastomerring zum Aufschieben auf einen Nasenaufsatz (auch bekannt als Nasendip). Der Elastomerring dient einem möglichst luftdichten Abschließen des Nasenlochs gegenüber einem Äußeren.

In einigen Ausführungsformen umfasst der Nasenapplikator, z. B. handelsübliche oder herkömmliche, Nasenspray-Kartuschen, die eine integrierte Förder- bzw. Pumpvorrichtung aufweisen. Mittels dieser integrierten Förder- bzw. Pumpvorrichtung können nach einer Entlüftung (Priming) verschiedene Dosiergroßen abgeben werden, beispielsweise mittels Verlängerung bzw. Verkürzung des Hubwegs dieser Vorrichtung.

In manchen Ausführungsformen wird eine Förder- bzw. Pumpvorrichtung vom Benutzer/Patienten mechanisch, also von Hand, ausgelöst. Hierzu kann beispielsweise ein Federspeicher manuell oder fremdenergetisch geladen, eingestellt und ausgelöst werden.

In einigen Ausführungsformen umfasst die Dosiserfassungseinrichtung des Nasenapplikators einen Sensor, konfiguriert zum Erfassen der abgegebenen Substanz oder deren Wirkstoffmenge. Das Erfassen der Dosis bzw. ihres Volumens oder Menge kann beispielsweise mittels direkter Messung, z. B. mittels eines Durchflusssensors, etc.) und/oder mittels abgeleiteter Messung erfolgen. Bei einer abgeleiteten Messung wird die Dosis rechnerisch ermittelt bzw. abgeleitet. Dies kann beispielsweise durch Messen von Umdrehungen (min-1), Winkel, Hubweg, Weg, Hubanzahl, Zeit, Ventilbetätigungen (Anzahl und/oder Zeit) etc. erfolgen.

Alternativ oder ergänzend kann das Erfassen der Dosis mittels eines Spraysensors erfolgen, welcher ermöglicht, festzustellen, ob sich tatsächlich Tröpfchen des zerstäubten Fluids bzw. Aerosol im Bereich des Nasenaufsatz bilden bzw. bildet. Beispielsweise nutzt der Spraysensor den Effekt, dass das zerstäubte Fluid bzw. Aerosol Licht streut, und arbeitet als sogenannter Streulicht-Sensor.

In manchen Ausführungsformen kann die Dosiserfassungseinrichtung beispielsweise in die Steuervorrichtung des Nasenapplikators integriert sein. In anderen Ausführungsformen liegt sie separat vor.

In einigen Ausführungsformen kann die Dosis mittels eines Flusssensors, eines Durchflusssensors oder mittels einer Tröpfchenverteilungsmessung und/oder Partikelverteilungsmessung erfolgen.

In manchen Ausführungsformen kann die Tröpfchenverteilung und/oder Partikelgrößenverteilung zur Ermittlung der Dosis gemessen werden.

In einigen Ausführungsformen kann mittels der Auslöseparameter, wie z. B. Auslösegeschwindigkeit, Auslösekraft, Druck, Beschleunigung, Flussrate etc., die Tröpfchengrößenverteilung und/oder Partikelgrößenverteilung eingestellt bzw. angepasst bzw. justiert werden. Dies ist insbesondere dazu vorgesehen, um auf einfache Art und Weise die Tröpfchenverteilung und/oder Partikelgrößenverteilung auf verschiedene Wirkstoffe anzupassen.

In manchen Ausführungsformen umfasst der Datenspeicher des Nasenapplikators wenigstens ein internes Speicherbauteil, welches nicht volatil und mehrfach überschreibbar bzw. beschreibbar ist und/oder ein externes Speicherbauteil, welches insbesondere austauschbar sein kann (z. B. SSD-Speicherkarte, USB-Speicher, HDD, etc.). Als externer Speicher kann alternativ oder ergänzend auch ein Netzwerk, ein Smart Device, eine Ladestation (Docking Station) oder das Internet betrachtet werden.

In einigen Ausführungsformen kann, z. B. als finaler, Schritt des von der Steuervorrichtung getriggerten Verfahrens vorgesehen sein, am Ende der Behandlung oder Therapie ohne oder nach einer automatischen Datensicherung in oder auf einer hierzu geeigneten (weiteren)internen oder externen Speichervorrichtung die persönlichen Daten und/oder individuellen Steuerungsdaten aus dem Datenspeicher des Nasenapplikators zu löschen und/oder den Nasenapplikator auf die Werkseinstellungen zurückzusetzen. Damit kann der Nasenapplikator oder Teile hiervon nach Beendigung der Therapie des Patienten vorteilhafterweise an weitere Patienten weitergegeben werden, ohne dass Datenschutzbedenken dem entgegenstehen müssten.

In manchen Ausführungsformen kann der Datenspeicher, insbesondere der externe Datenspeicher, als digitaler Schmerzausweis implementiert sein und, beispielsweise auf Reisen oder bei Kontrollen, vom Benutzer/Patienten zum Nachweis gegenüber behandelnden Ärzten und/oder Kontrollierenden genutzt werden.

In einigen Ausführungsformen können der Benutzer/Patient im Zusammenwirken mit dem Nasenapplikator als vorprogrammiertes, selbstregulierendes System betrachtet werden, welches eine Programmierung überflüssig macht und potenzielle Fehlerquellen eliminiert. Ähnliches ist aus der Verwendung von *IV-PCA Smartpumps* zur Verabreichung von Opiaten bekannt. Das vorprogrammierte, selbstregulierende System umfasst bestimmte Dosisgrenzen (beispielsweise Dosis Limits, Konzentrationslimits, Zeitlimits, Limits basierend auf einem therapeutischen Effekt, d. h. z. B. eine geringere Dosis bei einem niedrigen Schmerzscore). Diese Dosisgrenzen können Dosisgrenzen pro Zeit, eine bestimmte Anzahl an Auslösungen oder Betätigungen, Grenzen einer Einzeldosis, ein Tagesdosislimit, ein Konzentrationslimit und/oder dergleichen sein. Bei Erreichen der Dosisgrenzen können zwei Konsequenzen erfolgen: Die Abgabe weiterer Applikationsdosen kann entweder pausiert werden oder die Dosis so runtergefahren werden, dass kein therapeutischer Effekt mehr auftreten kann. Insbesondere wird hier jedoch trotzdem ein Placebo-Effekt ermöglicht.

In manchen Ausführungsformen kann der Benutzer/Patient als Regelkreis betrachtet werden und das Auslöseverhalten des Nasenapplikators kann aufgrund der erfassten bzw. ermittelten pharmakologischen Daten entsprechend der Benutzer-/Patientenreaktion in puncto Dosis und Therapie angepasst werden. Hierzu können einerseits direkte Messwerte (Ist Werte), z. B. der Blutzuckerwert oder die Opioidkonzentration im Blut, oder andererseits indirekte Messwerte (abgeleitete und/oder geschätzte Werte), z. B. Pharmakokinetik ("PK-") Kurven einer (Referenz-)Population, herangezogen werden. Vorzugsweise finden direkte Messwerte Eingang in die Anpassung des Auslöseverhaltens.

In einigen Ausführungsformen ist die elektronische Steuervorrichtung weiter programmiert, um einen Vorbestimmungszeitpunkt einer Applikationsdosis der Substanz vorbestimmen zu können, wobei subjektive therapeutische Beobachtungen eines patientenindividuellen therapeutischen Effekts, wie z. B. Schmerz, in Betracht gezogen werden. Diese Beobachtungen können das Schmerzniveau (*pain score*), den Schmerztypus, die Schmerzdauer, die Schmerzintensität und/oder die Schmerzfrequenz des Patienten sein oder umfassen.

Das bedeutet, dass der Benutzer/Patient bestimmt, was für ihn/sie noch erträglich ist bzw. wie stark er den Schmerz empfindet. Dies kann z. B. mittels manueller Eingabe auf einer eindimensionalen Ratingskala zum Feststellen der Schmerzintensität erfolgen. Solche Skalen können beispielsweise sein:
- eine numerische Ratingskala (NRS, beispielsweise von 1 (= schmerzfrei) bis 10 (= stärkste Intensität)),
- eine verbale Ratingskala (VRS, mit Beschreibungen der Schmerzintensität, beispielsweise "keine", "mäßig", "mittel", "schwer", "sehr schwer", oder auch Beschreibungen des zeitlichen Auftretens, beispielsweise "nie", "selten", "manchmal", "häufig", "immer"),
- visuelle Analogskala (VAS, beispielsweise als Linie, deren Endpunkte extreme Zustände darstellen, wie z. B. "kein Schmerz" und "unerträglicher Schmerz", auf welcher der Benutzer/Patient sein subjektives Schmerzempfinden markiert, ohne eine entsprechende Skalierung wahrzunehmen),

- *Functional Activity Scale* (FAS, beispielsweis mit den Einteilungen "keine Einschränkung" (*no limitation*), wenn der Benutzer eine bestimmte Aktivität ohne Einschränkung (z. B. aufgrund von Schmerzen) ausführen kann, "leichte Einschränkung" (*mild limitation*), wenn die Aktivität nur eingeschränkt ausgeführt werden kann (z. B. aufgrund von Schmerzen) und "erhebliche Einschränkung" (*significant limitation*), wenn die Aktivität nicht ausgeführt werden kann (z. B. aufgrund von Schmerzen oder aufgrund von Nebenwirkungen einer Schmerztherapie), oder eine
- Funktionsbeeinträchtigung wie z. B. Schlaflosigkeit, Beeinträchtigungen der Fähigkeit zu essen und/oder zu trinken und/oder Beeinträchtigungen beim tiefen Einatmen.

In manchen Ausführungsformen können objektive und/oder subjektive Beobachtungen in Betracht gezogen werden. Diese können z. B. umfassen:
- Vitalwerte (wie Blutdruck, Herzfrequenz, Temperatur etc.),
- Pupillenreaktion,
- Delirium
- Halluzinationen
- Schwindel
- Toleranz
- Abhängigkeit
- gesteigerte Schmerzempfindlichkeit (Hyperalgesie)
- Sedation (Sedierung),
- Nebenwirkungen (wie Juckreiz (*Itching*), Übelkeit (*Nausea*), Erbrechen (*Vomiting*) etc.),
- physiologische Beobachtungen,
- Gesamtdosis/Volumen und/oder Gesamtkonzentration der Substanz/des Wirkstoffs,
- verbleibende Füllmenge im Behältnis,
- Schlafverhalten.

In einigen Ausführungsformen kann die vorliegende Erfindung zu der sog. personalisierten Medizin (*Personalized Medicine*) gezählt werden, in deren Rahmen der Benutzer/Patient als vorprogrammiertes, selbstregulierendes System betrachtet und mit Dosisgrenzen (absolut) und/oder Dosisgrenzen pro Zeit, Konzentrationsgrenzen bzw. Mengengrenzen und/oder Beschränkung der Anzahl an Auslösungen und/oder Beschränkungen für eine Einzeldosis und/oder Tagesdosis, etc. sowie unten auf- und ausgeführte Alternativen eine möglichst individuelle Therapie angestrebt wird. Je mehr Input bzw. Information zum Status des Benutzers/Patienten verfügbar sind, umso individueller kann die Therapie gestaltet werden. Insbesondere, wenn hierbei Konzentrationsgrenzen betrachtet werden, können sich in bestimmten Ausführungsformen unterschiedlich hohe Dosen ergeben.

In manchen Ausführungsformen ist die elektronische Steuervorrichtung weiter programmiert, um einen Vorbestimmungszeitpunkt einer Applikationsdosis der Substanz vorbestimmen zu können, wobei alternativ oder ergänzend patientenspezifische Setup-Daten eingegeben und/oder in Betracht gezogen werden. Diese Setup-Daten können beispielsweise umfassen oder hervorgehen aus:
- Daten aus der (elektronischen) Patientenakte,
- Daten der individuellen Anamnese,
- Daten aus einem voroperativen Aufklärungsgespräch (*Pre-OP*),
- Daten die während einer Operation erhoben wurden (beispielsweise während einer Anästhesie),
- Daten, die in einem PACU (Post Anaesthesia Care Unit, Aufwachraum etc.) erhoben wurden und/oder
- individuellen anthropometrischen Patientendaten (beispielsweise Gewicht, Alter, Größe, Geschlecht etc.)

In einigen Ausführungsformen können alternativ oder ergänzend subjektive therapeutische Beobachtungen, z. B. mittels eines Schmerzscore wie hierin beschrieben, eingegeben werden und in Betracht gezogen werden.

In manchen Ausführungsformen ist die elektronische Steuervorrichtung weiter programmiert, um einen Vorbestimmungszeitpunkt einer Applikationsdosis der Substanz vorbestimmen zu können, indem alternativ oder ergänzend objektive therapeutische Beobachtungen in Betracht gezogen werden. Diese Beobachtungen können, vorzugsweise automatisch, mittels Sensordaten für Vitalwerte abgeglichen werden. Die Vitaldaten können beispielsweise umfassen:
- Herzfrequenz (HR),
- HRV,
- (partielle) Sauerstoffsättigung im Blut (SpO₂),
- Blutdruck,
- Blood pressure trending (BPT),
- elektrochemische Hautreaktionen,
- Temperatur,
- Photoplethysmogramm (PPG),
- Elektrokardiogramm (EKG),
- Atemfrequenz,
- Blutzuckerspiegel,
- Stress,
- Kohlenstoffmonoxid (CO)-Messung
- Elektroenzephalografie (EEG).

In einigen Ausführungsformen besteht die Möglichkeit, dass mittels der Steuervorrichtung veranlasste Verfahren zu personalisieren, anzupassen, zu modifizieren etc. Dies kann mittels eines User-Interface, Verwaltungssoftware und/oder über Peripheriegeräte erfolgen.

In manchen Ausführungsformen erstellt das mittels der Steuervorrichtung veranlasste Verfahren Wertetabellen zur Steuerung des Nasenapplikators.

In einigen Ausführungsformen berechnet das mittels der Steuervorrichtung veranlasste Verfahren je nach Lage des Gerätes unterschiedliche Auslöseparameter für den Nasenapplikator.

In manchen Ausführungsformen können der circadiane, ultradiane und/oder infradiane Rhythmus des Benutzers/Patienten Berücksichtigung finden.

In einigen Ausführungsformen überträgt der Nasenapplikator die Daten aus dem Datenspeicher kabelgebunden, beispielsweise mittels LAN, Powerline oder PowerLAN, und/oder drahtlos mittels entsprechender Protokolle, z. B. Wi-Fi, Bluetooth, WLAN, GPS, RFID, NFC, Barcode, QR-Code, ZigBee, Wibree, WiMAX, IrDA, WPAN, Infrarot etc. und/oder steht mit dem Datenspeicher in Signalverbindung.

Wenn hierin von einer Signal- oder Kommunikationsverbindung zweier oder mehrerer Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebunden, kabellosen oder auf andere Weise umgesetzte) Signalkommunikation besteht, beispielsweise durch eine Kopplung beider Komponenten, etwa mittels *pairing*, usw.

Unter *pairing* versteht man einen Prozess, um eine Verknüpfung zwischen Elektronikeinheiten zum Zwecke der Kommunikation herzustellen. Eines der bekanntesten Beispiele hierfür ist das Herstellen einer Bluetooth-Verbindung, mittels welcher verschiedenen Einrichtungen (z. B. Smartphone, Kopfhörer) miteinander verbunden werden. *Pairing* wird gelegentlich auch als *bonding* bezeichnet.

In manchen Ausführungsformen werden die hierin aufgeführten Daten mittels einer Schnittstelle zwischen Smart Device und Rechner übermittelt.

In einigen Ausführungsformen werden die hierin aufgeführten Daten webbasiert übermittelt, d. h. es ist keine Softwareinstallation notwendig.

In manchen Ausführungsformen werden die hierin aufgeführten Daten mittels einer verschlüsselten Datenübertragung übermittelt.

In einigen Ausführungsformen werden die hierin aufgeführten Daten vorzugsweise über eine einstellbare Reichweite hinweg übermittelt.

In manchen Ausführungsformen sind für den Nasenapplikator und die hiermit verbundenen Vorrichtungen vorzugsweise Low-Energy Lösungen vorgesehen, um möglichst wenig Strom zu verbrauchen.

In einigen Ausführungsformen ist für den Nasenapplikator und die hiermit verbundenen Vorrichtungen ein Flottenmanagement vorgesehen, d. h. ein Verwalten, Planen, Steuern und Überwachen von mehreren Nasenapplikatoren mit den verbundenen Vorrichtungen.

In manchen Ausführungsformen besteht eine Integrationsmöglichkeit des Nasenapplikators inklusive der hiermit verbundenen Vorrichtungen in eine bestehende IT-Infrastruktur. Es können hierzu Schnittstellen, z. B. mit klinischen Geräten und/oder mit anderen Medizinprodukten und/oder mit Diagnostikvorrichtungen und/oder mit einem Labor etc. vorgesehen sein.

In einigen Ausführungsformen können Schnittstellen zu elektronischen Patientenakten und/oder zu Krankenhaus Qualitätsberichten und/oder zu nationalen, europäischen und internationalen Datenerhebungsinitiativen bzw. Datenbanken vorgesehen sein. Somit kann auf Daten von z. B. interner und externer Forschung, auf interne und externe Umfragedaten (z. B. Patientenzufriedenheit, Nebenwirkungen etc.) zugegriffen werden. Innerhalb der Europäischen Union ist beispielweise die internationale statistische Klassifikation der Krankheiten und verwandter Gesundheitsprobleme (*International Statistical Classification of Diseases and Related Health Problems* (ICD-10) von Interesse, innerhalb Deutschlands können die diagnosebezogene Fallgruppierungen (*Diagnosis Related Groups* (DGRG)) sowie die Qualitätsverbesserung in der postoperativen Schmerztherapie (QUIPS) Vergleichsdaten liefern und in den USA die Programme der *Risk Evaluation and Mitigation Strategies* (REMS), die DEA-Überwachungsprogramme bzw. die DEA-Dokumentation.

In manchen Ausführungsformen ist ein modulares/erweiterbares Dock oder eine Docking Station für den Nasenapplikator oder die elektronische Steuereinheit hiervon und/oder weitere Peripheriegeräte vorhanden. Das Dock oder die Docking Station dient zur Aufbewahrung und/oder Verwaltung des Nasenapplikators und/oder von Peripheriegeräten. Das Dock oder die Docking Station ist für die Aufbewahrung und/oder Verwaltung des Applikators und/oder von Peripheriegeräten geeignet. Das Dock kann z. B. zum Laden, für Software Updates, Pairing, Hardware Checks und/oder Freigaben usw. vorgesehen sein.

Das Dock oder die Docking Station zählt in manchen Ausführungsformen ebenfalls als Peripheriegerät. Optional dient das Dock oder die Docking Station in einigen Ausführungsformen jedoch nicht zum Datentransfer zwischen Dock oder Docking Station einerseits und einem mit ihr körperlich in Kontakt stehenden Nasenapplikator und/oder einem Server oder Cloudserver anderseits, in anderen hingegen schon.

In einigen Ausführungsformen sind das Dock oder die Docking Station als eigenständige IT-Infrastruktur, z. B. im Internet oder Intranet (via Hub/Router/Switch), vorgesehen. Alternativ oder ergänzend ist das Dock über entsprechende Schnittstellen in eine bestehende IT-Infrastruktur integrierbar und/oder integriert.

In einigen Ausführungsformen der vorliegenden Erfindung sind die Datenschnittstellen verkabelt, z. B. ausgestaltet als USB, USB-C, Thunderbolt, LAN, etc.

In manchen Ausführungsformen kann vorgesehen sein, mittels z. B. IBM Watson Zugang zu ausgelagerter Systemintelligenz zu schaffen und somit Ankopplung an bestehende Krankenhausinformationssysteme (KIS/PACS) über gängige Schnittstellen wie HL7 und DICOM bereitzustellen. Beispielsweise kann somit einem Techniker Zugriff auf den Nasenapplikator, auf die mit dem Nasenapplikator verbundenen Vorrichtungen, auf Interfaces/Schnittstellen und/oder auf die Software ermöglicht werden.

In einigen Ausführungsformen ist ein zentraler Server vorgesehen.

In manchen Ausführungsformen umfasst der Nasenapplikator, insbesondere dessen elektronische Steuervorrichtung, eine Energiespeichervorrichtung, insbesondere einen aufladbaren Akku oder eine Batterie.

In einigen Ausführungsformen kann die Energiespeichervorrichtung eine anwendungsfall- und/oder indikationsbezogene Batterieauslegung aufweisen. In diesen Ausführungsformen kann je nach Indikation eine unterschiedlich dimensionierte Energiespeichervorrichtung vorgesehen sein, insbesondere Energiespeichervorrichtungen mit unterschiedlicher Lebensdauer bzw. unterschiedlichen Laufzeiten. So kann postoperativ beispielsweise eine Energieversorgung von drei Tagen ausreichend sein, während bei der Behandlung von Migräne eine längere Energieversorgung (z. B. über zwei Wochen) vorteilhaft sein kann.

In manchen Ausführungsformen kann ein kabelloses Laden des Energiespeichers vorgesehen sein, beispielsweise mittels Pins (z. B. in einer Ladeschale) oder ohne Pins (z. B. Qi, mittels induktiver Energieübertragung).

In einigen Ausführungsformen kann ein verkabeltes Laden des Energiespeichers vorgesehen sein, beispielsweise mittels eines Netzteils oder mittels Power over Ethernet (PoE).

In manchen Ausführungsformen können als Energiespeicher Batterien zum Wechseln vorgesehen sein, z. B. Einwegbatterien (Knopfzellen, AA, AAA, AAAA, Blockbatterien, etc.), wiederaufladbare Batterien, wiederaufladbare Batteriepacks, etc.

In einigen Ausführungsformen kann als Energiespeicher ein externer Stromanschluss vorgesehen sein oder PoE verwendet werden.

In manchen Ausführungsformen kann eine Solarzelle die notwendige Energie liefern. Die Solarzelle kann direkt am Nasenapplikator oder z. B. an einem Ladedock oder einer Ladestation vorgesehen sein.

In einigen Ausführungsformen kann die notwendige Energie mittels eines Aufziehmechanismus, beispielsweise wie bei einem Uhrwerk, oder mittels sogenanntem "energy harvesting", d. h. beispielsweise durch ein- oder mehrmaliges Drücken eines Knopfs oder eines Hebels, erzeugt werden. Die so gewonnene Energie kann sofort oder später für das Applizieren von Applikationsdosen genutzt werden.

In manchen Ausführungsformen ist das Substanzreservoir des Nasenapplikators integral vorgesehen, in anderen Ausführungsformen nicht integral, insbesondere Co-Packed, d. h. mit dem Nasenapplikator und/oder einer zugehörigen Komponente mitverpackt. Sowohl in den integralen als auch in den nicht integralen Ausführungsformen ist das Substanzreservoir (wieder-)befüllbar, insbesondere von außen.

In einigen Ausführungsformen ist das Substanzreservoir ein bereits vorgefülltes Einwegbehältnis und/oder ein vor Ort befüllbares Einwegbehältnis, z. B. gefertigt aus Glas, Kunststoff, Metall, Nicht-Eisen Metall, Verbundwerkstoffen, etc. oder aus einer Kombination dieser Materialien.

In manchen Ausführungsformen ist das Substanzreservoir vorgesehen mit einer positionsunabhängigen Auslöseeinrichtung, d. h. ein Auslösen kann mit einer Auslösefunktionalität in allen Achsen und Freiheitsgraden erfolgen. Dies kann beispielsweise durch ein kollabierbares und/oder ein energiebeaufschlagtes Behältnis und/oder dergleichen realisiert werden.

In einigen Ausführungsformen hat das Substanzreservoir ein fixes, in anderen Ausführungsformen ein variables Füllvolumen.

In manchen Ausführungsformen kann das Substanzreservoir konservierungsmittelfrei, in anderen Ausführungsformen mit konservierungsmittelhaltigen Substanzen befüllbar sein und/oder dafür geeignet sein.

In einigen Ausführungsformen ist das Substanzreservoir mit wenigstens einer Konnektierungsvorrichtung versehen, welche dazu dient das Substanzreservoir mit dem Abgabedosierer, insbesondere lösbar, zu verbinden. Dies kann mittels einer Luerverbindung, einer Nadel, einem Schraubverschluss, einer Steckverbindung, einer Klickverbindung, mittels Verpressen oder dergleichen erfolgen. Ferner können zur Verbindung auch mechanische Pins und/oder ein Formschluss von der vorliegenden Erfindung umfasst sein.

In manchen Ausführungsformen kann am Substanzreservoir eine Einrichtung, insbesondere ein Barcode/QR-Code und/oder eine RFID- Einrichtung, zur Erfassung und/oder Speicherung von ausgebbaren Informationen zur Rückverfolgbarkeit einer oder mehrerer Applikationen, vorgesehen sein. Diese können ferner z. B. eine Beschriftung, ein QR-Code, ein Chip, ein RFID/NFC-Tag, eine Bildaufnahme, welche jeweils eingescannt werden können, etc. oder eine Kombination hiervon sein oder umfassen. Applikationen sind beispielsweise Informationen über die Substanz wie Name, Verfallsdatum, pharmakologische Daten, Konzentration usw.

In einigen Ausführungsformen können am Substanzreservoir zur Fehlervermeidung im Sinne des Poka-Yoke- oder Schlüssel-und-Schloss-Prinzips technische Lösungen zum Einsatz kommen. Hierzu können beispielsweise eine Beschriftung, ein QR-Code, ein Barcode, ein Chip, ein RFID/NFC-Tag, eine Bildaufnahme, welche jeweils eingescannt werden können, etc. oder auch eine Kombination hiervon am Substanzreservoir vorgesehen sein. Entsprechende Steuer- und/oder Rechenvorrichtungen mit geeigneter Software können hierzu ebenfalls von der vorliegenden Erfindung umfasst sein. Damit kann u. a. vorteilhaft eine unsachgemäße Handhabung und/oder das Einsetzen eines falschen Substanzreservoirs in den Nasenapplikator vermieden werden. Mechanische Pins und/oder ein entsprechender Formschluss können ebenso diesem Ziel dienen und sind deshalb ebenfalls von der vorliegenden Erfindung umfasst.

In manchen Ausführungsformen ist das Substanzreservoir ein nicht-integrales, vorgefülltes Einwegbehältnis z. B. aus Glas, Kunststoff, Metall, Nicht-Eisen Metall, Verbundwerkstoffen, etc. oder einer beliebigen Kombination hiervon, welches getrennt vom Nasenapplikator, insbesondere von dessen Abgabedosierer, gelagert wird. Insbesondere werden hierdurch Kompatibilitätsprobleme zwischen dem/den Material(ein) des Substanzreservoirs der Substanz vermieden. Durch die getrennte Lagerung kann das Risiko, dass extrahierbare (*Extractables*) oder auslaugbare Stoffe (*Leachables*) in die Substanz migrieren, reduziert oder gar vermieden werden.

In manchen Ausführungsformen ist das Substanzreservoir ein nachfüllbares, integriertes Mehrwegbehältnis mit Filter, in anderen Ausführungsformen ohne Filter.

In einigen Ausführungsformen ist das Substanzreservoir ein externes Mehrwegbehältnis mit Filter, in anderen Ausführungsformen ohne Filter.

In manchen Ausführungsformen umfasst der Nasenapplikator ein zusätzliches Substanzreservoir, insbesondere ein Flüssigkeitsreservoir, in welchem eine weitere Substanz aufgenommen sein kann. Vorzugsweise unterscheidet sich die weitere Substanz von der ersten Substanz. Die weitere Substanz kann medizinisch oder nicht medizinisch sein, z. B. Hyaluronsäure, Ectoine, Aloe Vera, usw.

In einigen Ausführungsformen umfasst der Nasenapplikator oder sein Substanzreservoir mehrere Fluidkammern, beispielsweise die vorstehend genannte zweite Fluidkammer. In solchen Ausführungsformen kann der Nasenapplikator dazu geeignet sein, mehrere Therapien, z. B. Schmerzmittel oder Cannabinoide einerseits und z. B. und Insulin andererseits, zu unterstützen. In bestimmten Ausführungsformen kann vorgesehen sein, mit einem Fluid aus der zweiten Fluidkammer und/oder im Substanzreservoir die Substanz in der zweiten Fluidkammer und/oder im Substanzreservoir zu neutralisieren oder zu zerstören, oder umgekehrt. Dies kann insbesondere bei Diebstahl oder Missbrauch von Vorteil sein. Hierfür kann in der zweiten Fluidammer und/oder im Substanzreservoir beispielsweise ein Trocknungsmittel vorgesehen sein, welches die Substanz und damit der in ihr enthaltene Wirkstoff durchläuft (ähnlich dem Prinzip eines Filters oder Kaffeefilters) oder von welchem der Wirkstoff oder die Substanz aufgesaugt wird (beispielsweise wie von einem Schwamm) und das Trocknungsmittel die Substanz oder den Wirkstoff (z. B. dessen Salz) bindet.

Das Trocknungsmittel kann z. B. in einem Filter, der zweiten Fluidkammer oder einem Schwamm, z. B. als Granulat, vorgesehen sein.

Das Trocknungsmittel kann auch anderswo im Nasenapplikator vorgehalten werden, vorzugsweise so, dass die Substanz, welche den Wirkstoff enthält, das Trocknungsmittel auf Wunsch durchläuft oder darin gespeichert wird oder zum Liegen kommt.

Trocknungsmittel können z. B. Natriumsulfat, Calciumsulfat oder Calciumchlorid sein.

Als Filter können z. B. ein Molekularsieb, ein Filtervlies oder ein Ionentauscher vorgesehen sein.

Trocknungsmittel, insbesondere wie vorstehend diskutiert, kann ergänzend oder alternativ auch an anderer Stelle im Gehäuse angeordnet sein. Also nicht beschränkt auf die zweite Kammer und/oder das Substanzreservoir.

In manchen Ausführungsformen umfasst der Nasenapplikator eine Kühlvorrichtung, geeignet um ein, mehrere oder alle Substanzreservoir(s), insbesondere dessen Inhalt, zu kühlen.

In bestimmten Ausführungsformen umfasst der Nasenapplikator eine Heizvorrichtung, geeignet um ein, mehrere oder alle Substanzreservoir(s), insbesondere dessen Inhalt oder deren Inhalte, zu heizen. Dies ist insbesondere vorteilhaft, um ein Einfrieren der Substanz zu verhindern oder um die Degeneration/Zerstörung der Substanz durch Kälte zu verhindern.

In einigen Ausführungsformen kann die Steuervorrichtung als Einwegvorrichtung vorgesehen sein, in anderen Ausführungsformen als wiederverwendbare elektronische Steuervorrichtung. Auch hybride Ausführungsformen, bei denen ein Teil der Steuervorrichtung wiederverwendbar und ein Teil als Einwegbauteil vorgesehen ist, sind von der vorliegenden Erfindung umfasst.

In manchen Ausführungsformen sammelt, speichert, überwacht, verarbeitet, berechnet, simuliert, regelt und/oder steuert die Steuervorrichtung eine, mehrere oder alle Funktionalitäten des Nasenapplikators.

In einigen Ausführungsformen wird der Nasenapplikator mittels der Steuervorrichtung angesteuert durch einen auf den Nasenapplikator aufgespielten Algorithmus (*Embedded Software*)*.*

In manchen Ausführungsformen kann der Abgabedosierer mittels der Steuervorrichtung separat angesteuert werden oder zusammen mit der Dosiserfassungsvorrichtung.

In einigen Ausführungsformen kann die Dosiserfassungsvorrichtung mittels der Steuervorrichtung separat angesteuert werden oder zusammen mit dem Abgabedosierer.

In manchen Ausführungsformen wird der Nasenapplikator mittels der Steuervorrichtung angesteuert durch einen dezentral gespeicherten Algorithmus, z. B. webbasiert, über einen Stationsrechner oder Smart Devices (*Non-Embedded Software*)*.*

In einigen Ausführungsformen steht die Steuervorrichtung mit weiteren Vorrichtungen kabellos oder kabelgebunden in Signalkommunikation, beispielsweise um mit Peripheriegeräten interagieren zu können, entsprechende Sende- und/oder Empfangsvorrichtungen können vorgesehen sein. Sie können mit der Steuervorrichtung in Signalverbindung stehen. Zu den Peripheriegeräten können u. a. zählen:
- Vorrichtungen zur Patientenauthentifizierung,
- Armbänder,
- Sensoranordnungen,
- Docking Stations,
- Rechner (PC),
- Smartgeräte,
- Drucker,
- andere medizinische Geräte,
- Vorrichtungen in einem Rechenzentrum,
- Hub, Router, Accesspoint,
- Server,
- (Cloud)Server.

Das erfindungsgemäße System kann eines oder mehrere der vorstehend genannten und/oder weiterer Peripheriegeräte aufweisen.

In einigen Ausführungsformen fungieren Peripheriegeräte des Nasenapplikators als Funkzellen für ein Ortungssystem bzw. zur Generierung eines Geofence.

Ein Geofence ist ein virtueller Zaun bzw. eine virtuelle Grenze, die einen physischen Standort "umgibt". Wie ein realer Zaun trennt ein Geofence den jeweiligen Standort von der Umgebung ab - wenn auch in digitaler Art und Weise anstatt in Form einer physischen Barriere. Im Gegensatz zu einem echten Zaun kann ein Geofence optional allerdings auch Bewegungen innerhalb der "Einzäunung" erkennen.

Ein solcher virtueller Zaun kann eine beliebige Größe, Form oder Peripheriegeräte Koppelung haben. Sogar eine gerade Linie zwischen zwei Punkten kann möglich und vorgesehen sein.

Geofences werden z. B. mit Hilfe einer Kartensoftware erstellt und ermöglichen dem Benutzer, den virtuellen Zaun direkt über die gewünschte Fläche zu zeichnen und/oder mittels GPS-, W-LAN-, WPAN-, RFID-, Bluetooth- Signalen und/oder mit anderen Peripheriegeräten eine Koppelung usw. zu erstellen. Die digitale Grenze besteht z. B. aus einer Anzahl von Koordinaten für Breite und Länge - und bei einem kreisförmigen Geofence lediglich aus einem Punkt, der das Zentrum bildet mittels W-LAN-, WPAN-, RFID-, Bluetooth-Signalen und/oder mit anderen Peripheriegeräten eine Koppelung usw.

Geofences lassen sich dazu verwenden, Objekte innerhalb des virtuellen Zaunes z. B. per mittels W-LAN-, WPAN-, RFID-, Bluetooth- Signalen und/oder andere Peripheriegeräte Koppelung etc.-Tracking zu überwachen und/oder bestimmte Aktion für einen Bereich definieren und/oder zu aktivieren. Auf diese Weise erkennt man zum Beispiel sofort, wenn ein Gerät wie die erfindungsgemäße Medikamentenabgabevorrichtung, insbesondere der Nasenapplikator, von seinem Einsatzort entfernt wird. Umgekehrt können virtuelle Zäune auch dazu eingesetzt werden, Objekte aus einem bestimmten Bereich herauszuhalten.

In manchen Ausführungsformen kann die Elektronik der Steuervorrichtung redundant ausgelegt werden, z. B. zweikanalig, per Überwachungssystem/Watchdog, etc. Dies kann insbesondere vorteilhaft dazu beitragen, eine sichere Funktionsweise zu gewährleisten, damit die Sicherheit zu erhöhen und den verschiedenen Risikoklassen in der Medizintechnik gerecht zu werden.

In manchen Ausführungsformen umfasst die Abrufvorrichtung des Nasenapplikators einen Auslösemechanismus, der seinerseits wenigstens einen der im Folgenden beschriebenen Merkmale umfasst.

So kann der Auslösemechanismus rein oder im Wesentlichen mechanisch arbeiten, z. B. ähnlich wie bei einem Insulin-Pen oder einem Respimat^{®} Soft-Mist-Inhaler.

Alternativ kann der Auslösemechanismus eine Kombination aus Mechanik (Auslösemechanismus) z. B. Herzkurve, Stellvorrichtung und/oder Übersprung und Elektronik (Ansteuerung) sein.

In einigen Ausführungsformen kann der Auslösemechanismus einen unidirektionalen, bidirektionalen, multidirektionalen, rotations-, schritt- und/oder encodergesteuerten und/oder stufenlosen Kolben und/oder eine Stellvorrichtung, etc. sein oder umfassen.

Ergänzend hierzu kann in manchen Ausführungsformen ein gegenläufiger Dosiereinstellmechanismus (vgl. Insulin Pen) vorgesehen sein. Alternativ ist auch eine elektronische Dosierung (vgl. elektronischer Insulin Pen) von der vorliegenden Erfindung umfasst.

In manchen Ausführungsformen umfasst der Auslösemechanismus eine Pumpe, z. B. eine Mikropumpe, Kolbenpumpe, Rotationspumpe, Peristaltikpumpe, Kreiselpumpe, Fliehkraftpumpe oder dergleichen.

In einigen Ausführungsformen basiert der Auslösemechanismus auf einem bidirektionalen Zweitakt-Prinzip (ansaugen/drücken), z. B. gemäß des Funktionsprinzips des Respimat^{®}s.

In manchen Ausführungsformen können der die Ausbringvorrichtung oder der Auslösemechanismus eine Mikrodosierung veranlassen, z. B. im Bereich von 1 µL bis 300 µL.

In einigen Ausführungsformen kann die Ausbringvorrichtung oder der Auslösemechanismus programmiert oder konfiguriert sein, um eine Dosierung oder Dosis pro Zeiteinheit, also z. B. 100 µL/sec, auszubringen.

In einigen Ausführungsformen kann eine abgegebene Menge (Applikationsdosis) leicht und/oder genau reproduziert werden, da die Menge bzw. der Umfang der Applikationsdosis mittels der vorliegenden Erfindung unabhängig von einer Betätigung durch den Benutzer/Patienten sind.

Eine hohe Reproduzierbarkeit und Genauigkeit der abgegebenen Menge durch eine user-unabhängige Auslösung mit stets gleichen Auslöseparametern, z. B. Kraft, Geschwindigkeit, Beschleunigung, Druck, Flussrate, Hub/Weg, genereller Energieeintrag kann vorteilhaft zur Patientensicherheit beitragen.

In manchen Ausführungsformen kann der Auslösemechanismus eine Auslösung durch einen mechanischen oder elektronischen Impulsgeber veranlassen. In diesen Ausführungsformen ist keine Krafteinbringung in das System nötig.

In einigen Ausführungsformen ist die Applikationsdosis der Substanz einstellbar, insbesondere variabel, insbesondere mittels Ausgestaltungen des Auslösemechanismus, wie hierin beschrieben.

In einigen Ausführungsformen befindet sich die Substanz in einer Druckkartusche.

In manchen Ausführungsformen wird die Tröpfchenwolke mittels "vibrating mesh", "piezo", porösen Materialien und/oder Verdampfung erzeugt. Die Ausbringmenge kann über die Zeit oder eine vordosierte Menge festgelegt werden, welche dann zerstäubt wird.

In einigen Ausführungsformen umfasst der Auslösemechanismus ein Dosierventil, welches beispielsweise solange offen bleibt, wie es gedrückt gehalten wird. Auch ein Dosierventil mit Dosierkammer, ein zeitgesteuertes Ventil, oder ein durchflussgesteuertes Ventil, etc., ist von der vorliegenden Erfindung umfasst.

In einigen Ausführungsformen ist der manuell verstellbare Mechanismus zum Verändern des Fassungsvolumens der Dosierkammer z. B. mittels eines Drehknopfs, als Ratsche oder dergleichen ausgebildet, mit z. B. einer Zahlenskala oder Angaben zur Abgabemenge, Dosis, etc. Dabei kann der Anschlag der Ratsche gegen einen Energiespeicher, z. B. eine Feder oder ein elastisches Element, wirken oder nicht. Die Auslösung bzw. Ausbringung wird z. B. manuell analog gleich einem klassischen Nasenspray ausgelöst. Nach erfolgter Applikation rutscht, beispielsweise über eine Schlossmutter oder dergleichen, die Ratsche in die neue Ausgangsposition des Stopfens nach. Die Ausbringvorrichtung kann mit dem Stopfen verbunden sein oder nicht, z. B. durch einen Kolben.

In manchen Ausführungsformen ist der Mechanismus manuell verstellbar, in anderen automatisch oder kombiniert manuell und automatisch oder teilautomatisch. Dies gilt für jede unter die Erfindung fallende Ausführungsform des Mechanismus.

In einigen Ausführungsformen kann die Abrufvorrichtung seitlich angeordnet sein.

In manchen Ausführungsformen kann die "Ratsche" eine herkömmliche Ratsche sein. Sie kann ein gegenläufiges Gewinde aufweisen. Dies kann wiederum auf jeden hierin genannten Mechanismus zutreffen.

In einigen Ausführungsformen kann der Mechanismus zum Verändern des Fassungsvolumens der Dosierkammer gleichzeitig einen Energiespeicher, beispielsweise in Form eines Federspeichers, laden und mittels der Abrufvorrichtung, welche den Energiespeicher freisetzt, applizieren.

In manchen Ausführungsformen kann die Dosiseinstellung bzw. die Dosisapplikation eine Kombination aus manuellem und automatischem Vorgehen sein.

In einigen Ausführungsformen stellt das Substanzreservoir zugleich die Dosierkammer dar. Dies bedeutet, dass aus dem Substanzreservoir heraus direkt oder indirekt über den Nasenaufsatz appliziert wird, ohne dass die zu applizierende Applikationsdosis zunächst in einer vom Substanzreservoir fluidisch zumindest vorübergehend separierten Kammer oder Dosierkammer aufgenommen werden würde. Eine eigenständige Bestückungsvorrichtung ist in diesen Ausführungsformen somit nicht erforderlich.

Auf diese Weise kann vorteilhaft sichergestellt werden, dass die Ausbringvorrichtung immer in dieselbe Richtung bewegt, dreht, fördern, was erlaubt, das unausweichliche Spiel auf einfachere Weise auszugleichen, als wenn eine Links-RechtsBewegung, eine Auf-Ab-Bewegung ausgeführt würde.

Basierend auf Messergebnissen eines optionalen Flusssensors oder einer optionalen Vorrichtung zur Mengenerfassung, etc. in jeglicher Art kann in manchen Ausführungsformen der Motor zum Ausbringen gesteuert werden, beispielsweise indem einfach die Stromzufuhr abgeschaltet oder dem Controller ein entsprechendes Signal, insbesondere zum Abschalten, gegeben wird.

In einigen Ausführungsformen ist eine andere Ausgestaltung vorgesehen, bei welcher man z. B. manuell die Applikationsdosis bzw. ihre Menge einstellt. Beispielsweise wird manuell ein Energiespeicher gespannt und manuell ausgelöst.

In manchen Ausführungsformen kann die Ausbringvorrichtung einen separaten Stopfen vorantreiben, alternativ kann der Stopfen in die Ausbringvorrichtung integriert sein.

In einigen Ausführungsformen, insbesondere bei einem Einweggerät, können Stopfen und Bestückungsvorrichtung unlösbar miteinander verbunden und/oder ineinander integriert sein. In anderen Ausführungsformen, insbesondere bei einem Mehrweggerät, können Stopfen und Bestückungsvorrichtung zwei Teile sein, oder die Spindel und der Stopfen können voneinander gelöst werden.

In manchen Ausführungsformen kann der Nasenaufsatz integral mit dem Gehäuse ausgestaltet sein, oder mit diesem verbunden, etwa aufgesteckt, aufgeschraubt oder dergleichen. Hierzu kann eine Verbindungsstelle vorgesehen sein. Sie kann innerhalb oder außerhalb eines umschlossenen Lumens des Gehäuses angeordnet sein.

In einigen Ausführungsformen weist die Ausbringvorrichtung einen Kolben mit einem endseitigen Stopfen auf, welcher in das Substanzreservoir einfährt, welches in diesen Ausführungsformen gleichzeitig als Dosierkammer dienen kann.

In manchen Ausführungsformen ist ein Stellmotor angeordnet, um einen Anschlag zu verschieben bzw. einzustellen, etwa mittels einer Spindel oder Stellschraube.

In einigen Ausführungsformen begrenzt der Anschlag, welcher alternativ vorgesehen sein könnte, manuell eingestellt zu werden, den Weg eines Auslösemechanismus. Der Auslösemechanismus kann in diesen Ausführungsformen als Teil der Ausbringvorrichtung verstanden werden.

In manchen Ausführungsformen kann vorgesehen sein, dass der Auslösemechanismus mittels Federspeichers einer Feder oder eines anderen Energiespeichers vorgespannt und im vorgespannten Zustand ausgelöst wird, z. B. manuell. Der Auslösemechanismus kann alternativ durch einen Motor angetrieben und/oder ausgelöst werden.

In einigen Ausführungsformen kann der Anschlag durch einen Außenring mit Innengewinde ausgestaltet sein, welcher entlang einer zylindrischen Führung mit Außengewinde verschiebbar vorgesehen sein kann.

In manchen Ausführungsformen kann der Anschlag per Motor und/oder manuell verschiebbar oder verstellbar vorgesehen sein.

In einigen Ausführungsformen kann die Abrufvorrichtung in Form eines endseitigen Druckknopfs ausgestaltet sein, der gegen eine Feder gedrückt wird.

In manchen Ausführungsformen kann ein Ventil ein Rückschlagventil sein oder ein Ventil, das erst bei einem bestimmten Druck öffnet, z. B. ab 4 bar Druck, und nach Druckabfall unter 4 bar wieder schließt.

In einigen Ausführungsformen kann am Ende der Dosierkammer oder nach der Verbindungsstelle ein Filter vorgesehen sein.

In manchen Ausführungsformen wird die Dosierkammer mittels einer schiefen Ebene eingestellt. Hierzu kann ein geeigneter Mechanismus vorgesehen sein. Die Auslösung und Ausbringung kann analog gleich wie bei einem klassischen Nasenspray erfolgen. Eine optionale Einrichtung wie eine Rückstellfeder kann die Ausbringvorrichtung und/oder andere Komponenten erneut in deren Ausgangslage bringen.

In einigen Ausführungsformen kann die Einstellung/Verstellung des Mechanismus manuell oder automatisch per z. B. Motor erfolgen. Die Bewegung kann dabei rotational oder linear sein.

In manchen Ausführungsformen kann das Substanzreservoir mit nachlaufenden Stopfen, einer Beutelflasche, einem "Bag", einem Reservoir mit Steigrohr etc., ausgestaltet sein.

In einigen Ausführungsformen kann der Mechanismus zum Verändern des Fassungsvolumens der Dosierkammer im Nasenaufsatz integriert sein oder vor oder nach der Anschlussstelle liegen.

In manchen Ausführungsformen ist der Mechanismus zur Veränderung des Fassungsvolumens die Dosierkammer selbst.

In manchen Ausführungsformen weist der Stopfen den Kolben auf oder besteht hieraus, welcher durch Gas, welches in einem Druckbehälter unter Druck steht, in das Substanzreservoir getrieben wird. Beim Betätigen oder Herunterdrücken der Abrufvorrichtung wird eine Applikationsdosis, wie sie in der Dosierkammer bereitgestellt ist, abgegeben.

In einigen Ausführungsformen wird die Dosierkammer mittels schiefer Ebenen oder Ähnlichem, wie z. B. Anschlägen und dergleichen, eingestellt. Beim manuellen Auslösen der Abrufvorrichtung schiebt das Gas den Kolben mit dem Stopfen vor und in das Substanzreservoir hinein. Auch in diesen Ausführungsformen kann das Einstellen und Auslösen manuell oder automatisch erfolgen.

In manchen Ausführungsformen können die schiefen Ebenen z. B. linear oder kreisförmig (kurvenförmig) angeordnet sein.

In manchen Ausführungsformen wird die Dosierkammer bzw. ihr Volumen per schiefer Ebene(n) und Motor eingestellt, was alternativ per Hand erfolgen könnte. Der Federspeicher wird in diesen Ausführungsformen manuell geladen bzw. vorgespannt, dies könnte alternativ mittels Motors oder anderer Energiequellen erfolgen.

In einigen Ausführungsformen wird die Auslösung mittels eines Federspeichers umgesetzt. In diesen Ausführungsformen kann der Hub automatisch oder manuell eingestellt werden und/oder die Auslösung z. B. manuell stattfinden, beispielsweise indem der Federspeichermechanismus manuell oder automatisch rotiert wird.

In manchen Ausführungsformen kann diese Rotation mittels eines Motors realisiert werden.

In einigen Ausführungsformen ist der Mechanismus zum Verändern des Fassungsvolumens der Dosierkammer für die Applikationsdosis der Substanz verstellbar.

In einigen Ausführungsformen des Nasenapplikators weist dieser einer Pumpe auf, insbesondere eine Mikropumpe, die vorzugsweise nicht direkt mit der Wirbelkammer, welche im Nasenaufsatz vorliegen kann, gekoppelt ist, sondern die Dosierkammer mit der gewünschten Ausbringmenge oder Applikationsdosis aus dem Substanzreservoir heraus befüllt.

In manchen Ausführungsformen befüllt die Pumpe die Dosierkammer. In manchen Ausführungsformen kann die Pumpe direkt mit der Verbindungsstelle bzw. dem Nasenstück verbunden sein.

In einigen Ausführungsformen kann die Dosierkammer zusätzlich auch manuell, mittels z. B. Ratsche, oder automatisch, z. B. mittels Motors, verstellt werden. Die Pumpe schaltet z. B. nach Erreichen eines bestimmten Druckes oder Zeit ab oder wird gestoppt oder abgeschaltet.

In manchen Ausführungsformen kann hierzu ein Flusssensor eingesetzt werden, welcher die Pumpe ansteuert.

In einigen Ausführungsformen verschiebt die in die Dosierkammer mittels Pumpe eingebrachte Substanz den die Dosierkammer begrenzenden Kolben unter Vergrößerung des Fassungsvolumens der Dosierkammer.

In manchen Ausführungsformen kann ein Rückschlagventil die Dosierkammer derart begrenzen, dass Substanz aus dem Substanzreservoir in die Dosierkammer einströmen kann, nicht aber über das Rückschlagventil auch wieder heraus.

In einigen Ausführungsformen kann die Pumpe, welche Teil der Bestückungsvorrichtung ist oder sein kann, mittels Motors oder alternativ per Hand betrieben werden z. B. mittels eines Drehknopfs oder eines Hebels. Die Zahlenangaben, die in diesen Ausführungsformen am Drehknopf vorgesehen sein können, können die eingestellte oder einstellbare Dosismenge angeben.

In einigen Ausführungsformen kann ein Federspeicher manuell oder mittels des Motors mittels einer Feder geladen oder gespannt werden. Wird dieser ausgelöst, so wird die Ausbringmenge durch die Wirbelkammer des Nasenaufsatzes gedrückt.

Ein Trigger kann in manchen Ausführungsformen vorgesehen sein. Er hat die Funktion der Abrufvorrichtung. Er kann beispielsweise das System aus dem Standby wecken und die nächste Dosis nachladen (also die Dosierkammer befüllen).

In einigen Ausführungsformen kann der Trigger mechanisch oder elektrisch ausgelegt sein. So kann mittels des Triggers eine Ratsche gelöst werden oder ein Signal an die elektrische Steuervorrichtung geschickt werden, welche dann einen Aktor löst.

In manchen Ausführungsformen dichtet ein Ventil, welches in das Rückschlagventil integriert sein kann, aber nicht muss, die Verbindungsstelle ab. Zum Befüllen der Dosierkammer ist dieses Ventil geschlossen und ein anderes Ventil geöffnet. Sobald sich die Ausbringvorrichtung bewegt, z. B. in Richtung Verbindungsstelle, schließt das Rückschlagventil. Nun sind alle Ventile zu und es baut sich Druck in der Kammer auf.

In einigen Ausführungsformen können eines oder mehrere der vorhandenen Ventile entweder elektrisch geschaltet werden, sich bei einem Mindestdruck öffnen und/oder über die Abrufvorrichtung manuell geöffnet werden.

In bestimmten Ausführungsformen kann die Abrufvorrichtung verschiedene Raststufen einnehmen, je nachdem, ob, oder abhängig davon wie weit (bis zu welchem Grad), die Dosierkammer gefüllt ist.

In manchen Ausführungsformen kann eine Feder vorgesehen sein, welche auf den Stopfen drückt.

In einigen Ausführungsformen weist das Gehäuse des Nasenapplikators einen Deckel auf, welcher in seinem geöffneten Zustand das Innere des Gehäuses freigibt, so dass z. B. das optional als Vial ausgestaltete Substanzreservoir aus dem Gehäuse entnommen und beispielsweise durch ein anderes ersetzt werden kann.

In manchen Ausführungsformen kann zum Herstellen einer Fluidverbindung zwischen der Dosierkammer und dem Substanzreservoir beispielsweise eine Nadel vorgesehen sein, welche z. B. ein Septum eines Vial durchsticht.

Die Pumpe kann wiederum durch einen Motor angetrieben werden, alternativ geschieht dies manuell.

In einigen Ausführungsformen können mittels eines Motors die Pumpe, der Mechanismus zum Verändern des Fassungsvolumens der Dosierkammer und/oder das Laden des Federspeichers betrieben werden. Der Motor kann dabei optional den Ausbringmechanismus oder die Ausbringvorrichtung antreiben. All diese Komponenten können vorgesehen und ausgestaltet sein, um in beliebiger Kombination manuell oder automatisch betrieben und/oder angesteuert zu werden.

In manchen Ausführungsformen ist an einem Endbereich des Kolbens (Ausbringvorrichtung) vorzugsweise ein Rückschlagventil vorgesehen, welches beim Ausbringen (Kolben geht Richtung Deckel) schließt. Am gegenüberliegenden Endbereich, d. h nahe der Verbindungsstelle, ist ein Ventil vorgesehen, welches schaltbar ist bzw. beim Befüllen der Dosierkammer durch die Pumpe geschlossen ist und, wenn sich der Kolben (Ausbringvorrichtung) auf dieses Ventil zu bewegt, öffnet. Dies kann man mittels eines schaltbaren Ventils (elektrisch oder mechanisch), welches beim Auslösen öffnet, umgesetzt sein oder werden. Dasselbe könnte auch mit einem Rückschlagventil umgesetzt werden, welches erst bei einem gewissen Druck, z. B. 4 bar, öffnet. Dann würde beim Befüllen der Dosierkammer durch die Pumpe dieses Ventil solange geschlossen bleiben, bis der Druck in der Dosierkammer über den Öffnungsdruck des Ventils - ähnlich wie bei einem Überdruckventil oder Druckzuschaltventil - angestiegen ist.

In einigen Ausführungsformen füllt die Pumpe die Dosierkammer, und mittels des sich ergebenden Druckanstiegs werden die Bestückungsvorrichtung und/oder die Ausbringvorrichtung von der Verbindungsstelle weggedrückt. Dabei wird z. B. ein Federenergiespeicher geladen. Die so gespeicherte Energie wird anschließend genutzt, um die Ausbringvorrichtung in Richtung Verbindungsstelle zu bewegen.

In manchen Ausführungsformen kann der Motor die Pumpe, die Bestückungsvorrichtung und/oder die Ausbringvorrichtung antreiben.

In einigen Ausführungsformen kann die Ausrichtung des Vials, um z. B. 180°, gedreht sein.

In manchen Ausführungsformen umfasst oder ermöglicht der Auslösemechanismus ferner eine reversible Pumprichtung (Ansaugen). Dies kann insbesondere z. B. für die Entsorgung und/oder Neutralisierung der Substanz bzw. des Wirkstoffs bei Missbrauch und/oder Diebstahl vorgesehen sein. So kann beispielsweise eine Luftpumpe oder ein anderer Fördermechanismus oder Aktor vorgesehen sein, welche den Stopfen des Substanzreservoirs aus dem Reservoir drückt und somit die Substanz mit dem Trocknungsmittel in Berührung kommt, wodurch Erstere von Letzterem gebunden werden kann.

In einigen Ausführungsformen hat das Gehäuse des Nasenapplikators die Funktion einer Abrufvorrichtung zum Betätigen des Auslösemechanismus.

In manchen Ausführungsformen ist der Nasenapplikator für die Bedienung durch Rechtshänder geeignet und/oder angepasst, in anderen für die Bedienung durch Linkshänder. In bestimmten Ausführungsformen ist er sowohl für die Bedienung durch Links- als auch für die Bedienung durch Rechtshänder geeignet und/oder angepasst.

In einigen Ausführungsformen sind die Auslösung des Auslösungsmechanismus und/oder die Höhe der dadurch bedingten Applikationsdosis unabhängig von einer Betätigung durch den Benutzer/Patienten.

In manchen Ausführungsformen des Nasenapplikators ist vorgesehen, dass der Benutzer/Patient die Höhe einer abzugebenden Applikationsdosis selbst einstellt. Dies kann beispielsweise basierend auf Berechnungen oder Anweisungen eines Arztes erfolgen. Diese können auf unterschiedliche Art und Weise dem Benutzer/Patienten mitgeteilt werden, beispielsweise über ein Smartphone oder dergleichen. In bestimmten Ausführungsformen kann zu diesem Zweck eine Software, insbesondere eine Applikation (App), beispielsweise für ein Smartphone, von der vorliegenden Erfindung umfasst sein.

In einigen Ausführungsformen umfasst die Abrufvorrichtung zum Aktivieren des Auslösemechanismus wenigstens eine der folgenden Einrichtungen oder ist als solche ausgestaltet:
- einen integrierten Auslöseknopf, beispielsweise ausgestaltet als Softtouch-Knopf, als Druckknopf, als Schalter, als Bereich (Button) auf einem Touchscreen, als Sensor oder dergleichen;
- eine integrierte Eingabevorrichtung, beispielsweise eine Tastatur, ein Touchscreen, ein Drehknopf (oder mehrere), ein integrierter Sensor oder eine Kamera zum Erfassen von biometrischen Daten, beispielsweise Fingerabdruck, Gesicht oder Sprache, eines Codes, oder dergleichen;
- eine externe Eingabevorrichtung, die, vorzugsweise drahtlos, mit dem Nasenapplikator, insbesondere dessen Abrufvorrichtung verbunden ist, beispielsweise eine Applikation (App), eine Fernbedienung, Eingabe mittels Web oder Cloud, Rechner, Smart Device oder dergleichen.

In bestimmten Ausführungsformen kann die externe Eingabevorrichtung ausgestaltet sein analog zu den hierin genannten Beispielen der Ausführung der integrierten Eingabevorrichtung.

In manchen Ausführungsformen kann ein bestimmtes Verhalten des Benutzers/Patienten die Abrufvorrichtung steuern bzw. beeinflussen. Beispielweise kann ein zweimaliges Drücken für eine Auslösung mit dem ersten Drücken ein "Wecken" des Nasenapplikators ggf. verbunden mit einer Systemüberprüfung, einer Überprüfung der Berechtigung etc., einer Berechnung der Applikationsdosis und Bereitstellen derselben bewirken, während das zweite Drücken schließlich ein Auslösen der Applikationsdosis bewirkt.

In einigen Ausführungsformen kann der Nasenapplikator, insbesondere mittels einer seiner Vorrichtungen, eine Ausgabeinformation erzeugen, vorzugsweise automatisch, vorzugsweise nach einer Substanzdosisabgabe. Diese Ausgabeinformation kann eine Log-Datei der Behandlung sein oder umfassen. Dabei kann jedes erfassbare Signal bzw. jede erfassbare Interaktion, insbesondere jede Betätigung, geloggt werden. Alternativ oder ergänzend kann die Ausgabeinformation das Erstellen eines standardisierten und/oder auf den Benutzer/Patienten bezogenen personalisierten Berichts sein oder umfassen. Die Ausgabeinformation kann mittels eines digitalen (online) Dashboards, mittels Druckfunktionen (z. B. für das Betäubungsmittelbuch (BtM)), mittels Exports, etc. an eine hierzu jeweils geeignete Vorrichtung erfolgen, vorzugsweise drahtlos, in manchen Ausführungsformen auch kabelgebunden und/oder mit Kontaktpins.

In einigen Ausführungsformen kann der Abgabedosierer Auslösemechanismus auf andere Art und Weise aktiviert werden, beispielsweise *breath-activated* oder *nose-activated*, d. h. der Auslösemechanismus wird beispielsweise automatisch mittels eines Unterdrucksensors aktiviert, wenn der Nasenapplikator in die Nase eingeführt ist und durch die Nase eingeatmet wird. Auch eine mechanische Umsetzung dieses Auslösemechanismus ist von der vorliegenden Erfindung umfasst.

In manchen Ausführungsformen ist eine Optik in den Nasenapplikator integriert, beispielsweise zum Erfassen von biometrischen Daten, wie Fingerabdruck oder Gesicht, zum Erfassen eines QR-Codes, eines Barcodes oder dergleichen.

In einigen Ausführungsformen können die Ausgabeinformationen eine Angabe der Zeitdauer (Countdown/Timer) bis zur nächsten Freigabe sein oder umfassen. Die Anzeige bzw. der Ablauf dieser Zeitdauer kann beispielsweise optisch (z. B. mittels einer farbigen Anzeige, einer LED oder Push-Nachricht), akustisch (z. B. mittels Alarms), taktil (z. B. mittels Vibration) oder auf analoge Art und Weise erfolgen. Eine farbige Anzeige kann hierbei beispielsweise umfassen, dass der Nasenapplikator oder ein Abschnitt hiervon die Farbe ändert, um seinen Status zu signalisieren, beispielsweise auf "grün" wechselt, wenn eine neue Applikation erfolgen darf.

In manchen Ausführungsformen können die Ausgabeinformationen eine Ausgabe einer Schritt-für-Schritt Anleitung (z. B. für ein Setup oder eine weiterführende Bedienung oder dergleichen) mittels eines visuellen und/oder akustischen Mediums, beispielsweise eines Smartphones oder dergleichen, sein oder umfassen.

In manchen Ausführungsformen sind Schritt-für-Schritt Anleitungen auf dem Nasenapplikator und/oder auf entsprechenden Peripheriegeräten und/oder auf Smart Devices vorgesehen.

In einigen Ausführungsformen der vorliegenden Erfindung können Eingabe- und/oder Ausgabemöglichkeiten jeweils barrierefrei vorgesehen sein. Hierunter fallen z. B. Ein- und/oder Ausgaben in Braille, optische, akustische oder taktile Ein- und Ausgaben, z. B. Vibration, Schall etc. Dies ist insbesondere auch vorteilhaft für den Einsatz der vorliegenden Erfindung bei schlechten Sichtbedingungen und/oder bei Nacht. Diese Eingabe- und/oder Ausgabemöglichkeiten können insbesondere kontaktlose Eingabe- und/oder Ausgabemöglichkeiten sein oder umfassen.

In einigen Ausführungsformen umfasst der Nasenapplikator einen QR-Codeleser. Dieser kann zum Scannen der UDI ("*Unique Device Identification*") der Einwegkartusche, zum Erkennen des Einweggehäuses, der Applikatorelektronik, eines Einwegarmbands und/oder dessen Armbandelektronik, und/oder zum automatisierten Auslesen in einer *Docking Station* dienen.

In einigen Ausführungsformen ist von der vorliegenden Erfindung auch eine Druckerfunktionalität umfasst. Diese kann u. a. dazu dienen, einen Bericht/Report zu erstellen, Aufkleber zu fertigen (z. B. für das BtM-Buch und/oder die Patientenakte), usw.

In manchen Ausführungsformen der vorliegenden Erfindung ist ein Datenzugang direkt mittels eines eigenen Eingabe- und Ablesedisplays vorgesehen. Alternativ oder ergänzend kann ein Datenzugang indirekt mittels einer "digitalen online Verwaltungssoftware" beispielsweise mittels eines Smartphones, Tablets und/oder Rechners und/oder mittels anderer Schnittstellen vorgesehen sein.

In manchen Ausführungsformen umfasst der Nasenapplikator und/oder wenigstens eine seiner Vorrichtungen berührungsempfindliche (*touch-sensitive*) Oberflächen.

In einigen Ausführungsformen kann die vorliegende Erfindung ein in den Nasenapplikator integriertes Mikrofon und/oder ein Smartphone-Mikrofon umfassen. Dieses Mikrofon kann dazu dienen, um z. B. ein Schmerztagebuch aufzuzeichnen.

In bestimmten Ausführungsformen kann die vorliegende Erfindung eine "*shake to wake*" Funktion umfassen. Hierzu geeignete Vorrichtungen, beispielsweise ein Beschleunigungssensor, ein Gyroskop, ein Winkelsensor, ein Lagesensor und/oder dergleichen sind ebenfalls von der vorliegenden Erfindung umfasst.

In einigen Ausführungsformen sind die genannten Vorrichtungen ergänzend dazu vorgesehen, einen unsachgemäßen Umgang mit dem Nasenapplikator zu detektieren, beispielsweise Fall, Schock, Vibration, übermäßiger Druck auf den Applikator, groben Umgang oder dergleichen.

In manchen Ausführungsformen kann die vorliegende Erfindung eine in den Nasenapplikator integrierte Kamera und/oder eine Smartphone-Kamera umfassen. Diese Kamera kann dazu dienen, um beispielsweise in die Nase, Ohren, usw. schauen zu können nach Übertragung an einen Arzt eine Diagnose stellen zu lassen, und dergleichen (*Digital Health Diagnostics*). Sie kann ferner dazu dienen, das Maß einer Sedierung zu erkennen, beispielsweise mittels Aufnahmen einer Pupillenreaktion des Auges.

Patienten-Authentifizierung bedeutet, dass nur der berechtigte Benutzer/Patient das Gerät bedienen kann. Ein Benutzer kann u. a. beispielsweise ein Arzt, eine Pflegekraft und/oder ein anderer Berechtigter sein.

In einigen Ausführungsformen weist der Nasenapplikator hierzu ein integriertes Authentifizierungsmodul auf, in anderen Ausführungsformen ist das Authentifizierungsmodul extern vorgesehen, in bestimmten Ausführungsformen teilintegriert. Hierbei ist der dem Authentifizierungsmodul zugrundeliegende Authentifizierungsmechanismus vorzugweise mit genau einem einzigen, konkreten Gerät gekoppelt. Hierzu erforderliche Signalübertragungen können z. B. wie hierin offenbart vorgesehen sein und erfolgen. Entsprechende Ausgestaltungen der beteiligten Signalübertragungspartner können vorgesehen sein.

In manchen Ausführungsformen kann ein integriertes, also am oder im Nasenapplikator vorgesehenes, Authentifizierungsmodul optisch wirken, z. B. mittels QR-Codeleser oder mittels Vorrichtungen zum Erkennen und Vergleichen biometrischer Daten, in anderen Ausführungsformen kann es akustisch wirken, z. B. mittels Stimmerkennung, in weiteren Ausführungsformen taktil, z. B. mittels der Eingabe einer Geheimzahl oder eines Passworts. Das Authentifizierungsmodul umfasst hierbei die jeweils zur Authentifizierung geeignete Sensoranordnung und eine Auswertevorrichtung.

In einigen Ausführungsformen kann das Authentifizierungsmodul vorgesehen sein, um mit weiteren Einrichtungen, Vorrichtungen, Geräten kompatibel zu sein.

Es kann in bestimmten Ausführungsformen vorgesehen sein, eine Authentifizierung mittels Vibration (beispielsweise mittels eines bestimmten Vibrationsmusters) oder mittels Sprach- oder Stimmerkennung durchzuführen. In weiteren Ausführungsformen kann über den GPS-Standort des Nasenapplikators und/oder mittels einer Geofencing-Funktion ein Bereich festgelegt werden, in welchem das Gerät freigeschaltet ist.

In manchen Ausführungsformen kann ein teilintegriertes oder externes Authentifizierungsmodul am Patienten vorgesehen sein, in andern Ausführungsform ist es nicht am Patienten vorgesehen. Bei teilintegrierten oder externen Authentifizierungsmodulen kann der Authentifizierungsmechanismus beispielsweise auf Token, drahtloser Authentifizierung basieren. Das Authentifizierungsmodul kann eine Armbanduhr, ein RFID-Armband, ein Barcode/QR-Code, eine Kette, ein Ring, ein Smartphone, eine Smartwatch, ein Tablet, oder ergänzend eine Software für die vorgenannten Module (z. B. eine App), etc. sein oder umfassen. Das Authentifizierungsmodul umfasst hierbei die jeweils zur Authentifizierung geeignete Sensoranordnung und/oder Auswertungsvorrichtung, analog wie oben beschrieben.

In einigen Ausführungsformen ist das Authentifizierungsmodul mit Standard Krankenhaus-Patientenidentifikationshilfsmitteln kompatibel, beispielsweise kann es aufgeklebt oder ausgedruckt werden, oder es ist in diese integrierbar (z. B. in ein Patientenarmband).

In bestimmten Ausführungsformen dient das Standard Krankenhaus-Patientenidentifikationshilfsmittel selbst als Authentifizierungsmodul, beispielsweise der aufgedruckte Barcode/QR-Code.

In manchen Ausführungsformen kann es notwendig sein, mittels des Authentifizierungsmoduls eine Authentifizierung eines Administrators und/oder einer Benutzergruppe vorzunehmen, um den Nasenapplikator in Betrieb zu nehmen bzw. das Auslösen einer Applikationsdosis zu veranlassen bzw. Anpassungen am Nasenapplikator vorzunehmen.

In manchen Ausführungsformen ist das Authentifizierungsmodul vorgesehen, um ergänzend Techniker und/oder Systemadministratoren zu authentifizieren und ihnen Zugang zur Software des Nasenapplikators, insbesondere dessen Steuervorrichtung, zu gewähren.

In einigen Ausführungsformen kann es notwendig sein, mittels des Authentifizierungsmoduls die Authentifizierung eines Administrators und/oder einer Benutzergruppe vorzunehmen, um die Steuerungsvorrichtung des Nasenapplikators per Fernbedienung oder Fernsteuerung (*remote*) zu bedienen und/oder Daten auf ihr zu überschreiben (override-Funktion). Dies kann z. B. notwendig sein, um eine extra Medikamentenabgabe zu veranlassen. Die Authentifizierung kann hierbei mittels einer der hierin genannten Vorrichtungen und/oder Wirkweisen vorgenommen werden.

In manchen Ausführungsformen ist die Reichweite des Authentifizierungsmoduls variabel und/oder einstellbar, vorzugsweise stufenlos.

In einigen Ausführungsformen kann das Authentifizierungsmodul in sogenannte smarte Textilien integriert sein, beispielsweise in eine Brille, in Schuhe, in einen Patientenkittel, in ein Kissen und/oder in einen Kissenbezug und/oder in einen Bettbezug und/oder Matratze, in einen Arbeitskittel oder dergleichen.

In bestimmten Ausführungsformen kann das Authentifizierungsmodul auch am Bettrand, auf einem Nachttisch, in einer Steckdose in Patientennähe befestigt werden oder sein.

In manchen Ausführungsformen ist das Authentifizierungsmodul eine Vorrichtung, die im Ohr getragen wird (*In-Ear Wearable*) oder dergleichen.

In einigen Ausführungsformen ist oder umfasst das Authentifizierungsmodul ein subkutanes Implantat zur Authentifizierung und eine zum Auslesen desselben geeignete Sensoranordnung.

In manchen Ausführungsformen ist oder umfasst das Authentifizierungsmodul eine Zahnkrone oder Zahnschiene zur Authentifizierung und eine zum Auslesen derselben geeignete Sensoranordnung.

In einigen Ausführungsformen ist oder umfasst das Authentifizierungsmodul eine in den Nasenapplikator integrierte Kamera und/oder eine Smartphone-Kamera, um Daten, beispielsweise biometrische Daten, zu erfassen, alternativ oder ergänzend ein Mikrofon, integriert oder extern, zum Zweck der Stimmerkennung.

In einigen Ausführungsformen umfasst der Nasenapplikator ein Monitoring-/Überwachungssystem, wobei Monitoring die Beobachtung des Patienten und Überwachen zusätzlich noch die Beobachtung von dessen Umgebung ist oder umfasst. Mittels des Systems kann beispielsweise die sogenannte Therapietreue, d. h. in welchem Ausmaß das Verhalten des Patienten mit den vereinbarten Empfehlungen/Verschreibungen eines Mediziners übereinstimmt (*Compliance*/*Adherence*), überwacht werden.

In manchen Ausführungsformen umfasst das Monitoring-/Überwachungssystem eine automatisierte Medikamentenüberwachung in Echtzeit (*realtime*). Diese kann z. B. eine verbrauchte Menge, eine Restmenge, die Anzahl verbleibende Applikationen etc. erfassen und mittels einer hierzu geeigneten Speichervorrichtung intern oder extern speichern.

In einigen Ausführungsformen umfasst das Monitoring-/Überwachungssystem eine automatisierte Therapieüberwachung in Echtzeit (*realtime*). Diese kann z. B. Applikationszeiten und Applikationsmengen, versuchte (vergebliche) Applikationen während eines Sperrintervalls etc. erfassen und mittels einer hierzu geeigneten Speichervorrichtung intern oder extern speichern.

In manchen Ausführungsformen umfasst das Monitoring-/Überwachungssystem eine automatisierte Therapietagebuchführung in Echtzeit (*realtime*). Diese kann z. B. patientenindividuell Nebenwirkungen, Gemütszustände und/oder die Stärke, Dauer und/oder Häufigkeit von Beschwerden etc. erfassen und mittels einer hierzu geeigneten Speichervorrichtung intern oder extern speichern.

In einigen Ausführungsformen kann das Monitoring-/Überwachungssystem dazu vorgesehen sein, ein Mobilitätsprotokoll zu erstellen.

In einigen Ausführungsformen umfasst das Monitoring-/Überwachungssystem des Nasenapplikators eine Kombination aus den vorgenannten Echtzeit-Überwachungen.

Eine Echtzeit-Überwachung mittels des Monitoring- und/oder Überwachungssystem des Nasenapplikators ermöglicht vorteilhafterweise eine einfache und/oder genaue Protokollierung der Therapie

In manchen Ausführungsformen umfasst die Steuervorrichtung des Nasenapplikators integrierte, optische und/oder akustische Status- und/oder Alarmanzeigen, beispielsweise LED(s), Buzzer, Beep(s)und/oder ein Display, um den Status oder den Alarm, insbesondere mittels eines Error Code oder dergleichen, anzuzeigen.

In manchen Ausführungsformen umfasst die Steuervorrichtung des Nasenapplikators externe, optische und/oder akustische Status- und/oder Alarmanzeigen, beispielsweise in oder auf Peripheriegeräten, Smart Devices und/oder Stationsrechnern. Die externen Status- und/oder Alarmanzeigen können ebenfalls LED(s), Buzzer, Beep(s)und/oder ein Display umfassen, ferner können sie als Software Dashboard (App), Push-Nachricht oder dergleichen vorgesehen sein.

In manchen Ausführungsformen ist der Nasenapplikator konfiguriert, um automatische Meldungen, insbesondere einen Hilferuf und/oder einen Alarm an Dritte, z. B. an bestimmte Personengruppen, Ärzte, Pflegepersonal, Rettungsleitstelle oder dergleichen, zu senden. Dies kann insbesondere bei ungewöhnlichem Verhalten, etwa bei Abweichen von gespeicherten Bewegungsprofilen oder -mustern, oder beispielsweise bei ständigen Medikamentenabrufen, Überschreiten der maximal erlaubten Medikamentenmenge (*Dosing Limit*) oder dergleichen, und/oder wenn die Vitalwerte des Benutzers/Patienten außerhalb vorbestimmter Grenzen liegen. Alternativ oder ergänzend kann eine solche Meldung mittels aktiven Betätigens eines "Notfallknopfs" durch den Benutzer/Patienten vorgesehen sein.

In einigen Ausführungsformen ist ein "sicherer" Zustand des Nasenapplikators vorgesehen. Dieser ist insbesondere im Zustand "aus" gegeben. Im "sicheren" Zustand gibt das System egal unter welchen Umständen keine Dosis ab.

In manchen Ausführungsformen umfasst der Nasenapplikator eine Patientenüberwachungssensorik. Die Sensorik ist geeignet, patientenindividuelle Daten zu erfassen. Diese Daten können objektive Daten wie Position, Bewegung und/oder Vitalwerte (z. B. HR, HRV, SpO₂, Blutdruck, elektrochemische Hautreaktion, Temperatur, Photoplethysmogramm (PPG), Electrocardiogram (ECG), Blood pressure trending (BPT), Respiratory rate, Stress, etc.) oder subjektive Daten, erfasst mittels Patientenfeedback wie hierin beschrieben, sein oder umfassen.

Derartige Überwachungssysteme können die Therapietreue und somit den therapeutischen Effekt (z. B. durch Reminder, unterstützende Informationen, Wertcoupons etc., Statusabfrage, Vorschläge, Aktivitätstracker, Benachrichtigung von Angehörigen/Bekannten, etc.) vorteilhafterweise erhöhen oder optimieren.

In einigen Ausführungsformen kann vorgesehen sein, den Nasenapplikator mit weiteren Funktionen, wie beispielsweise Reminder, weitergehender unterstützender Information, Gamification (z. B. Punkte sammeln, TV-Minuten sammeln, die bei der Krankenhausverwaltung gegen Zugang zum Fernsehprogramm eingetauscht werden können, Wertcoupons etc.), Statusabfragen, Vorschlägen, Aktivitätstracker, Benachrichtigung von Angehörigen/Bekannten, Teilnahme an Umfragen, z. B. online-Umfragen, etc. zu ergänzen.

In manchen Ausführungsformen kann der Nasenapplikator vorgesehen sein, sogenanntes *social monitoring and communication* zu ermöglichen, d. h. ein, vorzugsweise bidirektionales, Kommunikationsmittel zu Familie, Freunden und Pflegekräften usw. bereitzustellen.

In einigen Ausführungsformen kann der Nasenapplikator zusätzlich zur medizinischen Substanz in einem weiteren Substanzreservoir ein Gegenmittel (Antidot) aufweisen, geeignet um z. B. Nebenwirkungen zu verringern bzw. zu verhindern oder eine Überdosierung zu vermeiden oder dergleichen.

In manchen Ausführungsformen ist der Nasenapplikator konfiguriert, um eine Atemgasanalyse, z. B. eine Kapnographie, durchzuführen und das Ergebnis der Analyse beispielsweise anzuzeigen, an ein Peripheriegerät zu übermitteln und/oder in einer hierzu geeigneten Speichervorrichtung zu speichern.

In einigen Ausführungsformen kann der Nasenapplikator mit Sensoranordnungen und/oder Auswerteeinrichtungen verbunden sein, mittels derer die Position des Benutzers/Patienten erfasst und gespeichert werden können. Die Position kann die Lagerung des Patienten erfassen, ob er/sie aufsteht, gestürzt ist und/oder dergleichen. Diese Sensoranordnungen können intern oder extern vorgesehen sein, vorzugsweise wie hierin ausgeführt, beispielsweise integriert in *smarte* Textilien mit integrierten Sensoren. Der Nasenapplikator kann zu diesem Zweck weiter geeignete Aktuatoren usw. aufweisen. Auf diese Art und Weise kann beispielsweise ein Mobilitätsprotokoll etc. erstellt werden.

In manchen Ausführungsformen kann die Patientenüberwachungssensorik oder Teile hiervon implantiert sein. In anderen Ausführungsformen kann sie oder Teile hiervon in eine Zahnkrone oder -schiene oder in einen Ohrring oder Ohrclip integriert sein.

In einigen Ausführungsformen kann die Patientenüberwachungssensorik oder Teile hiervon, Befindlichkeiten bzw. Zustände des Benutzers/Patienten, wie z. B. Schmerz, detektieren bzw. messbar machen. Dies kann über Körperreaktionen wie beispielsweise Hautgalvanik, Blutdruckänderung, Herzrateänderung und/oder Pupillendilatation ermittelt werden.

In manchen Ausführungsformen umfasst der Nasenapplikator ein Nasenapplikator-Ortungssystem. Dabei kann das Nasenapplikator-Ortungssystem konfiguriert sein, um den Nasenapplikator zu orten, die Substanz zu orten und/oder den Benutzer/Patienten zu orten. Dabei kann ein integriertes Echtzeit-(Realtime)-Ortungssystem z. B. ein Flottenmanagement oder dergleichen mittels drahtloser Konnektivität (z. B. via Wi-Fi, Bluetooth, WLAN, GPS, GSM, GPRS, Starlink, WPAN, Infrarot, RFID, NFC, Barcode, QR-Code, ZigBee, Wibree, WiMAX und/oder IrDA etc.) zum Einsatz kommen. Das Nasenapplikator-Ortungssystem dient vorteilhafterweise dem Zweck, die Geräteverwaltung zu erleichtern und die Patientensicherheit zu erhöhen.

In manchen Ausführungsformen kann die Einrichtung virtueller Zonen bzw. Bereiche die Positionsbestimmung vereinfachen.

In einigen Ausführungsformen umfasst der Nasenapplikator ein Anti-Missbrauchsystem und/oder ein Anti-Diebstahlsystem.

In manchen Ausführungsformen ist das Gehäuse des Nasenapplikators unzugänglich ausgestaltet, derart, dass ein Zugang zu der oder jeder Substanz verhindert wird. Dies kann beispielsweise mittels eines Einweggehäuses realisiert werden, das beim Öffnen zerstört würde.

In einigen Ausführungsformen umfasst der Nasenapplikator eine Vorrichtung zu seinem physischen Fixieren. Dies kann beispielsweise mittels eines Kensington^{®} Schlosses, eines Sicherungskabels bzw. -Seils, Kabelschlosses, etc. ausgestaltet sein.

In manchen Ausführungsformen können für die Datenübertragung und/oder Stromübermittlung gemeinsame Leiter vorgesehen sein. Beispielsweise sei hier ein LAN - Kabel für die Datenübertragung und/oder Strom (POE) mit integriertem Stahlseil genannt.

In manchen Ausführungsformen ist der Nasenapplikator mit einem vorbestimmten Geofence vorgesehen. In diesen Ausführungsformen ist von der vorliegenden Erfindung die Möglichkeit umfasst, die Funktionalität des Nasenapplikators außerhalb eines vordefinierten Bereichs zu beeinflussen, beispielsweise indem ein Auslösen verhindert oder der Wirkstoff der Substanz neutralisiert wird. Dies kann mittels einer Softwarelösung und/oder drahtloser Konnektivität realisiert werden. Wie hierin bereits ausgeführt, können Peripheriegeräte hierbei als Funkzelle dienen.

In einigen Ausführungsformen kann der Nasenapplikator kindersicher ausgestaltet sein. Die Kindersicherung kann beispielsweise mittels einer Hardware (z. B. Abdeckung, Verschluss und/oder Safe) und/oder mittels einer Software (z. B. Patienten Authentifizierung etc.) realisiert sein.

In manchen Ausführungsformen können Peripheriegeräte des Nasenapplikators mit Sicherheitsverschluss und/oder Manipulationsschutz vorgesehen sein, z. B. ein Armband mit eingelegtem Stahldraht, Kevlar, etc.), insbesondere können Peripheriegeräte stufenlos verstellbar und somit individuell auf den Benutzer/Patienten einstellbar sein.

In einigen Ausführungsformen kann eine Echtzeit-Ortung (Realtime), insbesondere ein Geofencing, zum Flottenmanagement und/oder Sperren/Entsperren des Nasenapplikators und/oder zum Auslösen eines Neutralisierens des Wirkstoffes in der Substanz dienen.

In manchen Ausführungsformen kann der Wirkstoff in der Substanz beispielsweise durch ein flüssiges und/oder festes und/oder gasförmiges Additiv, wie z. B. eine Base, Säure, Antagonist, Gips, Zement und/oder Kälte (N₂O ≙ -89° C oder CO₂ ≙ -78,5° C) etc. neutralisiert bzw. zerstört werden.

In einigen Ausführungsformen kann ein Trocknungsmittel, beispielsweise wie hierin offenbart, vorgesehen sein, um von der Substanz durchlaufen zu werden, wobei das Trocknungsmittel dabei die Substanz oder einen hierin enthaltenen Stoff oder Wirkstoff (z. B. ein Salz) bindet.

Als Trocknungsmittel können z. B. in Frage kommen:
- Natrium Sulfat
- Calcium Sulfat
- Calcium Chlorid

In manchen Ausführungsformen ist ein Filter vorgesehen, welchen die Substanz durchlaufen kann, z. B. ausgestaltet als Molekularsieb, Filterfließ, Ionenaustauscher, oder dergleichen.

In einigen Ausführungsformen kann der Nasenapplikator konfiguriert sein, um beispielsweise zwischen der Behandlung zweier unterschiedlicher Patienten wieder aufbereitet zu werden. Ein Wiederaufbereiten kann z. B. ein Programmieren oder Umprogrammieren, ein (neu) Laden oder ein Austauschen von Batterien oder anderen Energiespeichervorrichtungen wie hierin beschrieben, ein (erneutes) Befüllen des oder der Substanzreservoirs und/oder das Einlegen einer neuen Kartusche sein oder umfassen. Ferner kann das Wiederaufbereiten ein Überprüfen, beispielsweise in Form eines Systemchecks, ein ganz oder teilweises Entsorgen von Nasenapplikator und/oder Substanz, Hygienemaßnahmen, wie Putzen und/oder Desinfizieren, und/oder eine Wartung, welche ergänzend ein Systemupdate beinhalten kann, sein oder umfassen. Die Steuervorrichtung kann optional programmiert sein, um Schritte zum Wiederaufbereiten, wie z. B. das Löschen von Patientendaten automatisch oder auf Aufforderung zu löschen, etwa nach Auswählen eines entsprechenden Menüpunkts im Steuerungsmenü der Speichervorrichtung.

In manchen Ausführungsformen ist der Nasenapplikator ein vollständiges Einwegprodukt, welches komplett nach geltenden Regularien nach erstmaliger Verwendung am Patienten entsorgt werden muss. Dabei müssen unter Umständen der Nasenapplikator, die Substanz(en) und/oder ein eventuelles Peripheriegerät einzeln entsorgt werden.

In einigen Ausführungsformen ist der Nasenapplikator teilweise ein Einwegprodukt und weist beispielsweise eine wiederverwendbare Elektronik, Einwegkomponenten, welche mit der Substanz und/oder dem Benutzer/Patienten in Kontakt gekommen sind (z. B. ein Einweggehäuse für die Elektronik, ein Einwegnasenaufsatz, eine Einwegpumpe oder dergleichen.

In manchen Ausführungsformen kann der Nasenapplikator konfiguriert sein, um automatisiert und/oder protokolliert eine Entsorgung der Restmenge der Substanz zu umfassen. Dies kann beispielsweise durch Abpumpen in Sondermüll oder in einen Spezialbehälter bzw. ein separates Behältnis für die Medikamentenentsorgung, Ansaugen und Neutralisieren, etc. erfolgen. Dies kann in bestimmten Ausführungsformen der Dokumentation der Medikamentenentsorgung dienen.

In einigen Ausführungsformen umfasst der Nasenapplikator eine modulare, vorzugsweise erweiterbare *Docking Station* für die elektronische Steuervorrichtung und/oder weitere Peripheriegeräte. Die Docking Station kann beispielsweise zum Laden der Energiespeichervorrichtung, zum Übertragen von Softwareupdates auf den Nasenapplikator und/oder die Peripheriegeräte, zum Pairing von zwei beliebigen vom Nasenapplikator umfassten Vorrichtungen, zur Hardwareüberprüfung, zur Implementierung von Freigaben auf den Nasenapplikator/das Peripheriegerät usw. konfiguriert sein.

In manchen Ausführungsformen ist der Nasenapplikator konfiguriert zur automatischen (Nach-)Bestellung der Substanz(en).

In einigen Ausführungsformen enthält die Schutzkappe des Nasenapplikators antibakterielle Mittel, um den Nasenaufsatz sauber zu halten.

In manchen Ausführungsformen umfasst der Nasenapplikator eine Spülvorrichtung für den Nasenaufsatz, da sonst das Substanzvolumen im Nasenaufsatz bis zur nächsten Auslösung "stehen" bleiben würde, was eine Besiedelung mit Mikroorganismen begünstigen könnte. Dies kann vorzugsweise bei sensiblen Wirkstoffen in der Substanz vorgesehen sein.

In einigen Ausführungsformen kann ein Primingprozess vorgesehen sein, während dem z. B. die Substanz in das Substanzreservoir zurückgeführt und automatisch aufgefangen wird. Es kann in manchen Ausführungsformen vorgesehen sein, dass der Nasenapplikator eine Auslösung blockiert, wenn der Primingprozess nicht abgeschlossen ist.

In manchen Ausführungsformen kann während des Primingprozesses ein akustisches Signal abgegeben werden. Dieses Signal kann ein Warnton sein, der beispielsweise ertönt, solange der Primingprozess nicht abgeschlossen ist. Ebenso kann erst mit oder nach Beendigung des Primingprozesses ein akustisches Signal, z. B. wie hierin ausgeführt, abgegeben werden.

Wenn hierin, und insbesondere vorstehend, von einem akustischen Signal die Rede ist, so soll dies nicht darüber hinwegtäuschen, dass auch ein optisches Signal vorgesehen sein kann, insbesondere entsprechend der Ausführungen für das akustische Signal. So kann ein optisches Signal andeuten, dass der Primingprozess noch nicht abgeschlossen ist. Alternativ oder ergänzend kann ein optisches Signal andeuten, dass der Primingprozess nun, oder erst jetzt, als abgeschlossen gilt.

In einigen Ausführungsformen kann innerhalb und/oder außerhalb des Nasenapplikators eine Datenauswertung via Software (z. B. Datenexport, Dashboard, Berichte, E-Mails, Notifications, Bildschirm, App etc.) vorgesehen sein.

In manchen Ausführungsformen kann bei der Datenauswertung vorgesehen sein, eine Datenverschlüsselung anzuwenden. Diese kann beispielsweise Verschlüsselungskey, Blockchain, Passwort, Passphrase, Token, Biometrik, und/oder Dongle, etc. sein oder umfassen.

In einigen Ausführungsformen des Nasenapplikators sind weitere Funktionalitäten zuschaltbar, beispielsweise ein Dashboard (Leitstand), von dem zentral die Vorrichtungen des Nasenapplikators angesteuert, überwacht etc. werden können, zur Therapie und/oder weitere Vorrichtungen und oder die Applikation weiterer Substanzen und/oder Funktionen zur Patientenverwaltung und/oder dergleichen.

In manchen Ausführungsformen kann der Nasenapplikator vorgesehen oder programmiert sein, interne und/oder externe Umfragen durchzuführen, z. B. zur Patientenzufriedenheit, zu Nebenwirkungen oder dergleichen. Diese Umfragen können standardisiert sein, beispielsweise gemäß der *International Statistical Classification of Diseases and Related Health Problems* (ICD-10, EU) oder gemäß *Diagnosis Related Groups* (DGRG) und/oder gemäß der *Qualitätsverbesserung in der postoperativen Schmerztherapie* (QUIPS)(Deutschland) und oder gemäß den *Risk Evaluation and Mitigation Strategies* (REMS) Programmen, den DEA-Überwachungsprogrammen bzw. der DEA-Dokumentation (USA).

In einigen Ausführungsformen umfasst der Nasenapplikator eine QIUPS Schnittstelle (Fragenbogen, Informationsmaterial etc.) zur Ermittlung des akuten und chronischen Schmerzempfindens des Benutzers/Patienten.

In manchen Ausführungsformen umfasst der Nasenapplikator eine Patientenaufklärung (online und/oder offline) zur Schulung und/oder Nachsorge *(aftercare)* des Benutzers/Patienten oder eine Möglichkeit hierzu. Diese kann beispielsweise mittels einer Online-Video-Sprechstunde, mittels eines virtuellen Assistenten, mittels einer Bedienungsanleitung, eines Beipackzettels, einer Information zu Nebenwirkungen, FAQs, Operations und/oder Produktinformationen, Formularen, Umfragen, Therapietagebuch, elektronische Patientenakte etc. erfolgen.

In manchen Ausführungsformen umfasst der Nasenapplikator eine Trainingsfunktion (online und/oder offline) für Ärzte und/oder Pflegepersonal, die z. B. mittels Reminder, neuen Schulungen, Studien, Geräteupdates, neuen Funktionalitäten, Substanzen, etc. erfüllt wird.

In einigen Ausführungsformen können mittels der vorliegenden Erfindung benutzerdefinierte Berichte erstellt werden, um die individuellen Anforderungen von Krankenhäusern oder anderen Einrichtungen und/oder Vorschriften zu erfüllen. Hierzu können beispielsweise die Vorschriften der *Food and Drug Administration* (FDA) in den USA, die Vorschriften des Bundesinstituts für Arzneimittel und Medizinprodukte (BfArM) in Deutschland oder die Vorschriften der Europäische Arzneimittel-Agentur (*European Medicines Agency,* EMA) in Europa etc. gehören.

In manchen Ausführungsformen ist der Nasenapplikator vorgesehen, IoT *(Internet of Things)* zu unterstützen, beispielsweise mittels *Healthcare Tracking, Identification*/*Authentication, Data Collection*/*Data Mining* und/oder *Sensing.* Mittels hierzu geeigneter Kommunikationsvorrichtungen, insbesondere ohne menschliches Zutun, können dann Daten über das Netzwerk übermittelt werden. Hierzu kann optional vorgesehen sein, eines oder mehrere der einzelnen Vorrichtungen und/oder Peripheriegeräte des Nasenapplikators mit eineindeutigen Identifikatoren zu versehen, damit sich die einzelnen Vorrichtungen und Peripheriegeräte in einem Netzwerk erkennen können.

In einigen der Ausführungsformen ist eines der Peripheriegeräte programmiert, um von einem ersten der Nasenapplikatoren erste Daten zu empfangen, diese zu verarbeiten, und um basierend auf dem Ergebnis der Verarbeitung zweite Daten zu erstellen und Letztere an einen zweiten der Nasenapplikatoren und/oder an den ersten Nasenapplikator zu senden. Dabei können zweite Daten z. B. ein Algorithmus sein, oder Ergebnisse, die beim Ablaufen des Algorithmus erzielt wurden.

Insbesondere können auf diese Weise Erfahrungen, die bei der Nutzung des konkreten, ersten Nasenapplikators an einem ersten Benutzer gewonnen wurden, und welche sich in den ersten Daten widerspiegeln können, vorteilhaft genutzt werden, um die spätere Nutzung dieses ersten Nasenapplikators zu verbessern. Hierzu können aufgrund der vom ersten Nasenapplikator empfangenen Daten nach deren Auswertung, Bearbeitung, Verarbeitung usw. mittels eines der Peripheriegeräte zweite Daten erstellt werden und z. B. von diesem Peripheriegerät auf den ersten Nasenapplikator übertragen werden, was von der vorliegenden Erfindung umfasst ist. Zweite Daten können Zuordnungen des konkreten ersten Benutzers zu einer Gruppe von Benutzern sein, also z. B. zu seiner Klassifizierung oder zur Klassifizierung seines Krankheitsbildes oder seines Benutzerverhaltens umfassen. Sie können als Formeln, Algorithmen oder in anderer Form vorliegen und übertragen werden. Sie können sich von einem reinen Update, welches ohne Betrachtung des konkreten Benutzers, seines Verhaltens und/oder seiner Charakterisierung als Patient, sondern z. B. als medizinisch unterscheidungsloser Konsument, erfolgt, unterscheiden.

Insbesondere können auf diese Weise Erfahrungen, die bei der Nutzung des konkreten, ersten Nasenapplikators an einem ersten Benutzer gewonnen wurden, und welche sich in den ersten Daten widerspiegeln können, vorteilhaft genutzt werden, um die spätere Nutzung eines zweiten Nasenapplikators zu verbessern. Hierzu können aufgrund der vom ersten Nasenapplikator empfangenen Daten nach deren Auswertung, Bearbeitung, Verarbeitung usw. mittels eines der Peripheriegeräte zweite Daten erstellt werden und z. B. von diesem Peripheriegerät auf einen zweiten Nasenapplikator übertragen werden, was von der vorliegenden Erfindung umfasst ist. Zweite Daten können wie vorstehend ausgeführt sein. Sie können dem Benutzer des zweiten Nasenapplikators dienen, Erfahrungen, welche z. B. oder u. a. durch die Benutzung des ersten Nasenapplikators durch den ersten Benutzer gewonnen wurden, für seine künftige Nutzung seines zweiten Nasenapplikators vorteilhaft für sich zu verwenden.

In manchen Ausführungsformen ist vorgesehen, dass der Einsatz eines Nasenapplikators zunächst mit einem "Basis-Algorithmus" startet.

Der anfängliche Algorithmus, hier auch "Basis-Algorithmus" genannt, startet optional mit einem voreingestellten Modell und Parameter-Set für die entsprechende Indikation, welche anhand z. B. des Alters, Ko-medikation, signifikanten Vorerkrankungen usw. initial angepasst werden kann. Nach dem Start der Behandlung oder Benutzung wird der "Basis-Algorithmus" (Modell und/oder Parameter) anhand der applizierten Dosen und/oder der daraus resultierenden Konzentrationen und vorzugsweise mithilfe des Abgleichs zwischen theoretisch errechneten nächsten Applikationszeitpunkten und den tatsächlichen nächsten Applikationszeitpunkten, des Betätigungsverhaltens des Benutzers und/oder des Inputs von Vitalwerten trainiert, um den Patienten zu charakterisieren. Hierauf basierend kann somit die Benutzung oder Behandlung dieses Patienten bei weiterer Verwendung seines Nasenapplikators optimiert werden.

Hierzu können Verfahren, Methoden und Technologien der Künstlichen Intelligenz und/oder des Machine Learnings (oder der Sonderfall des Machine Learnings, das Deep Learning) angewandt werden, um den "Basis-Algorithmus" (Modell und/oder Parameter) zu trainieren, was beispielsweise auf dem Peripheriegerät stattfinden kann, etwa unter Rückgriff auf Daten, die an einer Vielzahl von Nutzungen verschiedener Nasenapplikatoren gewonnen worden sein können.

Die somit erzeugten Behandlungsdaten (applizierte Dosen, Applikationszeitpunkte, Vitalwerte, usw.) bzw. die "Trainingsentwicklung" des "Basis-Algorithmus" (Modell und/oder Parameter) eines jeden einzelnen Benutzers können strukturiert gesammelt und am Ende der Therapie oder in Echtzeit homogenisiert in entsprechenden Datenbanken gesammelt werden. Diese Datenbank kann dann mit Hilfe von "Big Data" Analyse Methoden (z. B. Cluster Analyse, Data Mining, Kurvenanalyse, Musteranalyse, usw.) und/oder Verfahren, Methoden und Technologien der Künstlichen Intelligenz und/oder des Machine Learnings (oder des Sonderfalls des Machine Learnings, das Deep Learning) nach Gemeinsamkeiten, Mustern, Auffälligkeiten, usw. durchsucht werden mit dem Ziel aussagekräftige und umsetzbare Verbesserungen/Erkenntnisse für den "Basis-Algorithmus" abzuleiten bzw. auszuwerten und diese mittels Übertragen zweiter Daten zu implementieren.

In einem optionalen Schritt kann die "eigene" Datenbank mit anderen Datenbanken, wie z. B. Langzeit Patientendaten z. B. EHR (electronic health record), EMR (electronic medical record) und/oder HIS (healthcare information system) und/oder Gendatenbanken und/oder die Kombination von Genotyp mit Phänotypen und/oder klinische Studien und/oder Langzeitstudien z. B. QUIPS (Qualitätsverbesserung in der postoperativen Schmerztherapie), Pain-Out, usw. und/oder "wearables & sensors" Daten und/oder weiteren Computermodellen wie z. B. In-silico-Medizin Computersimulationen und/oder anderen Quellen vernetzt werden. Ziel dieser Vernetzung ist es, die Datenlage/Basis (Menge) für die "Big Data" Analysemethoden bzw. die KI-Methoden und den daraus resultierenden Ergebnissen zu vergrößern bzw. zu erweitern mit denen dann der "Basis-Algorithmus" "trainiert" wird. Ziel ist es, den "trainierten neuen Basis-Algorithmus" dann für zukünftige Behandlungen zu verwenden. Er kann als zweite Daten verwendet und entsprechend übertragen werden. Alternativ oder ergänzend können zweite Daten basierend auf einem solchen "trainierten neuen Basis-Algorithmus" auf dem Peripheriegerät erzeugt und auf einen Nasenapplikator übertragen werden. Dieser Vorgang kann für jede Indikation individuell angepasst werden.

In einigen Ausführungsformen kann der Nasenaufsatz des Nasenapplikators konfiguriert sein, um Temperatur, Feuchtigkeit, Luftdurchfluss, Unterdruck usw. zu messen.

In manchen Ausführungsformen des Nasenapplikators kann dieser konfiguriert sein, Aufzeichnungen des Schlafrhythmus durchzuführen, und basierend auf dieser Aufzeichnung eine Anpassung der weitergehenden Dosierung *(Dosing Schedule)* vor/während/nach dem Schlaf vorzunehmen etc.

In manchen Ausführungsformen des Nasenapplikators kann dessen Gehäuseoberfläche einen Abperleffekt (Lotus Effekt) aufweisen, d. h. es kann selbstreinigend, antibakteriell etc. vorgesehen sein.

In einigen Ausführungsformen des Nasenapplikators kann das Gehäuse des Nasenapplikators kühlbar sein, z. B. für Insulin. Eine Kühlvorrichtung kann vorgesehen sein, ebenso wärmedämmende Materialen oder Vorkehrungen.

In bestimmten Ausführungsformen des Nasenapplikators kann das Gehäuse des Nasenapplikators heizbar sein, um ein Einfrieren, Ausflocken oder Degenerieren des Wirkstoffes zu vermeiden. Eine Heizvorrichtung kann vorgesehen sein, ebenso wärmedämmende Materialen oder Vorkehrungen.

In manchen Ausführungsformen des Nasenapplikators kann das Gehäuse des Nasenapplikators eine Isolation gegen Kälte, Hitze, Feuchtigkeit und/oder Flüssigkeit aufweisen.

In einigen Ausführungsformen des Nasenapplikators können an diesen zusätzliche Module anklickbar sein. Entsprechende Halterungen, Vorrichtungen usw. können am Nasenapplikator vorhanden sein.

In manchen Ausführungsformen des Nasenapplikators kann dieser über die AUX- oder Lightning-Buchse mit einem Smartphone, insbesondere mit dem Smartphone des Benutzers/Patienten, verbindbar sein. In diesen Ausführungsformen stellt das Smartphone die Elektronik und die entsprechende App die erforderliche Software des Nasenapplikators dar.

Hierin gilt, dass neben einer leitergebundenen Signalübertragung, als z. B. "verkabelt", auch noch eine "drahtlose" Variante, also eine kabellose Variante von der vorliegenden Erfindung umfasst ist, wann immer eine Signalübertragung hierin offenbart ist.

In bestimmten Ausführungsformen sind der Nasenapplikator und/oder eines der Peripheriegeräte programmiert, um ein Lagerungssignal, Aufstehsignal, Sturzsignal, usw. abgeben zu können. Hierzu können Accessoires wie z. B. Smartwatches, verstau- und/oder verklebbare Sensoren sowie Textilien oder smarte Textilien mit integrierten Sensoren, Aktuatoren, Haptik usw., Mobilitätsprotokoll etc. vorgesehen sein.

Der Nasenapplikator und/oder einer der Peripheriegeräte können zum Ausüben diese Funktionalitäten vorgesehen und konfiguriert sein. Sie können genutzt werden zum Abgeben eines Signals an Dritte (z. B. an eine Leitstelle, ins Stationszimmer usw. etwa nach Sturz des Benutzers), sie können aber auch genutzt werden bei der Vorbestimmung der Höhe einer oder mehrerer der nachfolgenden Applikationsdosen. Beispielsweise kann vorgesehen sein, die Abgabe eine Applikationsdosis und/oder der Höhe davon abhängig zu machen, dass der Benutzer zwischenzeitlich aufgestanden ist, also z. B. das Bett doch wenigstens für weniger Schritte oder etwas körperliche Betätigung verlassen hat.

In einigen Ausführungsformen ist der Nasenapplikators konfiguriert, um selbst, insbesondere automatisch, eine Konzentration der Substanz zu mischen und/oder das interne Substanzreservoir zu füllen und/oder mit einer Substanz aus einem zweiten Substanzreservoir zu verdünnen, z. B. 100 µg/100 µL verdünnen auf 50 µg/100 µL.

In manchen Ausführungsformen ist der Nasenapplikator geeignet zum *Energy-Harvesting,* d. h. zur Energiegewinnung z. B. durch eine oder mehrere Drehung(en) am Gehäuseboden. Somit kann ein hybrides System zwischen mechanischen und elektronischen Systemen erzeugt werden wobei die benötigte Energie jeweils bedarfsgerecht erzeugt wird.

In einigen Ausführungsformen ist die Steuervorrichtung des Nasenapplikators weiter programmiert, um beim Vorbestimmen der Höhe der nächsten Applikationsdosis Dₙ die festgesetzte Zielkonzentration C_{ED} oder ihren Mindestwert und/oder den ermittelten zeitlichen individuellen Konzentrationsverlauf, insbesondere die ermittelte individuelle PK-Kurve PK_{Ind}, zu berücksichtigen.

In manchen Ausführungsformen ist die Steuervorrichtung weiter programmiert, um beim Vorbestimmen der Höhe der nächsten Applikationsdosis Dₙ die festgesetzte Zielkonzentration C_{ED} oder ihren Mindestwert und/oder den ermittelten zeitlichen individuellen Konzentrationsverlauf, insbesondere die ermittelte individuelle PK-Kurve PK_{Ind}, zu berücksichtigen.

In einigen Ausführungsformen ist die Steuervorrichtung weiter programmiert, zum Begrenzen der Höhe der nächsten Applikationsdosis Dₙ und/oder der daraus resultierenden Konzentration; zum Begrenzen der kumulierten Höhe aller innerhalb einer vorbestimmten Zeitdauer abgegebenen Applikationsdosen D₀, D₁, D₂, ..., Dₙ₋₁, Dₙ, Dₙ₊₁, ... und/oder die daraus resultierenden Konzentrationen; und/oder zum Begrenzen der kumulierten Höhe aller abgegebenen Applikationsdosen D₀, D₁, D₂, ..., Dₙ₋₁, Dₙ, Dₙ₊₁, ... die daraus resultierenden Konzentration, wobei die Steuervorrichtung hierzu optional auf im Datenspeicher gespeicherte Daten die Substanz betreffend zurückgreift.

In einigen Ausführungsformen ist eines der Peripheriegeräte programmiert, um von einem ersten der Nasenapplikatoren erste Daten zu empfangen, diese zu verarbeiten, und basierend auf dem Ergebnis der Verarbeitung zweite Daten an einen zweiten der Nasenapplikatoren zu senden.

In einigen Ausführungsformen ist eines der Peripheriegeräte programmiert, um von einem ersten der Nasenapplikatoren erste Daten zu empfangen, diese zu verarbeiten, und basierend auf dem Ergebnis der Verarbeitung zweite Daten an den ersten Nasenapplikator zu senden.

In einigen Ausführungsformen weist der erfindungsgemäße Nasenapplikator weiter eine Einweg-Schutzhülle zum Aufsetzen auf den Nasenaufsatz auf, insbesondere wenn der Nasenaufsatz als Nasenaufsatz-Reservoir ausgestaltet ist.

In manchen Ausführungsformen weist die Einweg-Schutzhülle, vorzugsweise end- oder stirnseitig, einen Filter, z. B. wie hierin beschrieben, auf. Der Filter erlaubt ein Austreten von Substanz aus dem Nasenapplikator, vorzugsweise jedoch nicht ein Eintreten von pathogenen Teilchen aus der Nase des Patienten in den Nasenapplikator.

Die Einweg-Nasenaufsätze und/oder die Einweg-Schutzhülle ermöglichen, dass verschiedene Patienten mit demselben Nasenapplikator behandelt oder gar immunisiert werden können.

In manchen Ausführungsformen erfolgt das Auswechseln der Einweg-Nasenaufsätze manuell und/oder automatisch. Der Nasenapplikator kann hierfür vorbereitet sein.

In einigen Ausführungsformen der vorliegenden Erfindung erkennt die Elektronik des Nasenapplikators das Auswechseln des Einweg-Nasenaufsatzes und erlaubt nur im Anschluss hieran die Applikation einer nachfolgenden Dosis.

Durch verschiedene Maßnahmen kann das Substanzreservoir vor, insbesondere retrograder, Kontamination geschützt und so z. B. eine Kreuzinfektion vermieden werden. Dies kann in manchen Ausführungsformen mittels mechanischer Barrieren erfolgen. Solche Barrieren können insbesondere Ventile sein oder umfassen, z. B. mechanische Rückschlagventile.

In einigen Ausführungsformen ist wenigstens eine solche mechanische Barriere im Bereich der Spitze oder des freien Endes des Nasenaufsatzes (welcher in die Nase eingeführt wird) angeordnet. In manchen Ausführungsformen ist wenigstens eine solche mechanische Barriere im Bereich, in oder an der Anschlussstelle zwischen Leitung zum Substanzreservoir und Einweg-Nasenaufsatz, oder dieser Leitung, angeordnet. In einigen Ausführungsformen kann auf beiden Seiten der Anschlussstelle jeweils wenigstens eine solche mechanische Barriere angeordnet sein.

In einigen Ausführungsformen sind zur Vermeidung einer Kontamination antimikrobielle Komponenten, wie z. B. Silberfedern, Silberspiralen, Silberkugeln, etc., welche sich in der Flüssigkeitssäule befinden können, vorgesehen. In manchen Ausführungsformen können antimikrobielle Beschichtungen der formulierungsberührenden Komponenten vorgesehen sein. In bestimmten Ausführungsformen kann die Flüssigkeit mittels z. B. UV-Licht, welches vom Nasenapplikator ausgehen kann, keimfrei gehalten werden.

In einigen Ausführungsformen kann ein Sensor zur Detektion von mikrobieller Kontamination enthalten sein. Dieser Sensor kann kontaktlos bzw. unter Kontakt arbeiten. Dies kann z. B. mittels optischer Detektion oder mittels einer Sonde erfolgen, insbesondere basierend z. B. auf Zytometrie (Zellvermessung), Refraktometrie (Ausleuchten von Molekülen) oder dergleichen. In bestimmten Ausführungsformen können in festgelegten Zeitabständen kleine Proben entnommen werden und mittels Massenspektrometrie, chemischer Synthese oder Fluoreszenz-in-situ-Hybridisierung analysiert werden.

In manchen Ausführungsformen sind im Gebrauch Filtermembranen in der Flüssigkeitssäule zwischen Substanzreservoir und Einweg-Nasenaufsatz angeordnet. Die Filtermembranen können in Serie, parallel oder als Labyrinth angeordnet sein.

In einigen Ausführungsformen können ein oder mehrere mechanische Filter Verwendung finden, wie z. B. 22 µm Filter. Die Filter sind in manchen Ausführungsformen im Bereich der Anschlussstelle des Einweg-Nasenaufsatzes angeordnet. In einigen Ausführungsformen werden zwei oder mehr Filter verwendet, von denen einer auf dem Einweg-Nasenaufsatz vorgesehen ist, und wobei der andere endseitig oder stromab der Anschlussstelle für das Substanzreservoir angeordnet und/oder stromab der Anschlussstelle für den Einweg-Nasenaufsatz angeordnet ist. Die Filter können vorgesehen sein, um eine Kontamination des Substanzreservoirs und/oder das Bilden von Luftblasen zu verhindern. Der Filter kann ein Sinterfilter sein.

In manchen Ausführungsformen kann der Nasenapplikator nach Entfernen des benutzen Einweg-Nasenaufsatzes, z. B. automatisch, eine Spülung durchführen, bevor ein neuer Einweg-Nasenaufsatz aufgesetzt wird. In einigen Ausführungsformen kann die für die Spülung benötigte Flüssigkeitsmenge automatisch in einem separaten Reservoir gesammelt werden und/oder einfach z. B. in eine Mullbinde gespritzt werden, um anschließend als kontaminierter Sondermüll entsorgt zu werden.

Hierbei der Impfstoff selbst als Spülflüssigkeit verwendet werden. Alternativ oder ergänzend kann in manchen Ausführungsformen eine neutrale Flüssigkeit, welche mittels Ventilen zugeschaltet wird als Spülflüssigkeit verwendet werden. Diese Spülflüssigkeit wird in diesen Ausführungsformen aus einem eigenen Reservoir entnommen, welches vorgesehen und z. B. im Nasenapplikator angeordnet sein kann.

Die Anschlussstelle zwischen Einweg-Nasenaufsatz und Substanzreservoir kann mittels unterschiedlicher Anschlüsse realisiert werden. Hierfür kann in einigen Ausführungsformen z. B. ein Schnellverschluss, ein Schnappverschluss, ein Luer-Lock, eine Konusverbindung, eine Schraubverbindung oder dergleichen vorgesehen sein.

In einigen Ausführungsformen kann der Nasenapplikator eine zweite Kartusche mit einer Trägermatrix aufweisen oder hiermit in Fluidkommunikation verbunden sein. Die Trägermatrix, oder das Trägermaterial, wird vor der Substanz, z. B. dem Impfstoff, oder gleichzeitig hiermit appliziert oder mit der Substanz, z. B. dem Impfstoff, gemischt. Diese Trägermatrix oder dieses Trägermaterial ermöglicht es, dass der Wirkstoff länger auf der Nasenschleimhaut verweilt und somit vorzugsweise die Immunreaktion steigert.

In manchen Ausführungsformen kann sich in der zweiten Kartusche z. B. ein Katalysator oder Permeationsverstärker (Permeationsenhancer) befinden, welcher die Nasentraktschleimhaut anregen und die Immunreaktion verbessern kann. Beispiele für einen solchen Katalysator oder Permeationsverstärker sind hydrophiles Polyethylenglykol, Chitosan, Natriumdodecylsulfat, Lauroylcarnitin, N-Succinyl-Chitosan, AT-1002, Methyl-β-cyclodextrin, etc.

In einigen Ausführungsformen weist der Nasenapplikator ein vergleichsweise großes Substanzreservoir von z. B. 20 ml oder mehr Volumen auf. Ein solches Substanzreservoir kann z. B. den Impfablauf beschleunigen helfen, da es nicht mehr notwendig ist, einzelne Dosen jeweils mit einer Spritze z. B. aus einem Vial aus Glas aufzuziehen.

In manchen Ausführungsformen können manche oder alle Parameter der Applikation der Substanz vom Nasenapplikator oder hiermit in Signalverbindung stehenden Vorrichtungen aufgezeichnet werden, was insbesondere bei Impfstoffen von Nutzen auch bei der erforderlichen Dokumentation sein kann. Die Parameter können z. B. Ort, Zeit, Temperatur, Arzt, Vitalparameter des Patienten, etc. beinhalten. Mit dieser Ausführungsform kann beispielsweise eine automatisierte, lückenlose Chargenverfolgung ermöglicht werden.

In einigen Ausführungsformen kann der Nasenapplikator einen integrierten Kühlkreislauf beinhalten, welcher die Substanz auf der entsprechenden Lagertemperatur hält. In anderen Ausführungsformen kann der Nasenapplikator automatisch die Substanz, z. B. einen Impfstoff, auf einer gewünschten, insbesondere konstanten, Temperatur halten, wie z. B. 20°C [Grad Celsius].

In manchen Ausführungsformen kann der Nasenapplikator ausgestaltet sein, um die Dosierkammer zum Verändern ihres Volumens linear zu verfahren.

In einigen Ausführungsformen enthält der Einweg-Nasenaufsatz den Impfstoff bereits. Nach seinem Abfüllen wird er z. B. oben und unten hermetisch versiegelt, z. B. durch Aluminiumfolie. Somit kann nichts verdunsten oder auf andere Weise verloren gehen, und es kann keine Luft eintreten und Oxidationvorgänge in Gang setzen. Auch anderweitige Kontamination während der gesamten Lagerungszeit kann auf diese Weise vorzugsweise vermieden werden.

In manchen Ausführungsformen befindet sich, z. B. in einem unteren oder stromaufwärts gelegenen Abschnitt des Einweg-Nasenaufsatz ein Überdruckventil, das z. B. bei 4 bar Druck öffnet. Durch Koppeln/Verbinden des Einweg-Nasenaufsatzes mit dem Gehäuse des Nasenapplikators und z. B. nach Betätigen der hierin diskutierten Abrufvorrichtung oder eines anderen Mechanismus oder Schalters fährt der Kolben, welcher die Bestückungs- bzw. Ausbringvorrichtung umfasst, nach vorne. Dabei wird die Luft in der Dosierkammer komprimiert. Bei 4 bar öffnet das Ventil im Einweg-Nasenaufsatz und bringt somit die Impfdosis aus. Dies eröffnet eine Möglichkeit, auch nur geringe Substanzen, z. B. Impfstoff, exakt dosiert zu applizieren. In diesen Ausführungsformen muss lediglich vor dem Aufsetzen des Einweg-Nasenaufsatzes auf das Handstück das obere und untere Aluminiumsiegel abgenommen oder ggf. durchstochen werden.

In manchen Ausführungsformen kann anstelle des Substanzreservoirs eine Ansaugleitung für Umgebungsluft, vorzugsweise mit einem Luftfilter, in der Ansaugleitung oder an beliebiger Stelle innerhalb oder an der Leitung vorgesehen sein. Beim Zurückfahren der Bestückungsvorrichtung wird z. B. der maximale Weg zurückgelegt und dabei optional gefilterte oder gar steril gefilterte Luft in die Dosierkammer gesaugt. Beim Umkehren der Bewegung in die Schubrichtung wird die gefilterte Luft komprimiert und dann durch die Ausbringvorrichtung durch den Einweg-Nasenaufsatz gedrückt, sobald das integrierte Druckventil bei den beispielhaften 4 bar öffnet. Die Substanz, z. B. der Impfstoff, welche sich im Einweg-Nasenaufsatz befindet, wird somit vorteilhafterweise praktisch restlos appliziert.

In einigen Ausführungsformen kann die Substanz ebenfalls variabel dosiert werden, indem nur ein Teil der vorhandenen Substanz ausgebracht wird, beispielsweise indem nur ein Teil des maximal möglichen Hubwegs ausgenutzt wird und somit die generierte Druckluft reduziert wird. Würde die maximale Dosis beispielsweise 150 µL betragen, wenn man die ganze vorhandene, vorabgefüllte Substanzmenge appliziert, so kann manuell oder mittels Algorithmus auch eine hiervon abweichende, individuelle Dosis abgeben werden, z. B. nur 100 µL, indem nicht der ganze Hubweg verfahren wird.

In manchen Ausführungsformen kann der Primingprozess, sprich das Entlüften des Nasenaufsatzes bzw. des Systems, vorteilhaft entfallen, z. B durch das Verwenden einer größeren Dosierkammer. Beispielsweise könnte eine Dosierkammer ein Volumen von 300 µL aufweisen, von denen 150 µL mit Substanz für die Applikationsdosis verwendet werden und die verbleibenden 150 µL mit Substanz befüllt werden, um mit ihnen beim Ausbringen der 300 µL Substanz ein Totvolumen, hier beispielhaft 150 µL, des Einweg-Nasenaufsatzes zu befüllen, insbesondere zu spülen oder primen. Die Steuerung des Nasenapplikators kann entsprechend programmiert sein, um die Dosierkammer entsprechend mit mehr Substanz bestücken zu lassen, als für die Applikationsdosis erforderlich. Das Wegfallen des Primingprozesses, wofür die über die Applikationsdosis hinausgehende Menge an Substanz zusätzlich aus der Dosierkammer gefördert wird, kann vorteilhaft beispielsweise einen Impfarzt oder jene Person, welche die Applikationsdosis appliziert, entlasten, da nicht mehr vorab geprimt werden muss.

In einigen Ausführungsformen wird durch die Kombination von komprimierter Luft und Flüssigkeit die Geschwindigkeit der generierten Tröpfchenwolke beeinflusst. Dies kann den Vorteil haben, dass tiefere Regionen bzw. die Zielregion des Nasentrakts besser erreicht werden können.

In manchen Ausführungsformen ist der Nasenapplikator vorgesehen und geeignet, bereits am Markt erhältliche, herkömmliche Einzeldosissysteme (z. B. wie auch das als UnidoseTM bekannte) anzutreiben oder allgemein zu verwenden. In diesen Ausführungsformen kann der Aktuator, z. B. die Ausbringvorrichtung bzw. Bestückungsvorrichtung, etwa in Gestalt des optionalen Elektromotors samt Spindel, die erforderliche manuelle Auslösung dieser Unidose-Syteme übernehmen. Insbesondere kann ein Unidose-System auf den Nasenapplikator, z. B. mittels einer Klickverbindung, aufgesteckt und mit der Ausbringvorrichtung bzw. Bestückungsvorrichtung, oder einem Linearaktuator allgemein, welcher z. B. die Bestückungs- bzw. Ausbringvorrichtung antreibt, verbunden werden. Durch das Betätigen der Abrufvorrichtung verfährt z. B. die Ausbringvorrichtung bzw. Bestückungsvorrichtung mit den gewünschten Auslöseparametern und löst somit das Unidose-System aus. Man hat somit ein Substanzreservoir in Form der Einzeldosis, in dem sich die flüssige Substanz mit dem Wirkstoff befindet, und stromauf hiervon einen beweglichen Stopfen sowie stromab einen Tröpfchengenerator. Wird mittels der Bestückungs- bzw. Ausbringvorrichtung der Stopfen verschoben in Richtung Substanz, wie bei einer klassischen Spritze üblich, so wird die Substanz im Substanzreservoir druckbeaufschlagt und zum Tröpfchengenerator befördert, welcher dann die flüssige Substanz in eine Tröpfchenwolke (Aerosol) umwandelt.

In einigen Ausführungsformen befindet sich die auszubringende Substanz in dem in diesen Ausführungsformen bis auf eine Öffnung verschlossenen Substanzreservoir. Der die Öffnungen zumindest zunächst verschließende Stopfen grenzt die Substanz gegenüber dem Tröpfchengenerator ab. Beim Auslösen wird das Substanzreservoir in Richtung zum Tröpfchengenerator oder auf diesen zu bewegt. Dabei werden der Stopfen und somit auch die flüssige Substanz mit Druck beaufschlagt. Im Stopfen, seitlich davon oder an anderer geeigneter Stelle kann ein Kanal vorgesehen sein, der erst ab einem gewissen Druck die flüssige Substanz durch ihn hindurch zum Tröpfchengenerator passieren lässt, wo die Substanz zu einer Tröpfchenwolke (Aerosol) umgewandelt wird.

In manchen Ausführungsformen kann der Stopfen ab einen vorbestimmten Verfahrweg des Stopfens in Kontakt mit einer zumeist ortsfesten Kanüle gelangen und von deren Spitze durchdrungen werden. Somit kann sich die im Substanzreservoir befindende und unter Druck stehende Substanz zum Tröpfchengenerator bewegen.

Mittels mancher erfindungsgemäßer Ausführungsformen können einer oder mehrere der hierin genannten Vorteile erzielbar sein, zu welchen folgende zählen:
Die Arzneitherapie wird durch die Tatsache erschwert, dass Patienten auf dieselbe Dosis derselben Substanz sehr unterschiedlich reagieren. Es finden sich Unterschiede in der Wirksamkeit, sowie im Spektrum und Schweregrad von unerwünschten Wirkungen und Nebenwirkungen.

Ein wesentlicher Anteil der interindividuellen Variabilität (d. h. Unterschiede zwischen verschiedenen Patienten) einzelner Arzneistoffe kann auf die genetische Ausstattung (Pharmakogenetik) des Patienten zurückgeführt werden. Eine identische Dosierung bei unterschiedlichen Patienten führt zu sehr unterschiedlichen Konzentrations-Zeit-Kurven (Pharmakokinetik) und damit zu sehr unterschiedlichen Wirkungen (Pharmakodynamik) und somit ist eine Dosisindividualisierung vorteilhaft. Zusätzlich zu der Interindividuellen Variabilität sollte man die intraindividuelle Variabilität d. h. Unterschiede bei einem einzelnen Patienten in eine Arzneitherapie mitberücksichtigen. Die vorliegende Erfindung kann hierzu einen Beitrag leisten.

Insbesondere bei Mehrfachgabe von Substanzen mit enger therapeutischer Breite, langer Eliminationsphase und Nebenwirkungen muss der Kumulationseffekt (Akkumulation bzw. Anreicherung der Substanz) über die Gabe mehrerer, aufeinanderfolgender Applikationsdosen berücksichtigt werden. Die vorliegende Erfindung kann erlauben, eine für den Benutzer sichere Applikation einzelner oder mehrerer solcher Applikationsdosen durch diesen selber durchführen zu lassen.

Letzteres gilt insbesondere für ältere Menschen, für die eine korrekte bzw. individuell angepasste Dosierung eine ausschlaggebende Rolle spielt. Die vorliegende Erfindung kann hierzu einen Beitrag leisten.

Das vorsichtige "Titrieren" (was in einer Bestimmungs- oder Findungsphase erfolgt) auf eine Substanzkonzentration und das Einstellen der unterschiedlichen Applikationsdosen bzw. des therapeutischen Effekts unter Vermeiden eines "Überschießens", und die damit verbundene Vermeidung von unerwünschten Nebenwirkungen kann einen weiteren Vorteil darstellen.

Hierzu kann das Regeln auf die Zielkonzentration (C_{ED}) von Vorteil sein. Im Fall der Schmerztherapie steht das Erreichen der Zielkonzentration für einen Zustand, in welchem der Patient mit seine Schmerztherapie zufrieden ist. Eine über die Zielkonzentration hinausgehende Konzentration trägt nicht mehr zur therapeutischen Wirkung bei, sondern erhöht nur mehr die zu vermeidenden Nebenwirkungen.

Die vorliegende Erfindung kann es erlauben, applikationsbedingte Problemstellungen von Nasenapplikatoren, wie die unterschiedliche Nasenanatomie, der Nasenzustand, der Applikationswinkel, wie tief der einzelnen Benutzer gewöhnlich den Nasenaufsatz einführt und dergleichen, beheben: eine voreingestellte Höhe der Applikationsdosis, deren Wirkung im Körper von den vorgenannten und weiteren Faktoren abhängt, ist erfindungsgemäße nicht erforderlich. Die vorliegende Erfindung ist geeignet, auf solche, vor allem interindividuelle, Besonderheiten bei der Vorbestimmung der Höhe der Applikationsdosen einzugehen. Somit kann der gewünschte therapeutische Effekt gesichert eintreten.

Wird die Substanz, wie ebenfalls von der Erfindung umfasst, nasal appliziert, also nicht invasiv, so können Nachteile anderer Applikationsformen vermieden werden. Bei der oralen Gabe beispielsweise muss die Substanz erst über die Leber metabolisiert werden (First-Pass Effekt) bevor sie ihre Wirkung entfalten kann. Weitere Nachteile sind der verhältnismäßige langsame Wirkeintritt und die Schwierigkeit individuelle Dosen zu verabreichen. Erschwerend für die orale Gabe sind Symptomatiken wie Schluckstörung und trockener Mund. Bei der sublingualen Gabe beispielweise sind die relative kleine Absorptionsfläche und das versehentliche Verschlucken problematisch. Die Wirkung wird ebenfalls beeinträchtigt durch zu frühes Trinken, Essen oder Sprechen nach der sublingualen Applikation.

Die intranasale Verabreichung mittels des erfindungsgemäßen Nasenapplikators ist schmerzfrei, leicht selbstverabreichbar, und wirkt rasch.

Das substanzbezogene Sperren weiterer Applikationen während einer Sperrdauer, welche überdies variabel und erfindungsgemäß von der Sperrvorrichtung in Beginn und Dauer einstellbar ist, kann ein zeitiges Finden der Zielkonzentration erlauben, und kann eine überschießende Gabe der Substanz vorteilhaft verhindern. Die Sperrvorrichtung stellt sicher, dass die applizierte Dosis ihren vollen Effekt etablieren kann und dadurch die Wirkung vom Benutzer bewertet werden kann.

Erfindungsgemäß wird somit vorteilhaft u. a. ein selbstregulierendes System vorgeschlagen, welches sich nach einer "Titrationsphase" (welche eine Bestimmungs- oder Findungsphase ist) anhand bereits abgegebener Applikationsdosen, das Betätigungsverhaltens des Benutzers und den vergangenen Zeitintervallen individualisierte bzw. personalisierte Applikationsdosen, bei einer Mehrfachdosierung, bestimmt und abgibt, welche den Bedürfnissen des Benutzers, entsprechend bzw. den gewünschten therapeutischen Effekt bewirkend, entspricht.

Erfindungsgemäß wird der Körper des Benutzers als Regelkreis bzw. Regelstrecke verwendet, da nur der Körper des Benutzers wiedergibt was die Substanz mit dem Körper macht (Pharmakodynamik) und die jeweilige Applikationsdosis bei Anfrage/Bedarf benutzerindividuell bestimmt wird und appliziert werden kann.

Erfindungsgemäß kann ferner unter Nutzung der Eigenschaften der Pharmakokinetik der Benutzer kategorisiert und der zeitliche Verlauf der Konzentration im Körper verfolgt werden. Nach einer initialen "Titrationsphase" kann man für den entsprechenden Benutzer zuordnen, ob er innerhalb z. B. der Gruppe der Pharmakokinetik zum Mittelwert oder "-SD" oder "+SD" tendiert und/oder seine individuelle Konzentrations-Zeit-Kurve definieren. Da erfindungsgemäße auch die Zielkonzentration, bei der der gewünschte therapeutische Effekt auftritt, ermittelt werden kann, leistet die vorliegende Erfindung vorteilhaft einen Betrag zur Weiterbildung der personalisierten Medizin.

Interindividuelle Variabilität (z. B. Alter, Vorerkrankungen, Medikamentenverbrauch) und intraindividuelle Variabilität (z. B. Manipulation, Mobilisation, zirkadianer Verlauf, Verbesserung des Krankheitszustands bzw. Genesungszustands) können von der Steuervorrichtung vorteilhaft berücksichtigt werden.

Ein Tätigwerden der Ärztin oder anderes berechtigtes Personal ist vorteilhafterweise hierbei nicht erforderlich, was Zeit und Ressourcen einspart.

Schließlich kann die vorliegende Erfindung dazu beitragen, dass vorteilhafterweise eine geringere Menge an Substanz zum Erzielen desselben Effekts nötig ist.

Ein "Abfangen" der Restmedikation z. B. aus dem OP, die als Ausgangs- oder Initialdosis abgegeben worden sein kann, ist erfindungsgemäß ebenfalls vorteilhafterweise möglich.

Der effizienteste Bereich für die Arzneimittelabsorption ist die stark vaskularisierte Seitenwand der Nasenhöhle. Die Antigenkonzentration, die verabreichte Dosis und das Verabreichungsvolumen sind drei zusammenhängende Parameter, die die Leistung der nasalen Antigenabgabe beeinflussen. Ein weiterer Vorteil der vorliegenden Erfindung kann darin bestehen, dass die nasale Immunisierung wesentlich niedrigere Antigendosen im Vergleich zum oralen, subcutanen oder intramuskulären Weg benötigt. Dies kann helfen Kosten einzusparen.

Ferner kann mittels der vorliegenden Erfindung eine bessere Patienten-Compliance aufgrund einer nadelfreien Arzneimittelverabreichung erreicht werden.

Ein weiterer Vorteil kann darin bestehen, dass Arzneimittel mittels der vorliegenden Erfindung für den Patienten schmerzfrei appliziert werden können. Auch dies kann helfen die Patienten-Compliance zu erhöhen und im optimalen Falle auch hierdurch die Immunisierung großer Bevölkerungsgruppen vorteilhaft einfach ermöglichen oder begünstigen. Speziell für Kinder stellt die nasale Arzneimittelapplikation mittels der vorliegenden Erfindung vorteilhaft eine schmerzfreie Alternative dar. Es gibt keine Reaktionen an der Einstichstelle wie bei der intramuskulären Gabe. Dies kann helfen, das Wohlbefinden der kleinen Patienten zu erhöhen.

Zudem können Verletzungen, zumeist des medizinischen Personals, welche beispielsweise durch die Applikationsnadel (needle stick) verursacht werden können, verhindert werden.

Mittels der vorliegenden Erfindung wird vorteilhafterweise die Übertragung von Krankheiten, die hauptsächlich über unzureichend sterile Nadeln übertragen werden, verhindert.

Mittels der Applikation per Nasenapplikator der vorliegenden Erfindung werden die zahlreichen im Nasenepithel vorhandene Mikrovilli als bessere Absorptionsfläche vorteilhaft genutzt.

Ein weiterer Vorteil der vorliegenden Erfindung kann darin bestehen, dass die intranasale Immunisierung einfach, leicht, bequem, effektiver und sicherer als andere Verabreichungswege, wie z.B. die orale, sublinguale, subkutane, intramuskuläre, rektale, vaginale, inhalative oder parenterale Gabe ist. Es gibt darüber hinaus verschiedene Darreichungsformen, um einen nasalen Impfstoff zu verabreichen, beispielsweise Tropfen, Pulver, Aerosol, Emulsion, Suspension und/oder Gels, was wiederum einen Vorteil darstellen kann.

Mittels der vorliegenden Erfindung, insbesondere für deren automatisierte Abgabe von Arzneimitteln, ist kein Kraftaufwand für die Applikation der Impfdosis notwendig. Vorteilhafterweise muss lediglich eine elektronische Abrufvorrichtung, beispielsweise in Form eines Knopfs oder Tasters, betätigt werden. Ein weiterer Vorteil kann darin bestehen, dass die Auslöseparameter reproduzierbar, also immer gleich, sind und aufgezeichnet werden können.

Mittels der vorliegenden Erfindung kann weiter vorteilhaft kontaminierter Müll vermieden werden, welcher gesondert behandelt und verwertet werden müsste. Ferner wird "normaler" Abfall reduziert, indem die Desinfektion einer Einstichstelle und das Anbringen eines Schutzpflasters entfallen. Dies kann helfen, Kosten einzusparen.

Mittels der vorliegenden Erfindung kann eine Substanz vorteilhafterweise mehreren Patienten (hintereinander, oder beispielsweise im Tagesablauf) appliziert werden. Somit ist der Nasenapplikator und/oder seine Einweg-Nasenaufsätze, insbesondere wie hierin beschrieben, vorteilhaft auch für Einsätze z. B. in der Notaufnahme, beim Rettungsdienst, bei Militäreinsätzen, in einem Lazarett, von Sanitätern, in Tagesklinken (day surgeries) und/oder Pflegeheimen, bei der Pflege zu Hause, in der mobilen Pflege, bei Katastropheneinsätzen, etc. geeignet.

Im Folgenden wird die Erfindung anhand exemplarischer Ausführungsformen derselben unter Bezugnahme auf die angehängte Zeichnung beschrieben. In den Figuren gilt:
- **Fig. 1**: zeigt einen Nasenapplikator als Beispiel einer Medikamentenapplikationsvorrichtung einer ersten exemplarischen Ausführungsform, sowie ein erfindungsgemäßes System;
- **Fig. 2**: zeigt schematisch und exemplarisch einen möglichen zeitlichen Verlauf einer Nutzung des erfindungsgemäßen Nasenapplikators als Beispiel einer Medikamentenapplikationsvorrichtung;
- **Fig. 3**: zeigt schematisch und exemplarisch die Höhe kumulierter Dosen verschiedener Applikationen einer Substanz an den Benutzer;
- **Fig. 4**: zeigt schematisch und exemplarisch das Ergebnis einer Vorbestimmung der Höhe der verschiedenen Applikationsdosen;
- **Fig. 5a**: zeigt schematisch anhand dreier exemplarisch ausgewählten zeitlichen Konzentrationsverläufe das Ermitteln eines individuellen Konzentrationsverlaufs für einen betrachteten Benutzer der erfindungsgemäßen Nasenapplikators;
- **Fig. 5b**: zeigt eine Fortführung des der Erfindung in manchen Ausführungsformen zugrundeliegenden Gedankens der Fig. 5a; und
- **Fig. 6**: zeigt einen Nasenapplikator einer weiteren exemplarischen Ausführungsform.

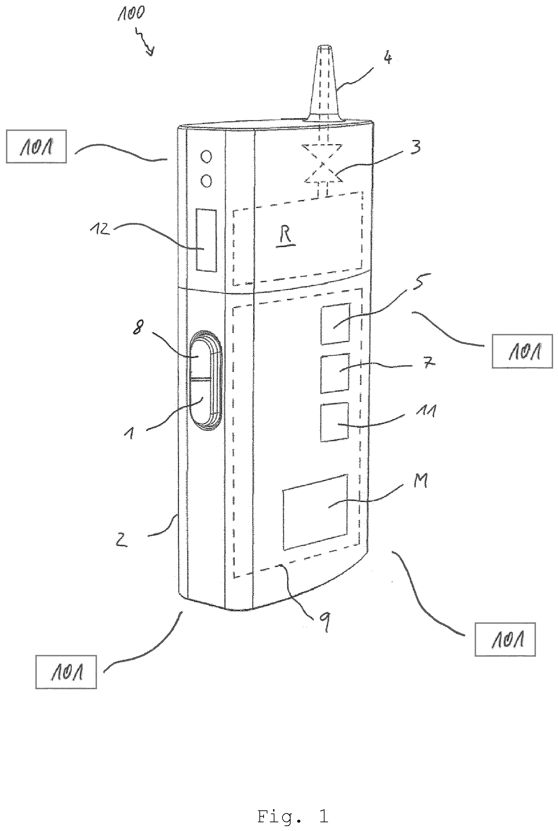

**Fig. 1** zeigt einen Nasenapplikator 100 einer ersten exemplarischen Ausführungsform in einer schematisch vereinfachten Darstellung.

Wie Fig. 1 zeigt, weist der Nasenapplikator 100 ein Gehäuse 2 auf, in oder an welchem optional eine, alle oder mehrere der im Folgenden genannten Vorrichtungen oder Komponenten unabhängig voneinander angeordnet sein können. In anderen Ausführungsformen können einzelne oder mehrerer dieser Komponenten in beliebiger Kombination auch extern, also nicht innerhalb des Gehäuses 2, vorliegen.

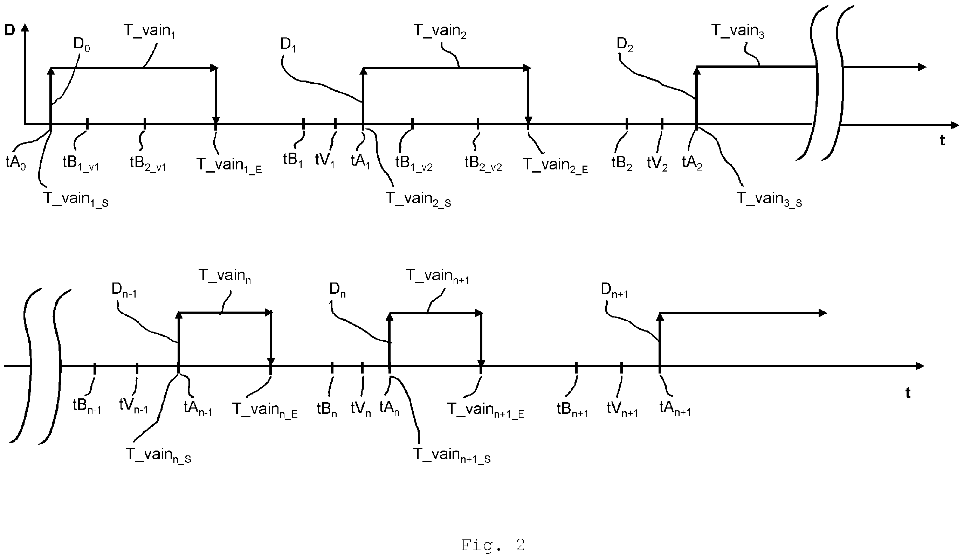

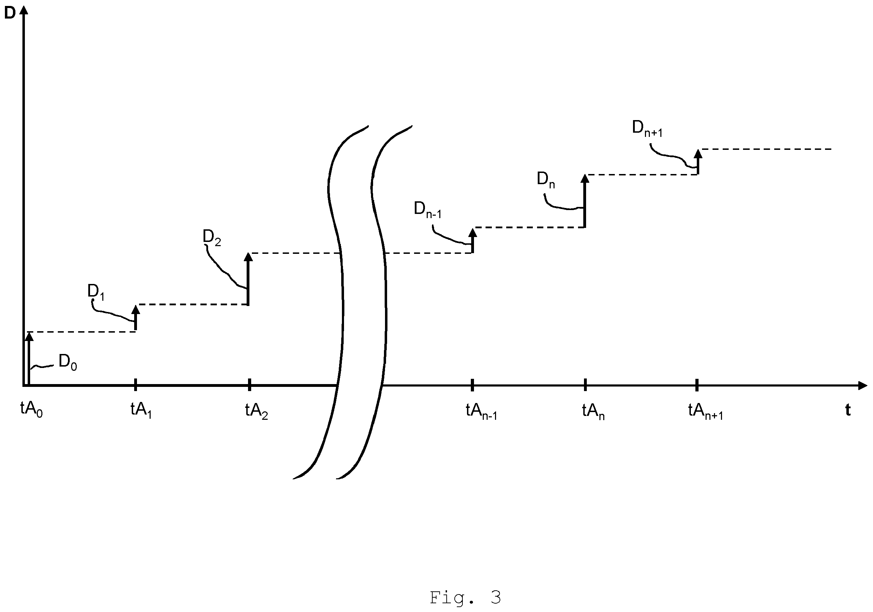

Der Nasenapplikator 100 weist eine Abrufvorrichtung 1 auf, welche der Benutzer zu betätigen hat, um sich eine Dosis D (siehe Fig. 2 oder 3) einer Substanz S aus einem Substanzreservoir R applizieren zu können. Die Substanz S kann ein medizinischer oder nicht-medizinischer Wirkstoff, insbesondere ein Schmerzmittel, sein. Die Abrufvorrichtung 1 kann zugleich eine optional vorgesehene Feedbackvorrichtung 8 sein oder aufweisen. Die Feedbackvorrichtung 8, sofern vorhanden, kann alternativ hierzu eine eigenständige Vorrichtung sein.

Hat der Nutzer die Abrufvorrichtung 1 betätigt, welche ein Schalter, ein Knopf, ein Einschub oder dergleichen sein kann, und damit ein Betätigungssignal ausgelöst, so kann ein Abgabedosierer 3 eine von ihm dosierte, also mengenmäßig oder volumenmäßig bestimmte, Menge der Substanz S freigeben. Sie kann aus dem Substanzreservoir R über das Lumen eines in ein Nasenloch einzuführenden Nasenaufsatzes 4 (in die Nase des Benutzers einströmen, einspritzen oder dergleichen. Sie kann alternativ aus dem Substanzreservoir R über einen Tröpfchengenerator in die Nase appliziert werden, der Teil des Nasenaufsatzes 4 oder einer anderen Einrichtung des Nasenapplikators 100 sein kann.

Eine, insbesondere optische oder haptische, Anzeige 12, z. B. Display, LED, usw. kann vorgesehen sein.

Zusätzlich oder ergänzend zu einer optischen Anzeige kann ein akustischer Melder, wie ein Lautsprecher, Buzzer, Piepser, ein haptischer Melder z. B. mittels Vibration, oder dergleichen vorgesehen sein.

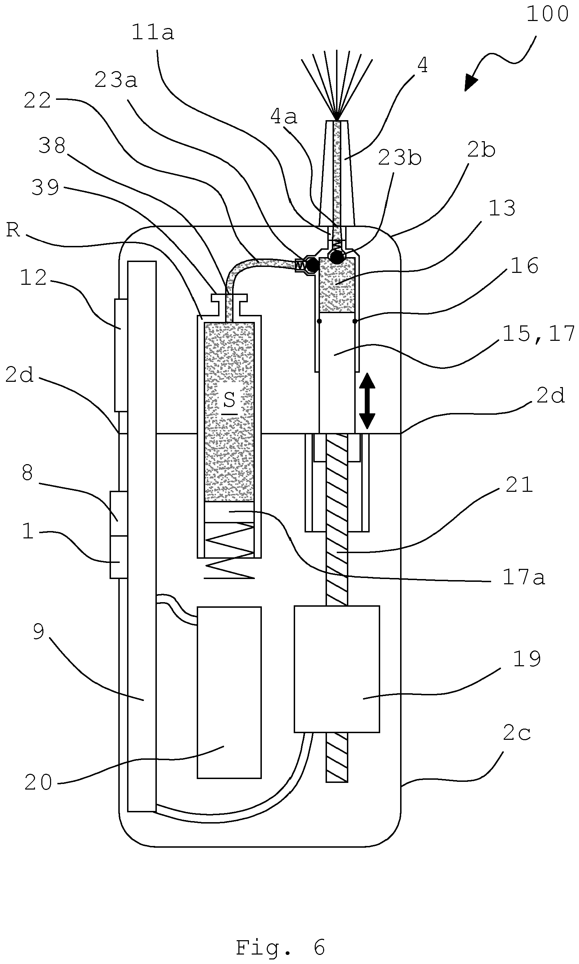

Auf das Substanzreservoir R kann hierfür optional mittels eines Energiebeaufschlagungsmechanismus oder einer anderen Ausbringvorrichtung, siehe z. B. Fig. 6, eingewirkt werden. Dieser kann mittels Druck, Kraft, Geschwindigkeit, Schwingung, Vibration, Rotation, elektrische/magnetische Kräfte, usw. wirken. Letztgenannte Kräfte oder physikalischen Ereignisse können z. B. die Auslösegeschwindigkeit, die Kraft, den Druck, die Beschleunigung, die Flussrate und damit die Parameter der Ausbringvorrichtung, z. B. eines Tröpfchengenerators, beeinflussen, sprich das Ergebnis des Tröpfchengenerators beeinflussen. Der Tröpfchengenerator, der beispielsweise Teil des Abgabedosierers 3 oder des Nasenaufsatzes 4 sein kann, kann somit beispielsweise ein Druckbeaufschlagungsmechanismus sein, der auf die Substanz S einwirkt; z. B. drückt ein Kolben in das Substanzreservoir R, oder eine Pumpe zieht aus dem Substanzreservoir R und beaufschlagt die Substanz S mit Druck, Kraft, Geschwindigkeit, usw. und schiebt diese durch den Tröpfchengenerator.

Der optionale Tröpfchengenerator kann mechanisch und/oder elektrisch funktionieren. Er kann z. B. als Düse, Verdampfer, Piezo-Element, usw. ausgeführt sein, oder dergleichen aufweisen. Er kann vorzugsweise einen Sprühnebel oder eine Tröpfchenwolke oder eine Partikelwolke erzeugen.

Sollte eine solche Freigabe durch den Abgabedosierer 3 nicht zu jedem Zeitpunkt und nicht aufgrund jeder Betätigung der Abrufvorrichtung 1 und/oder nicht zu jedem Zeitpunkt möglich sein, so kann optional eine Sperrvorrichtung 5 vorgesehen sein. Sie ist vorzugsweise konfiguriert, um, vorzugsweise zeitgesteuert, eine Freigabe oder Abgabe mittels des Abgabedosierers 3 während einer nach vorbestimmten Kriterien hinsichtlich ihrer Dauer und/oder des Zeitpunktes ihres Beginns und/oder Endes festgelegten Sperrdauer (siehe z. B. T_vainₙ in Fig. 2) zu verhindern, nicht zuzulassen, usw., selbst wenn die Abrufvorrichtung 1 während der Sperrdauer betätigt werden sollte. Während der Sperrdauer, welche in einigen Ausführungsführungsformen von fester oder unveränderlicher Dauer ist, während sie in anderen variabel oder veränderbar ist, führt eine Betätigung der Abrufvorrichtung 1 grundsätzlich oder generell nicht zu einer Applikation, wohingegen dies für eine Betätigung nach Ablauf der Sperrdauer nicht zutreffen muss oder nicht zutrifft.

Die Menge oder das Volumen jenes Teils der Substanz S, welcher in einzelnen Ausgangs- oder Applikationsdosen (Do, D₁, D₂ Dₙ, ... in Fig. 2 oder in Fig. 3) wie nachfolgend erläutert ist, zu verschiedenen Abgabezeitpunkten tA₀, tA₁, tA₂, ..., tAₙ, ... abgegeben wird und/oder abgegeben wurde, kann mittels einer optionalen Dosiserfassungsvorrichtung 7 erfasst werden. Die Dosiserfassungsvorrichtung 7 kann konfiguriert sein, um die einzelnen Ausgangs- oder Applikationsdosen D₀, D₁, D₂,..., Dₙ, ..., welche mit dem Nasenapplikator 100 abgegeben wurden und welche variabel, also keine fixen Dosismengen sind, zu messen, festzustellen, zu speichern, aufzusummieren usw.

Die Dosiserfassungsvorrichtung 7 kann beispielsweise eine Menge, ein Volumen oder einen anderen Parameter messen, welcher eine unmittelbare Aussage über die jeweilige Applikationsdosis erlaubt. Die Dosiserfassungsvorrichtung 7 kann ergänzend oder alternativ einen Parameter messen oder ermitteln, welcher eine mittelbare Aussage über die jeweilige Applikationsdosis erlaubt, etwa einen Fluss pro Zeit, eine Anzahl von Hüben einer Pumpe, eine Anzahl von Umdrehungen einer Pumpe, oder dergleichen.

Die Dosiserfassungsvorrichtung 7 oder eine andere Vorrichtung, etwa die elektronische Steuervorrichtung 9, kann konfiguriert sein, z. B. die Auslöseparameter wie z. B. die Geschwindigkeit, die Beschleunigung, die Kraft, den Weg, etc. zu erfassen Derartige, erfasste oder ermittelte Werte werden optional gespeichert.

Dosislimits und/oder Konzentrationslimits, welche eine, z. B. bezogen auf eine Zeiteinheit beschränkte oder vorbestimmte, Gesamtdosis und/oder Gesamtkonzentration beschränken, können optional vorgesehen sein.

Die Dosiserfassungsvorrichtung 7 kann, falls vorhanden, ergänzend oder alternativ programmiert sein, um einen Zeitpunkt, zu welchem eine oder mehrere, konkrete Applikationsdosen mittels des Nasenapplikators 100 verabreicht wurden oder werden, festzuhalten. Die Dosiserfassungsvorrichtung 7 kann weiterhin ergänzend oder alternativ programmiert sein, um eine Zeitdauer zu erfassen, beispielsweise zwischen zwei oder mehreren Abgabezeitpunkten tA₀, tA₁, tA₂,..., tAₙ, ... (siehe Fig. 2 oder Fig. 3) verabreichter Ausgangs- oder Applikationsdosen D₀, D₁, D₂,..., Dₙ, .... Weitere Zeitdauern zwischen beliebigen hierin genannten Zeitpunkten können ebenfalls erfasst werden (wie z. B. tA₁ -/"minus" T_vain_{1_E}, tA₁ - (insbesondere letztes) tB_{_v}, tB_{1_v1} - tA₀, tB_{_v} (insbesondere das letzte, also tB_{_v-1}) - tA₀, T_vain_{1_E} - letzte tB_{_v}, T_vain_{1_E} - tB_{1_v1}, welche deren Parameter nachstehen erläutert sind.

Eine elektronische Steuervorrichtung 9 kann optional vorgesehen sein, um die Funktion des Nasenapplikators 100 oder einige, beliebige oder alle der mit Bezug auf Fig. 1 genannten Vorrichtung oder Komponenten zu steuern, zu regeln und/oder zu überwachen. Sie kann, wie vorstehend ausgeführt, im Gehäuse 2 oder außerhalb vorgesehen sein. Sie kann, rein exemplarisch, Empfänger von Angaben über Ausgangs- oder Applikationsdosen D₀, D₁, D₂,..., Dₙ, ..., sein, die extern vorbestimmt wurden. Entsprechende kabelgebundene oder kabellose Signalverbindungen können, uni-direktional oder bi- oder mehr-direktional, zwischen der Steuervorrichtung 9 und der oder den Komponenten des Nasenapplikators 100 vorgesehen sein.

Eine optionale Erfassungsvorrichtung 11 kann vorgesehen sein, um zu erfassen, ob, dass und/oder wann der Benutzer, der hierin ein Nutzer, Anwender oder Patient sein kann, durch Betätigen der Abrufvorrichtung 1 versucht oder initiiert hat, sich eine weitere Applikationsdosis zu applizieren.

Das Substanzreservoir R kann in jeder Ausführungsform optional austauschbar sein, beispielsweise als ein Single-Shot-Reservoir, als Substanzreservoir mit einer ausreichenden Substanz S für eine Vielzahl von Applikationsvorgängen, usw.

Das Substanzreservoir R kann beispielsweise vorbefüllt, selbstbefüllbar, integriert, austauschbar, für einmalige Applikation und/oder für mehrfache Applikation ausgelegt sein.

Das Substanzreservoir R kann im Inneren des Gehäuses 2 oder extern hierzu angeordnet sein.

Die Substanz S kann jeden Aggregatzustand aufweisen (flüssig, gasförmig, fest, etc.).

Ein optionaler Datenspeicher M zum Speichern von Daten, oder mit hierin gespeicherten Daten, kann im Inneren des Gehäuses 2 oder extern hierzu angeordnet sein.

Diese Daten können den bisherigen Gebrauch des Nasenapplikators 100 durch den Benutzer betreffen, ebenso Zeitangaben zu den hierin genannten Zeitpunkten oder Zeitdauern (synonym: Zeitintervalle) usw.; sie können das Ergebnis einer Auswertung durch die Steuervorrichtung 9 sein, usw. oder optional Daten für pharmakologische, pharmakodynamische, pharmakometrische und/oder pharmakokinetische Daten die Substanz S und/oder zugrundeliegende medizinische Umstände wie die Grunderkrankung usw. betreffend; sie können substanzbezogen und/oder vom Arzt oder anderem berechtigten Personen festgelegt sein, z. B. ein tₘₐₓ-Wert der Substanz S, ein vom Arzt korrigierter, sog. "overruled" tₘₐₓ-Wert, z. B. der Zeitpunkt oder das Ende des Zeitintervalls nach Applikation, zu oder in dem die maximale Konzentration oder Blutkonzentration eintritt, die Halbwertzeit, die terminale Halbwertszeit, ein vom Arzt oder den anderen berechtigten Personen angepasster Wert, usw.

Die Daten können objektiv und/oder subjektiv sein.

Die Daten können objektive Werte (Sensorwerte) sein, z. B. Blutzuckerspiegel, Blutdruck, Herzrate, Atemfrequenz, Sauerstoffsättigung, usw.

Die Daten können subjektive Werte ("Effektwerte") sein, welche man nicht direkt messen kann. Sie werden abgeleitet anhand von erhobenen Werten wie z. B. Schmerzscore, Sedationscore, Nebenwirkungen etc.) und/oder gemessenen Werte (z. B. SpO₂, HR, HRV etc.)

Die Daten können empfohlene Konzentration, Kontraindikationen, Nebenwirkungen, Verfallsdatum, und/oder Beipackzettel, Fachinformation, Patienteninformation, Summary of Product Characteristics (SmPC), Montageanleitung, Demontageanleitung, Betriebsanweisung/Bedienungsanleitung etc. sein oder umfassen.

Die Daten können optional neben pharmakologischen und/oder pharmakokinetischen Daten, benutzerbezogene Daten wie z. B. Gewicht, Alter, Geschlecht, BMI, Vorerkrankungen, Komedikationen, Komorbiditäten, Allergien, etc. umfassen. Generell die Benutzer- und/oder Behandlungshistorie (z. B. Daten zu vorherigen Eingriffen, Prozeduren, Diagnosen, Anamnesen, Tests, erhaltene/verschriebene Medikationen, erhaltene Narkosemittel, Muskelrelaxans, Antiemetika usw.) umfassen.

Solche und andere Daten können der optionalen Steuervorrichtung 9 helfen, genauere Dosierungen zu berechnen, bzw. die Sicherheit der Therapie zu erhöhen.

Solche und andere Daten können Auslöseparameter betreffen, z. B. solche, welcher erforderlich sind, um eine definierte Tröpfchengrößenverteilung zu erhalten. Auslöseparameter können je nach vorbestimmter Dosis unterschiedlich sein. Sie kann man z. B. über die Auslösegeschwindigkeit der Ausbringvorrichtung einstellen.

Der optionale Datenspeicher M kann ein Hardware-Memory-Bauteil, ein Flash-Speicher oder ein externer Datenspeicher sein, beispielsweise angeordnet in einer Cloud, in einem Mobilgerät oder dergleichen und/oder via Wi-Fi/ Bluetooth etc. mit weiteren Komponenten des Nasenapplikators 100 in Signalverbindung stehen.

Die Sperrvorrichtung 5 und/oder die Erfassungsvorrichtung 11 sind vorzugsweise elektronisch, z. B. mittels der Steuervorrichtung 9, verwirklichte Komponenten.

Ein Timer oder Zeitmesser, etwa eine Uhr, kann vorgesehen sein. Die einzelnen Komponenten, wie vorstehend genannt, können auf ihn zurückgreifen.

Mittels des Zeitmessers kann optional z. B. eine absolute Zeitmessung (also basierend auf der richtigen/echten/aktuellen Uhrzeit) erfolgen, oder eine relative Zeitmessung, bei der, vorzugsweise beim Initialisieren oder erstmaligen Einschalten des Nasenapplikators, ein aktueller Zeitstempel erfasst wird, mittels welchem die Zeit zurückbestimmt werden kann.

Es kann in manchen Ausführungsformen vorgesehen sein, den Benutzer über den Ablauf der aktuellen Sperrdauer zu informieren, beispielsweise optisch oder akustisch oder haptisch, etwa mittels Vibration.

Da in Fig. 1 eine Reihe von Peripheriegeräten 101 angedeutet sind, zeigt diese Figur auch ein erfindungsgemäßes System. Die Peripheriegeräte 101 können gleich ausgestaltet sein, oder sich, zumindest einigen von ihnen, voneinander unterscheiden.

So können manche der Peripheriegeräte 101 Nasenapplikatoren sein, die optional wie der Nasenapplikator 100 ausgestaltet sind, und/oder als Docking Station, als Server, als Smart Device usw. ausgestaltet sein, vorzugsweise wie hierin beschrieben.

Optional können manche oder alle der Peripheriegeräte 101 in beliebiger Kombination miteinander oder untereinander kommunizieren, oder nicht.

In Fig. 1 ist angedeutet, dass sie alle auch mit dem Nasenapplikator 100 kommunizieren können, dies ist allerdings ebenfalls optional. In manchen Ausführungsformen können nur manche der Peripheriegeräte 101 dies.

Weiter kann optional vorgesehen sein, dass der Nasenapplikator 100 und/oder die Peripheriegeräte 101 und/oder die Peripheriegeräte 101 über den Nasenapplikator 100 und/oder die Docking-Station und/oder ein anderes Peripheriegerät 101 nach extern kommunizieren können.

Das Substanzreservoir R kann mittels z. B. Luer/Lock, Pin, Nadel, Spike mit oder ohne Belüftung, etc. verbunden sein.

Es kann ein Filter (z. B. ein 22 µm-Partikelfilter), z. B. aus oder mit Aktivkohle, Kunststofffasern, oder dergleichen, oder eine poröse Membran (z. B. einen Porex^{®} - Filter) vorgesehen sein. Dieser Filter kann am Ausgang der Dosierkammer vorgesehen sein. Es können mehr als nur ein Filter vorgesehen sein.

Das Substanzreservoir kann einen Stopfen aufweisen, der nachrücken kann. Damit ermöglicht man eine positionsunabhängige Auslösung des Nasenapplikators.

Alle Materialien können inert oder teilinert sein, z. B. aus oder mit Silikon, Glas, Cyclo Olefin Polymer (COP), Cycloolefin-Copolymere (COC), etc.

Das Substanzreservoir kann eine durchstechbare Membran aufweisen, einen Schraubanschluss, eine Steckanschluss, einen Schnellanschluss, etc.

Das Substanzreservoir kann ein Steigrohr aufweisen.

Das Substanzreservoir kann z. B. eine Beutelflasche, ein "Bag" (wie bei Infusionen) oder ein anderer Behälter sein, welcher kollabieren kann und ein Nachströmen von Luft in das Behältnis nicht benötigt. Mit solchen Lösungen kann man positionsunabhängig auslösen, also in jeder räumlichen Ausrichtung des Nasenapplikators.

**Fig. 2** zeigt schematisch und exemplarisch einen möglichen zeitlichen Verlauf einer Nutzung des erfindungsgemäßen Nasenapplikators 100.

Betätigt der Benutzer die Abrufvorrichtung 1, um sich beispielsweise eine hierin als "nächste" Applikationsdosis bezeichnete Applikationsdosis Dₙ zu applizieren, so wird mittels der elektronischen Steuervorrichtung 9 oder mittels ihrer Erfassungsvorrichtung 11 festgestellt, ob dieser Zeitpunkt, zu dem der Benutzer sich diese nächste Applikationsdosis Dₙ applizieren möchte, möglichweise in eine noch andauernde Sperrdauer T_vainₙ fällt oder nicht.

Fällt dieser Zeitpunkt, zu welchem die Abrufvorrichtung 1 betätigt wird, in die Sperrdauer T_vainₙ, so wird dem Benutzer jedenfalls zu diesem Zeitpunkt keine Applikationsdosis Dₙ appliziert. Solche Zeitpunkte gehen aus Fig. 2 mit Bezug auf eine frühere Sperrzeit T_vain₁ exemplarisch als Zeitpunkte tB_{1_v1} und tB_{2_v1} mit Bezug auf weitere Sperrzeiten z. B. als tB_{1_v2}, hervor und werden optional mit in die Vorbestimmung einbezogen.

Fällt der Zeitpunkt der Betätigung der Abrufvorrichtung 1 hingegen nicht in die Sperrdauer T_vainₙ, sondern liegt nach dem Ablauf oder Ende T_vain_{n_E} der Sperrdauer T_vainₙ wie im Beispiel der Fig. 2 anhand des Betätigungszeitpunkts tBₙ gezeigt, dann wird dem Patienten zu einem hierin als nächster Abgabezeitpunkt tAₙ bezeichneten Zeitpunkt die nächste Applikationsdosis Dₙ appliziert.

Die Höhe der nächsten Applikationsdosis Dₙ wird zu einem hierin als nächsten bezeichneten Vorbestimmungszeitpunkt tVₙ vor ihrer Applikation von der elektronischen Steuervorrichtung 9 festgelegt oder vorbestimmt, die entsprechenden Berechnungen selber ausgeführt oder Ergebnisse solcher Berechnungen übernommen haben kann.

Die Vorbestimmung der Höhe der nächsten Applikationsdosis Dₙ wird vom Nasenapplikator 100 oder seinen zu Fig. 1 genannten Komponenten, etwa der Steuervorrichtung 9, gleich ob sie vom Gehäuse 2 des Nasenapplikators 100 umfasst sind oder extern hierzu liegen, festgelegt.

Der erste Vorbestimmungszeitpunkt tVₙ kann, wie auch der Betätigungszeitpunkt tBₙ dabei zu oder vor dem ersten Abgabezeitpunkt tAₙ liegen, er kann somit mit dem ersten Abgabezeitpunkt tAₙ zusammenfallen, jedenfalls im Rahmen des technisch Möglichen, so dass zwischen erstem Vorbestimmungszeitpunkt tVₙ und nächstem Abgabezeitpunkt tAₙ ausreichend Zeit zum tatsächlichen Vorbestimmen bzw. Berechnen der Höhe der nächsten Applikationsdosis Dₙ und Zeit zum Ansprechen der entsprechenden Vorrichtungen oder Komponenten, welchen die vorbestimmte Höhe vor Applikation per Signal oder auf andere Weise mitgeteilt werden muss, verbleibt.

Bei der Vorbestimmung der Höhe der nächsten Applikationsdosis Dₙ, welche ein Beispiel der variablen Dosis D ist, also gerade nicht oder nicht ausschließlich durch Arzt oder anderen berechtigten Personen, Hersteller oder dergleichen für den Nasenapplikator 100 unveränderbar voreingestellt ist, können aus dem Datenspeicher M auslesbare Daten und/oder Daten, welche von den in Fig. 1 genannten Komponenten des Nasenapplikators 100 ermittelt wurden, berücksichtigt werden.

Zu diesen Daten zählt vorzugsweise bereits ein vor dem erfolgreichen Betätigungszeitpunkt tBₙ gelegener Ausgangszeitpunkt tA₀, zu welchem dem Benutzer möglicherweise eine Ausgangsdosis D₀ noch vor Abgabe der ersten Applikationsdosis D₁ verabreicht worden sein kann, sei es mittels des erfindungsgemäßen Nasenapplikators 100, von medizinischem Personal, z. B. noch in einem Aufwachraum postoperativ (post-OP), usw. Weiter können diese Daten die Höhe dieser optionalen Ausgangsdosis D₀ umfassen.

Im vorliegenden Fall wird angenommen, dass eine Ausgangsdosis D₀ zu einem Ausgangszeitpunkt tA₀, an den Benutzer abgegeben wurde.

Zu diesen Daten zählt vorzugsweise jeder vor dem erfolgreichen Betätigungszeitpunkt tBₙ gelegener Ausgangszeitpunkt tA₀, tA₁, tA₂, tAₙ₋₁, zu welchem dem Benutzer bereits eine Ausgangs- oder Applikationsdosis D₀, D₁, D₂, Dₙ₋₁ verabreicht wurde oder er sich selbst eine Dosis verabreicht hatte. Alternativ oder ergänzend zählen auch die seitdem jeweils verstrichene Zeit und vorzugsweise auch die jeweilige Höhe der vorstehend genannten Dosen dazu.

Betrachtet man die geplante Abgabe der nächsten Applikationsdosis Dₙ, so zählen zu diesen Daten beispielsweise zumindest die Höhe der vorangegangenen Applikationsdosis Dₙ₋₁ sowie, optional, deren Abgabezeitpunkt tAₙ₋₁ oder die seitdem verstrichene Zeit.

Das Vorbestimmen, welches zum ersten Vorbestimmungszeitpunkt tVₙ erfolgt, ergibt somit die Höhe der nächsten Applikationsdosis Dₙ. Bestimmt wird die Höhe zum nächsten Vorbestimmungszeitpunkt tVₙ, der nach dem nächsten Betätigungszeitpunkt tBₙ liegt, und nicht durch Arzt oder anderen berechtigten Personen, Hersteller oder dergleichen voreingestellt ist, was auch für alle oder manche der weiteren hierin genannten Applikationsdosen gelten kann. Das Vorbestimmen zum nächsten Vorbestimmungszeitpunkt tVₙ erfolgt unter Berücksichtigung von aus dem Datenspeicher M ausgelesenen Daten.

Nach erfolgter Applikation der nächsten Applikationsdosis Dₙ wird, vorzugsweise zum Abgabezeitpunkt tAₙ der nächsten Applikation Dₙ, erneut eine Sperrdauer T_vainₙ angesetzt und/oder begonnen. Sie kann dieselbe Länge wie vorangegangene oder nachfolgende Sperrdauern haben, oder hiervon abweichen. Die Länge der jeweiligen Sperrdauer kann vorbestimmt sein und/oder z. B. vom Arzt oder anderen berechtigten Personen eingestellt worden sein. Beim Ansetzen der nächsten Sperrdauer kann einer Logik oder einem Algorithmus gefolgt werden, für welche(n) bei jedem Vorbestimmungszeitpunkt tVₙ mitberechnet werden kann.

Wie Fig. 2 zu entnehmen ist, kann sich das vorstehend zu dieser Figur Beschriebene wiederholen.

Beim Vorbestimmen der Höhe einer übernächsten Applikationsdosis Dₙ₊₁ können nun aber, optional, neben den Höhen und/oder den Abgabezeitpunkten der vorstehend genannten Applikationsdosen und ggf. auch der Ausgangsdosis D₀ dann erstmals auch die Höhe und/oder der Applikationszeitpunkt t_{An} der hierin als nächsten Applikationsdosis bezeichneten, dann aber bereits applizierten Applikationsdosis Dₙ berücksichtigt werden, welche zwischenzeitlich als Daten erfasst und ebenfalls im Datenspeicher M vorgehalten werden können.

Das Vorbestimmen der Höhe der übernächsten Applikationsdosis Dₙ₊₁, welches zum Vorbestimmungszeitpunkt tVₙ₊₁ erfolgt, kann daher nun auch die Länge der Zeitspanne, die zwischen dem übernächsten Abgabezeitpunkt tAₙ₊₁ und dem nächsten Abgabezeitpunkt tAₙ sowie die Längen der Zeitspannen, die zwischen dem übernächsten Abgabezeitpunkt tAₙ₊₁ und dem Ausgangszeitpunkt tA₀ oder früheren Abgabezeitpunkten tA₁, tA₂ liegen, berücksichtigen.

**Fig. 3** zeigt schematisch und exemplarisch die applizierten Einzeldosen D₀, D₁, D₂, ..., Dₙ₋₁, Dₙ, Dₙ₊₁, ... und die über den Verlauf der Zeit t kumulierten Dosen der Substanz S, deren Höhen und/oder Abgabezeitpunkte tA₀, tA₁, tA₂, ..., tAₙ₋₁, tAₙ, tAₙ₊₁, ...usw. in die Vorbestimmung jeweils späterer Applikationsdosen einfließen können.

Die Zeitachse t sowie ihre Skalierung entsprechen dabei der in Fig. 2 verwendeten oder sind gedanklich als identisch zu anzunehmen. Der Zeitpunkt tA₁ und der Zeitpunkt tA₂ entsprechen somit den in Fig. 2 gezeigten absoluten Zeitpunkten tA₁ und tA₂.

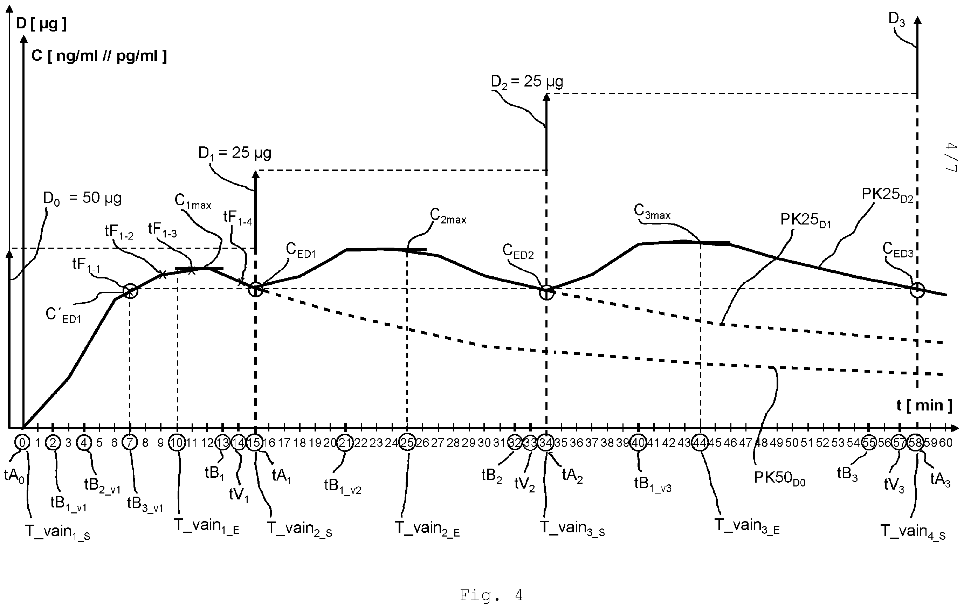

**Fig. 4** beschreibt schematisch und exemplarisch die hierin auch als "Titrationsphase" (welche eine Bestimmungs- oder Findungsphase ist) bezeichnete Phase in der Versorgung des Benutzers mit der Substanz S, in welcher die, den vom Benutzer gewünschten, therapeutischen Effekt bewirkende Zielkonzentration bestimmt wird. Auf ihr kann anschließend die Höhe folgender Applikationsdosen bestimmt werden und/oder zeitgesteuert ein individueller, dosis- bzw. konzentrationsbasierter Behandlungsplan aufgestellt werden.

Kurven, wie sie in Fig. 4 oder anderer Stelle hierin gezeigt sind, können basierend auf z. B. den Ausführungen von Dubois, A., Bertrand, J., & Mentre, F. (2011) in "Mathematical expressions of the pharmacokinetic and pharmacodynamic models implemented in the PFIM software." UMR738, INSERM, Paris Diderot University, erstellt werden. Die Steuervorrichtung kann zum Erstellen solcher Kurven und/oder zum Durchführen der hierfür erforderlichen Berechnungen programmiert sein. Der Inhalt jener Publikation wird hiermit per Verweis zum Gegenstand auch der vorliegenden Offenbarung gemacht

Fig. 4 zeigt schematisch und exemplarisch das Ergebnis einer Vorbestimmung der Höhe der verschiedenen Applikationsdosen D₁, D₂ und weiterer, die jeweils ein Beispiel für die jeweils nächste Applikationsdosis Dₙ, wie hierin verwendet sein können.

Zur Vorbestimmung der jeweiligen Applikationsdosis D₁, D₂, und weiterer wird in der hier diskutierten Ausführungsform auf Daten aus dem Datenspeicher M zurückgegriffen, welche pharmakologische, pharmakodynamische, pharmakometrische und/oder pharmakokinetische Daten umfassen. Die Daten können Modelle, insbesondere PK-Modelle, sein, ebenso PK-Kurven. Sie sind vorzugsweise patientenindividuell.

Zu diesen pharmakologischen, pharmakodynamischen, pharmakometrischen und/oder pharmakokinetischen Daten oder Modellen zählen im Beispiel der Fig. 4 hierin als PK-Kurven bezeichnete zeitliche Verläufe von Konzentrationen der Substanz S im Körper des Benutzers nach den jeweils applizierten Dosen. Die abgebildeten Kurven spiegeln den zeitlichen Verlauf der Konzentration von einer konkreten Substanz S, welche aus der applizierten Dosis resultieren, und der Höhe der applizierten Dosen wider. Die Kurven spiegeln zudem die Prozesse der Resorption, Verteilung, Metabolisierung und Elimination im Körper wider. Derartige Pharmakokinetik Modelle folgen statistischen Verteilungen z. B. unter Berücksichtigung von mit Mittelwert und Standardabweichung und sind zumeist patientenindividuell, d. h. sie können von Patient A zu Patient B unterschiedlich verlaufen, weshalb sie auch für den unterschiedlichen Benutzer oder Benutzerkollektive entsprechend unterschiedliche statistischen Verteilungen im Datenspeicher M gespeichert sein können. Da der Verlauf solcher PK-Kurven vom Applikationsweg und von der Dosis abhängt, sind die beiden in Fig. 4 mit Bezugszeichen versehenen PK-Kurven einmal als PK50 für die intranasale Gabe von 50 µg Fentanyl und einmal als PK25 für die intranasale Gabe von 25 µg Fentanyl bezeichnet.

Zu pharmakologischen, pharmakodynamischen, pharmakometrischen und/oder pharmakokinetischen Daten, wie sie im Datenspeicher M gespeichert sein können, zählen im Beispiel der Fig. 4 optional auch Angaben zur Substanz S wie tₘₐₓ, welche den Zeitpunkt angeben können, zu welchem nach Applikation einer Dosis D die höchste Konzentration C(t) im Körper, etwa im Blut, vorliegt. Die Dauer von tₘₐₓ kann, zumindest anfänglich oder im Nachgang zu einer ersten Dosis, hier der Ausgangsdosis D₀, als Sperrdauer verwendet werden. Ebenso kann die Sperrdauer an tₘₐₓ angelehnt sein, indem die Sperrdauer z. B. nur 90% von tₘₐₓ beträgt, oder um eine Anzahl von Minuten kürzer oder länger als tₘₐₓ kann bestimmt wird, usw. Ebenso sind Werte über 100% von tₘₐₓ möglich, z. B. 110% von tₘₐₓ, was also nach tₘₐₓ liegt, wenn man einen Delay miteinberechnen möchte, in dem die verabreichte Substanz vom Blut an den Wirkort gelangt, oder wenn tₘₐₓ 13 Minuten beträgt, die Sperrdauer aus praktikablen Gründen jedoch auf z. B. 10 Minuten festgelegt werden soll.

Die elektronische Steuervorrichtung 9 kann also programmiert sein, um auch solche im Datenspeicher M gespeicherten Daten die Substanz S betreffend auszulesen und diese beim Vorbestimmen der nächsten Applikationsdosis Dₙ der Substanz S zu berücksichtigen.

Wie in Fig. 4 schematisch und exemplarisch dargestellt wurde dem Benutzer zum Ausgangszeitpunkt tA₀ zunächst eine Ausgangsdosis D₀ von 50 µg intranasalem Fentanyl verabreicht. Die Konzentration flutet bis zu einem ersten Maximum C₁ₘₐₓ an, weshalb für diesen Zeitpunkt auch das Sperrdauerende T_vain_{1_E} der ersten Sperrdauer T_vain₁ festgelegt ist, deren Sperrdauerbeginn T_vain_{1_s} zu tA₀ liegt.

Würde dem Benutzer ausschließlich die Ausgangsdosis D₀ appliziert werden, so würde die Konzentration C(t) über die Zeit abnehmen und sich bei Annahme der in Fig. 4 gestrichelt gezeigten PK-Kurve PK50 nach etwa 30 Minuten halbiert haben.

Da eine solche Halbierung jedoch offensichtlich nicht den Bedürfnissen des Benutzers entspricht, der zum Betätigungszeitpunkt tB₁ die Abrufvorrichtung 1 betätigt hat, wird ihm zum ersten Abgabezeitpunkt tA₁ eine erste Applikationsdosis D₁ in Höhe von beispielsweise 25 µg Fentanyl verabreicht. Das zum Vorbestimmungszeitpunkt tᵥ₁ erfolgte Vorbestimmen dieser Höhe hat ergeben, dass 25 µg Fentanyl angesichts des Verlaufs der PK50-Kurve genügen, um im Wechselspiel zwischen abnehmender Wirkung oder Konzentration der Ausgangsdosis D₀ bei gleichzeitig ansteigender Wirkung oder Konzentration der ersten Applikationsdosis D₁ eine Konzentration erzielt werden kann, die einerseits nicht über der anfangs als genügend eingestuften Ausgangsdosis D₀ von 50 µg Fentanyl und andererseits nicht unter einer gewünschten therapeutischen Effekt bewirkenden Konzentration C_{ED} von etwa 45 µg Fentanyl liegt.

Die den vom Benutzer gewünschten therapeutischen Effekt bewirkende Konzentration C_{ED} von etwa 45 µg Fentanyl erkennt die Steuervorrichtung 9 darin, dass der Benutzer ab dem Betätigungszeitpunkt tB₁, der nur kurz vor dem ersten Abgabezeitpunkt tA₁ liegt, den gewünschten therapeutischen Effekt offensichtlich vermisst hat, was ihn zum Betätigen der Abrufvorrichtung 1 gebracht hat.

Wie Fig. 4 zeigt, versucht der Benutzer während der Sperrdauer T_vain₁ zwei weitere Applikationen anzufordern (zu den Zeitpunkten tB_{1_v1} und tB_{2_v1}), da der gewünschte therapeutische Effekt anscheinend nicht oder eben noch nicht eingetreten ist. Diese Applikationen werden verweigert, da die Konzentration der Ausganzdosis D₀ noch nicht ihr Maximum erreicht hat, jedenfalls aber die Sperrdauer T_vain₁ noch nicht beendet ist. Ihr Maximum wird sie erst gegen Ende der Sperrdauer T_vain_{1_E} erreichen. Da man nun erkennen kann, dass der Betätigungszeitpunkt tB₁ erst einige Zeit nach dem Ende der Sperrdauer T_vain_{1_E} erfolgt, kann das System anhand der PK-Kurve eine Konzentration ableiten in der, der gewünschte therapeutische Effekt auftritt. In diesem Fall ergibt eine Ausgangsdosis D₀ von 50 µg Fentanyl ein therapeutisches Zeitfenster von 8 Minuten (von Minute 7 bis Minute 15).

Man kann in Fig. 4 auch erkennen, dass die Konzentration für den gewünschten therapeutischen Effekt bereits bei Minute 7 erreicht ist. Dies kann man daran erkennen, dass ab diesen Zeitpunkt keine weiteren vergeblichen Betätigungszeitpunkte tB_{m_vn} während der Sperrdauer T_vain₁ mehr stattfinden. Wie man sehen kann hat die Maximalkonzentration der Ausgangsdosis D₀ jedoch noch nicht ihr Maximum erreicht. Man kann die Konzentration, welche bei dem letzten vergeblichen Betätigungszeitpunkt tB_{3_v1} vorherrscht, zur Verifikation benutzen, indem man diese vorherrschende Konzentration mit dem erwarteten Betätigungszeitpunkt nach T_vain_{1_E} abgleicht.

Die den gewünschten therapeutischen Effekt bewirkende Konzentration C_{ED} ab dem ersten Betätigungszeitpunkt tB₁ kennend, kann die Steuervorrichtung 9 die Höhe der ersten Applikationsdosis D₁ nun selbständig vorbestimmen. Sie kann eine Höhe von 25 µg Fentanyl vorbestimmen, im Wissen, dass das Abklingen der Konzentration C(t) aufgrund der Ausgangsdosis D₀ und das gleichzeitige Anfluten der Konzentration C(t) aufgrund der ersten Applikationsdosis D₁ zu einem zweiten Maximum C₂ₘₐₓ führen wird, welches nicht über einem therapeutisch vertretbaren Maximum liegen wird.

Anhand der Pharmakokinetischen Modelle (PK-Kurven) kann die Steuervorrichtung 9 optional eine Zeit berechnen, in der die Konzentration der Dosis D₁ unter den gewünschten therapeutischen Effekt fallen wird. In Fig. 4 findet dies nach weiteren 19 Minuten oder 34 Minuten seit tA₀ statt.

Ist aus vorstehenden Überlegungen die den gewünschten therapeutischen Effekt bewirkende Konzentration C_{ED} und/oder das jeweils nächste Maximum Cₙₘₐₓ bekannt, so kann aus der Kenntnis des Zeitpunkts, zu welchem die Konzentration C_{ED} oder das jeweils nächste Maximum Cₙₘₐₓ eintreten oder erreicht sein wird, die jeweils nächste Sperrdauer berechnet werden. So kann die Sperrdauer T_vain₁ beispielsweise dem Datenspeicher M im Zusammenhang mit der Höhe der Ausgangsdosis D₀ entnommen werden. Das Ende T_vain_{2_E} der folgenden, zweiten Sperrdauer T_vain₂ kann hingegen aus der Kenntnis des Zeitpunkts von C₂ₘₐₓ gewonnen werden, beiden können identisch miteinander sein. Alternativ kann optional zum Zeitpunkt, tA₁, zu welchem die Konzentration C_{ED} erzielt ist, bereits der Zeitpunkt errechnet werden, zu welchem die Konzentration C(t) erneut auf die Konzentration C_{ED} abgesunken sein wird. Die Hälfte der Differenz zwischen diesen beiden Zeitpunkten kann, ebenso wie ein anderer Anteil hiervon (z. B. 40 %, 35 %, usw.), als Dauer der jeweils nächsten Sperrdauer festgesetzt werden.

Alternative Vorgehensweisen sind ebenso umfasst. So könnte generell auch die errechnete Zeit zur nächsten Unterschreitung von C_{ED} als Sperrdauer gewählt werden, oder kurz davor. Dies wäre in Fig. 4 bei 58 Minuten ab tA₀ der Fall. Also könnte die Steuervorrichtung 9 bei tA₂, hier also tV₂, die Sperrdauer zur nächste Zielkonzentration C_{ED} (bei Minute 58 in Fig. 4) setzen.

Die Höhen der weiteren Applikationsdosen wie z. B. D₂ (in diesen beispielhaften Fall jeweils 25 µg Fentanyl) würden ähnlichen Überlegungen folgend wie zu D₁ vorbestimmt: sie wurden unter Berücksichtigung der Höhe von bereits verabreichten Applikationsdosen sowie der seit ihrer Applikation vergangenen Zeit festgelegt; für die zweite Applikationsdosis D₂ wurde dabei auf eine Restkonzentration, welche auf die Ausgangsdosis D₀ und die erste Applikationsdosis D₁ zurückzuführen ist, abgestellt, für die dritte, hier nicht gezeigte Applikationsdosis würde auf die Restkonzentration, welche auf die Ausgangsdosis D₀, die erste Applikationsdosis D₁ und die zweite Applikationsdosis D₂ zurückzuführen ist, abgestellt bzw. abgestellt werden. Eine Restdosis ist dabei mitbestimmt durch die Höhe der jeweils vorangegangenen Applikation sowie der seit ihrer Applikation vergangenen Zeit.

Die Höhe der nächsten Applikationsdosis Dₙ kann derart vorbestimmt werden, dass die Konzentration im Körper, insbesondere im Blut, des Benutzers erst nach Ablauf einer vorbestimmten Zeitdauer, einstellbar z. B. durch den Arzt oder andere berechtigten Personen, beginnend ab dem Abgabezeitpunkt tAₙ der nächsten Applikationsdosis Dₙ, erneut auf die Zielkonzentration C_{ED} absinken wird.

Wie Fig. 4 zeigt, betätigt der Benutzer zu Betätigungszeitpunkten tB_{1_v1}, tB_{2_v1} und tB_{3_v1} also während der Sperrdauer T_vain₁, die Abrufvorrichtung 1. Da diese drei Betätigungen aber innerhalb der Sperrdauer T_vain₁ liegen, kann der Benutzer hiermit keine Applikationsdosis D erwirken. Dennoch registriert die Steuervorrichtung 9 jedoch die drei vergeblichen Betätigungsversuche und ihre Betätigungszeitpunkte. Sie liegen im Beispiel der Fig. 4 bei Minute 2 (tB_{1_v1}) bzw. bei Minute 4 (tB_{2_v1}) bzw. bei Minute 7 (tB_{3_v1}), und können optional im Datenspeicher M gespeichert werden. Werden die vergeblichen Betätigungsversuche darauf analysiert, etwa in welchem zeitlichen Abstand sie zueinanderstehen, wann sie aufhören usw., so kann in erster aber ausreichender Näherung die vom Benutzer gewünschte, den beabsichtigten therapeutischen Effekt bewirkende Konzentration C_{ED} erkannt oder festgelegt werden.

In Fig. 4 sind Feedbackzeitpunkte tF_{1_1}, tF_{1_2}, tF_{1_3} und tF_{1_4} angegeben. Sie stehen für Momente, in welchen der Benutzer eine Feedbackvorrichtung 8 betätigt, um mittels Feedbacks anzudeuten, dass mit der von ihm verspürten Wirkung der zum Feedbackzeitpunkt jeweils vorliegenden Konzentration zufrieden oder nicht zufrieden ist. Er kann dem Nasenapplikator somit z. B. signalisieren, dass er derzeit schmerzfrei ist, dass er keine höhere Konzentration im Körper bevorzugen würde, usw.

In Fig. 4 sieht man bei der PK50_{D0} Kurve, dass man ab ca. Minute 45 eine "Gerade hat", sprich das Gefälle konstant bleibt (pseudo-equilibrium "Lambda").

Die Eliminationshalbzeit ist nun die Zeit in der sich, die vorherrschende Konzentration bei Minute 45, halbiert.

Laut Literatur liegt die Eliminationshalbzeit von Fentanyl zwischen 3 bis 12 Stunden, abhängig von der Gewöhnung.

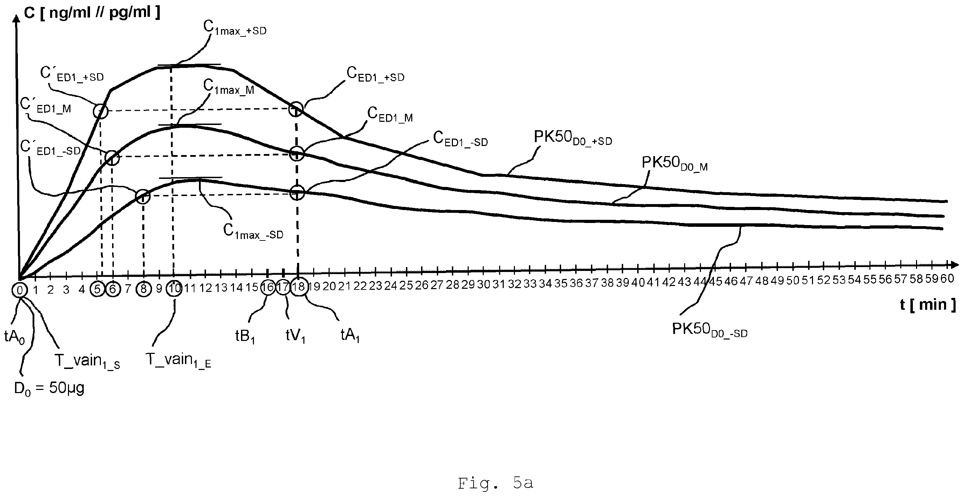

**Fig. 5a** zeigt exemplarisch und schematisch dargestellt mittels dreier exemplarischer PK-Kurven PK50_{D0_+SD}, PK50_{D0_M} und PK50_{D0_-SD} die statistische Verteilung (Mittelwert und Standardabweichung) der möglichen Konzentrationsverläufe im Körper des Benutzers nach Gabe von 50 µg intranasalem Fentanyl, welche an einer ausreichend großen Population gewonnen wurden. Es mag mehr als nur drei zwischen den dargestellten Grenzbereichen liegende Kurven geben, d. h. die entsprechende Untersuchung des Kollektivs/der Population könnte zu mehr als nur drei voneinander unterscheidbare PK-Kurven geführt haben. Sie alle, oder die ihnen zugrundeliegenden Modelle können im Datenspeicher M gespeichert sein und/oder von der Steuervorrichtung 9 berücksichtig werden. Allein aus Gründen der Vereinfachung werden hier nur drei PK-Kurven gezeigt und diskutiert.

Allgemein gilt, dass gleichhohe Dosen einer Substanz patientenindividuell zu unterschiedlichen Konzentrationen C(t) im Körper führen können. Dies wird durch verschiedene Faktoren beeinflusst, wie der Pharmakogenetik, die Intraindividualität, das Alter, das Gewicht, das Geschlecht, die Vormedikation, die Vorerkrankungen, etc. Ferner können verschiedene Nasenanatomien, der Nasenzustand, der Applikationswinkel, wie tief man den Nasenaufsatz einführt und wie man das, manuell ausgelöste, Nasenspray bedient eine Rolle spielen für den Verlauf der jeweiligen PK-Kurve, welcher die Konzentration C(t) über die Zeit im Körper beeinflusst.

Durch Verwendung von Populations-Pharmakokinetik Modellen wie der in Fig. 5a gezeigten PK-Kurven PK50_{D0_+SD} (Mittelwert zuzüglich einer Standardabweichung), PK50_{D0_M} (Mittelwert) und PK50_{D0_-SD} (Mittelwert abzüglich einer Standardabweichung) lassen sich solche Unterschiede abbilden.

Wie man erkennt führt dieselbe Dosis von 50 µg intranasalem Fentanyl für diese drei exemplarisch ausgewählten Kurven zu drei unterschiedlichen Konzentrationsmaxima C_{1max_-SD}, C_{1max_M} und C_{1max_+SD} gekennzeichnet. Eine Vielzahl weiterer Kurven ließe sich ebenfalls darstellen. Sie hätten wiederum andere Konzentrationsmaxima. Der Zeitpunkt bis zur maximalen Konzentration unterscheidet sich bei den drei gezeigten Kurven und auch bei jene nicht dargestellten aus der Vielzahl von Kurven jedoch nicht oder wenn, nur marginal. Das sich am Zeitpunkt, zu welchem das Konzentrationsmaximum C_{1max_-SD}, C_{1max_M} und C_{1max_+SD} erreicht wird, orientierende Ende T_vain_{1_E} der Sperrdauer T_vain₁ endet daher für jede der drei Kurven der Fig. 5a zum selben Zeitpunkt.

Nimmt man an, dass zum Zeitpunkt tA₀ die Ausgangsdosis D₀ (D₀=50 µg) appliziert wird, so kann die Steuervorrichtung 9 anhand der Populations-Pharmakokinetik-Modelle die Maximalkonzentrationen C_{1max_-SD}, C_{1max_M} und C_{1max_+SD} (und viele weitere) berechnen. Da die Zeit bis zum Erreichen dieser Maximalkonzentrationen aber bei allen Varianten gleich ist setzt Steuervorrichtung 9 die erste Sperrdauer (T_vain₁, siehe Fig. 5a) für alle drei PK-Kurven gleich der Zeit bis zum Erreichen der Maximalkonzentration, welcher in diesem Fall exemplarisch auf 10 Minuten festgelegt ist. Das Erreichen der Maximalkonzentration kann in einem Bereich stattfinden. In dem vorliegenden Fall von intranasalen Fentanyl liegt dieser zwischen 10 und 12,8 Minuten. Da der Konzentrationsanstieg in dem Bereich von 10 bis 12,8 Minuten nur sehr gering ist, rundet man die Zeit auf ein praktikables Maß wie hier z. B. 10 Minuten. Man könnte selbstverständlich auch 11, 12 oder 13 Minuten wählen. Somit wird, wenn bei t_{A0} die Ausgangsdosis D₀ (D₀=50 µg) appliziert wird, die daraus jeweils resultierenden Maximalkonzentration berechnet, die Anflutzeit bis zum Erreichen der Maximalkonzentration C_{1max_}-_{SD}, C_{1max_M} oder C_{1max_+SD} berechnet, die Länge der Sperrdauer T_vain₁ (hier 10 min) berechnet und T_vain_{1_S} und T_vain_{1_E} gesetzt.

Entsprechend der unterschiedlichen Verläufe der drei PK-Kurven PK50_{D0_+SD}, PK50_{D0_M} und PK50_{D0_-SD} würde man zum ersten Betätigungszeitpunkt tB₁, zu welchem Konzentration unter jene den Benutzer erwünschten therapeutisch Effekt bewirkende, Konzentration fällt, zu welchem der Patient auf Anforderung hin (Zeitpunkte die Betätigung durch den Patienten, das Vorbestimmen usw. betreffend sind hier aus Gründen der Vereinfachung nicht dargestellt oder fallen auf den ersten Abgabezeitpunkt, was in der Praxis tatsächlich auch der Fall sein kann) die erste Applikationsdosis D₁ erhalten würde, eine jeweils unterschiedliche Konzentration C_{ED1_+SD}, C_{ED1_M}, C_{ED1_-SD} errechnen (oder mit Blick auf die wie in Fig. 5a geplotteten PK-Kurven ablesen), welche jeweils den erwünschten therapeutischen Effekt bewirkt. Diese Konzentration ist hierin auch als Zielkonzentration oder C_{ED} bezeichnet. Ihr Wert beruht u. a. auf der Pharmakodynamik.

Es sei angemerkt, dass der Betätigungszeitpunkt tBₙ dem Vorbestimmungszeitpunkt tVₙ und/oder dem Abgabezeitpunkt tA₁ entsprechen kann. Es kann also z. B. gelten: tB₁=tV₁=tA₁.

Fig. 5a ist zu entnehmen, dass die Konzentration C_{ED1_+SD}, C_{ED1_M}, C_{ED1_-SD} bereits vor dem Abgabezeitpunkt t_{A1} als C'_{ED1_+SD}, C'_{ED1_M}, C'_{ED1_-SD} bei 5, 6 bzw. 8 min vorgelegen hatten. Dies kann auch mittels der ausbleibenden tB_{_v}'s abgeleitet werden.

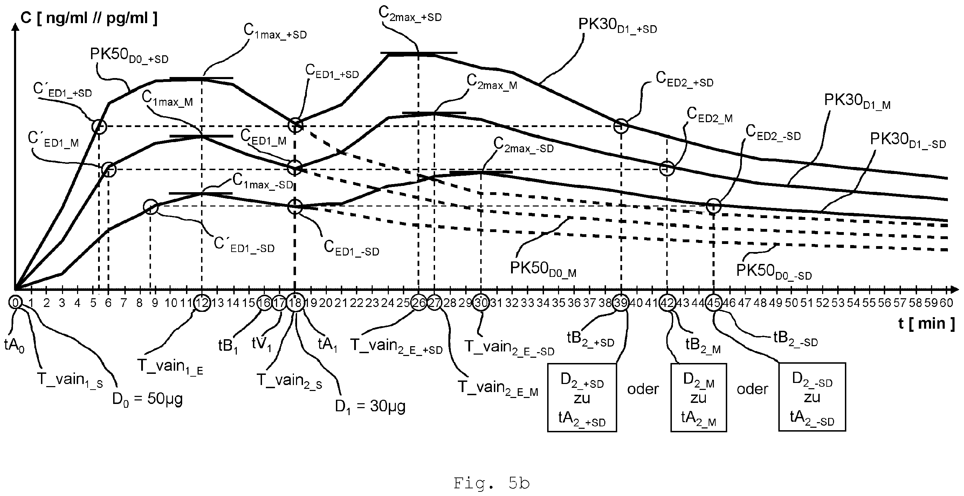

Vorstehende Ausführungen sind Basis für eine Ausführungsform, welche in Bezug auf Fig. 5b diskutiert ist, und in welcher der Nasenapplikator 100 das Betätigungsverhalten des Benutzers auf eine konkrete Weise in Betracht zieht.

Wenn man in Fig. 5b von t_{A0} ausgehend eine Linie (Gerade durch zwei Punkte) zu den entsprechenden Konzentrationen C_{1max_-SD}, C_{1max_M} und C_{1max_+SD} zieht, so erhält man drei Geraden von jeweils unterschiedlicher Steigung. Wenn man jetzt anhand der Zeitabstände den Konzentrationsanstieg betrachtet, so könnte man bereits eine Einschätzung vornehmen, welche Kurve dem Benutzer möglicherweise zuzuordnen ist, denn die PK50_{D0_+SD} Kurve hat im gleichen Zeit Intervall den größten Konzentrationsanstieg und die PK50_{D0_-SD} Kurve den geringsten. Da der gewünschte therapeutische Effekt von der Konzentration abhängt, kann man eine erste Einschätzung vornehmen.

**Fig. 5b** zeigt schematisch das exemplarische Vorgehen beim Ermitteln, Auswählen und/oder Festlegen einer individuellen PK-Kurve PK_{Ind}, welche den Wirkstoff-Konzentrations-Verlauf von 50 µg intranasalem Fentanyl eines konkreten, individuellen Benutzers wiedergibt. Anzumerken ist an dieser Stelle, dass das Ziel beim Ermitteln eines individuellen zeitlichen Konzentrationsverlaufs, wie hierin verwendet, nicht immer eine individuelle PK-Kurve des Benutzers sein muss. Es kann erfindungsgemäß auch genügen, wenn man sich z. B. an der statistischen Mittelwert-Kurve PK50_{D0_M} orientiert oder sich bzw. Grenzbereich-Kurven wie z. B. die Kurve "Mittelwert plus Standardabweichung" oder die Kurve "Mittelwert minus Standardabweichung" oder andere nähert und weiß, in welchem Bereich der Benutzer sich bewegt.

Fig. 5b umfasst die Darstellung der Fig. 5a, ergänzt diese jedoch ab dem ersten Abgabezeitpunkt tA₁ um Gabe einer ersten Applikationsdosis D₁ in Höhe von exemplarisch 30 µg Fentanyl.

Ab dem ersten Abgabezeitpunkt tA₁ werden jeweils die vor diesem Zeitpunkt angenommenen PK-Kurven PK30_{D1_+SD}, PK30_{D1_M} und PK30_{D1-SD} angelegt. Ausgehend von der in Fig. 5b ermittelten Zielkonzentrationen C_{ED1_+SD}, C_{ED1_M}, C_{ED1_-SD} ermittelt die Steuervorrichtung 9 bzw. der Algorithmus eine Vielzahl von möglichen Dosen und davon abgeleiteten Konzentrationen C(t), welche eine Beurteilung/Kategorisierung erlauben, sprich wann die erwarteten Zeitpunkte der nächsten Betätigung zum Abrufen einer weiteren Applikationsdosis eintreten müssten. In diesem Fall hat das System 30 µg festgelegt, und das System kumuliert die vorhergehenden Kurven und projiziert ausgehend von den ermittelten Zielkonzentrationen C_{ED1_+SD}, C_{ED1_M}, C_{ED1_-SD} die jeweiligen Kurvenverläufe in die Zukunft. Es wird ermittelt, zu welchem Zeitpunkt die Zielkonzentrationen C_{ED2_+SD}, C_{ED2_M}, C_{ED2_-SD} rechnerisch wieder unterschritten werden sollten. Im Beispiel der Fig. 5b sind sie mit tB_{2_+SD}, tB_{2_M} und tB_{2_-SD} gekennzeichnet. Anhand dieser Überprüfung kann die Steuervorrichtung 9 feststellen, in welchem Bereich oder auf welche Kurve der Benutzer sich befindet. Über diese und weitere Iterationen kann der Benutzer besser eingestuft werden.

Drei mögliche Fälle werden nachfolgend betrachtet:
Fall 1: Trifft auf die Konzentrationsveränderung im Körper des Benutzers die Populations-Pharmakokinetik Kurve PK30_{D1_M} (Mittelwert) zu, so liegt er gute 24 Minuten oberhalb der erwünschten therapeutischen Effekt-Konzentration, nämlich von Minute 18 bis Minute 42. Er würde erst bei Minute 42 zum Zeitpunkt tB_{2_M} eine Betätigung abgeben, was nach einer Vorbestimmung schließlich zu einer erneuten Abgabe einer (zweiten) von ihm angeforderte Applikationsdosis D_{2_M} zum Applikationszeitpunkt tA_{2_M} führt.
Fall 2: Trifft auf die Konzentrationsveränderung im Körper des Benutzers die Populations-Pharmakokinetik Kurve PK30_{D1+SD} (Mittelwert zuzüglich einer Standardabweichung) zu, so liegt er 21 Minuten oberhalb der erwünschten therapeutischen Effekt-Konzentration, nämlich von Minute 18 bis Minute 39. Er würde erst bei Minute 39 zum Zeitpunkt tB_{2_+SD} eine Betätigung abgeben, was nach einer Vorbestimmung schließlich zu einer erneuten Abgabe einer (zweiten) von ihm angeforderte Applikationsdosis D_{2_+SD} zum Applikationszeitpunkt tA_{2_+SD} führt.
Fall 3: Trifft auf die Konzentrationsveränderung im Körper des Benutzers die Populations-Pharmakokinetik Kurve PK30_{D1_-SD} (Mittelwert abzüglich einer Standardabweichung) zu, so liegt er gute 27 Minuten oberhalb der erwünschten therapeutischen Effekt-Konzentration, nämlich von Minute 18 bis Minute 45. Er würde erst bei Minute 45 zum Zeitpunkt tB_{2_-SD} eine Betätigung abgeben, was nach einer Vorbestimmung schließlich zu einer erneuten Abgabe einer (zweiten) von ihm angeforderten Applikationsdosis D_{2_-SD} zum Applikationszeitpunkt tA_{2_-SD} führt.

Die Steuervorrichtung 9 erkennt anhand der Betätigungszeitpunkte tB_{2_+SD}, tB_{2_M}, bzw. tB_{2_-SD} also, ob die individuelle PK-Kurve des Benutzers nun die PK-Kurve PK30_{D1_+SD}, die PK-Kurve PK30_{D1_M} oder die PK-Kurve PK30_{D1_-SD} (oder eine der unzähligen weiteren PK-Kurven, welche die Steuervorrichtung 9 ebenfalls betrachten kann) ist. Liegt der Abrufzeitpunkt bei tA_{2_+SD}, so trifft die PK-Kurve PK30_{D1+SD} zu. Liegt der Abrufzeitpunkt bei tA_{2_M}, so trifft die PK-Kurve PK30_{D1_M} zu. Liegt der Abrufzeitpunkt bei tA_{2_-SD}, so trifft die PK-Kurve PK30_{D1_-SD} zu.

Ist eine erste direkte Zuordnung zwischen einem zeitlichen Verlauf und den betrachteten Benutzer nicht möglich, so nähert sich die Steuervorrichtung 9 mittels Ausschlussverfahrens dennoch dem Ziel und kategorisiert/bestimmt für den konkreten Benutzer anhand seines Betätigungsverhaltens, welcher zeitliche Konzentrationsverlauf oder welche Kurve ihn am besten widerspiegelt. Nach der "Titrationsphase" (welche eine Bestimmungs- oder Findungsphase ist), welche in Fig. 5b gezeigt und sich über die in Fig. 5b möglicherweise noch hinausgehende Zeiten erstrecken kann, kann eine Kategorisierung bzw. Einordnung des Benutzers erfolgen. Dem anschließend kann die Höhe folgender Applikationsdosen bestimmt werden und/oder zeitgesteuert ein individueller, dosis- bzw. konzentrationsbasierter Behandlungsplan aufgestellt werden.

Folgt man vorstehendem Vorgehen, so kann durch Postulieren einer oder beliebig vieler PK-Kurven als mögliche, individuelle PK-Kurve des Benutzers a priori oder als eine Vielzahl von Hypothesen (wie: auf den Benutzer trifft die PK-Kurve PK30_{D1_+SD} zu, oder auf den Benutzer trifft die PK-Kurve PK30_{D1_M} zu, oder auf den Benutzer trifft die PK-Kurve PK30_{D1_-SD} zu) bereits im Anschluss an eine erste Ausgangs- oder Applikationsdosis D₀, D₁, D₂, usw. oder an eine nächste Applikationsdosis Dₙ ganz allgemein die Richtigkeit der zutreffenden der aufgestellten Hypothesen überprüft werden. Dabei gelangt man zu einem individuelles pharmakokinetisches Modell für den Benutzer, das als Grundlage für zukünftige Applikationsdosen genutzt werden kann. Im Beispiel der Fig. 5b wird die PK-Kurve PK30_{D1_+SD} bei Betätigungszeitpunkt tB_{2_+SD} als PK-Kurve PK_{Ind} festgelegt und bei künftigen Vorbestimmungen von Applikationsdosen den erforderlichen Berechnungen zugrunde gelegt.

Eine Überprüfung darauf, dass das so bestimmte, individuelle pharmakokinetische Modell tatsächlich zutrifft, kann im Laufe der weiteren Behandlung und im Zusammenhang mit weiteren Applikationsdosen D_{n,} Dₙ₊₁, usw. jederzeit optional erfolgen.

**Fig. 6** zeigt einen Nasenapplikator 100 einer weiteren exemplarischen Ausführungsform. Der Nasenapplikator 100 ist in Fig. 6 in Schnittdarstellung oder im - teils angedeuteten - Teilschnitt gezeigt.

Mittels der Abrufvorrichtung 1 kann der Benutzer eine nächste Applikationsdosis Dₙ der Substanz S abrufen. Nachstehend wird auf jene Komponenten eingegangen, welche nicht bereits mit Bezug auf Fig. 1 erläutert wurden.

So weist der Nasenapplikator 100 einen Abgabedosierer 3 auf, welcher eine Dosierung der abzugebenden Applikationsdosis vornimmt, also die Menge bestimmt. Der Abgabedosierer ist hier exemplarisch als eine Bestückungsvorrichtung 15 ausgestaltet, welche die Menge der auszubringenden Applikationsdosis festlegt, indem sie bestimmt, wie groß diese Menge jeweils sein wird. Zum Absondern der gewünschten Applikationsdosis kann die Bestückungsvorrichtung 15 wahlweise weiter oder weniger weit - von einem Nasenaufsatz 4 aus betrachtet - entlang des Doppelpfeils zurückgezogen werden, etwa automatisch oder per Hand.

Eine Ausbringvorrichtung 17 zum - hier: aktiven - Ausbringen der jeweils abgerufenen Applikationsdosis über die Verbindungsstelle 4a oder den Nasenaufsatz 4 ist in Fig. 6 exemplarisch als eine Kombination aus oder mit einem Motor, hier z. B. ein Elektromotor 19, und einer Spindel 21 vorgesehen.

Elektromotoren, wie sie hierin genannt sind, können z. B. bürstenlos sein oder eine Bürste aufweisen. Sie können z. B. als Innenläufer, Außenläufer und/oder nutenlose Motoren ausgestaltet sein. Es kann ein Encoder integriert sein, ein solcher kann extern angeordnet sein, selbiges gilt für einen Controller des Motors.

Der Motor kann in manchen Ausführungsformen ein Schrittmotor sein wie z. B. Hybrid-Schrittmotor, ein Flachmotor, ein Hohlwellenmotor, ein Servomotor, ein Asynchronmotor, ein Synchronmotor oder ein Wanderwellenmotor oder dergleichen.

Der Motor kann in einigen Ausführungsformen mit einer open und/oder closed loop-Steuerung angesteuert werden und/oder mittels eines Encoders und/oder eines Controllers. Encoder können z. B. optische Durchlichtencoder oder reflektive Encoder sein, magnetische Encoder oder dergleichen.

Der Motor kann über die Stromaufnahme gesteuert sein oder werden. Wenn der Motor z. B. auf eine Endlage (oben oder unten) zu- oder anfährt, steigt die Stromaufnahme des Motors z. B. von 0,5 A auf 1 A. Dies bedeutet, dass eine Endlage oder ein Anschlag erreicht worden ist. Somit kann man mit diesem Messwert auch den Motor ansteuern, ihm also signalisieren, wann er z. B. abschalten soll.

Zur Steuerung des Nasenapplikators oder dessen Motor kann optional ein Wegsensor, ein Durchflusssensor, Endschalter (Mikroschalter) genutzt werden. Ergänzend oder alternativ ist eine zeitliche Steuerung (z. B. Bestromung über eine vorbestimmte Zeitdauer) ist ebenfalls von der vorliegenden Erfindung umfasst.

Der Motor, oder die Spindelkombination, können in bestimmten Ausführungsformen eine Bremse z. B. eine Magnetbremse, Wirbelstrom, Federdruckbremse, einen Endschalter am oberen und/oder unteren Totpunkt bzw. Endpunkt des Hubs, usw. aufweisen.

Der Motor oder die Motor-Spindelkombination können alternativ oder ergänzend einen Dämpfer beinhalten, um z. B. Resonanz- und Geräuschprobleme zu unterbinden oder zu verbessern.

Der Nasenapplikator 100 der Fig. 6 weist optional eine vom Substanzreservoir R unterscheidbare Dosierkammer 13 auf. Sie dient dem Aufnehmen der Applikationsdosis der im Substanzreservoir R, für welche insbesondere jede hierin offenbarte Ausgestaltung vorgesehen sein kann, vorgehaltenen Substanz S, um sie anschließend abzugeben.

Die Dosierkammer 13 muss, um die Applikationsdosis abgeben zu können, zunächst mit dieser befüllt werden. Diese Aufgabe übernimmt in der Ausführungsform der Fig. 6 die Bestückungsvorrichtung 15.

Die Dosierkammer 13 kann zum Ausführen einer Abfolge eines Ansaug-Takts und eines Ausbring-Takts betätigt werden, wie nachfolgend beschrieben.

Die Entlüftung des Systems funktioniert identisch, nur dass es sich bei Luft um ein komprimierbares Medium handelt und die Substanz S ein nichtkomprimierbares Medium ist. Das System wird beim Entlüften den vollen Hubweg fahren und kann optional andere Parameterwerte für Geschwindigkeit, Kraft, Druck und/oder Beschleunigung für das Entlüften anstreben wie nach der Entlüftung und insbesondere für die Abgabe der Substanz S.

Wenn die Bestückungsvorrichtung 15 und/oder die Ausbringvorrichtung 17 vom oberen Totpunkt/Endpunkt/Startpunkt/Ausgangspunkt (auf der Seite des Einweg-Ventils 23a) bezogen auf die Ausrichtung in Fig. 6 weg bewegt wird, entsteht ein Unterdruck in der Dosierkammer 13. Dieser Unterdruck lässt aus dem Substanzreservoir R Substanz S in die Dosierkammer 13 einströmen. Der Stopfen 17a im Substanzreservoir R wird durch diesen Unterdruck ebenfalls nachgezogen und somit entsteht ein kollabierbares Behältnis (Ansaug-Takt). Hierbei schließt das Einweg-Ventil 23a, und das Einweg-Ventil 23b öffnen. Dies kann mechanisch, pneumatisch, hydraulisch, elektrisch, usw. erfolgen.

Alternativ zur Steuerung der Ventile 23a, 23b mittels Unterdrucks können die Ventile 23a, 23b elektrisch angesteuert werden. Dazu wird auf die Position der Bestückungsvorrichtung 15 zurückgegriffen.

Die Ventile 23a, 23b können auch in jeglicher Kombination der genannten Funktionsweisen ausgestaltet oder betrieben sein.

Zur Ausbringung der Substanz S (Ausbring-Takt) aus der Dosierkammer 13 heraus wird die Richtung der Bestückungsvorrichtung 15 umgekehrt, und sie wird hierdurch zur Ausbringvorrichtung 17. Diese verfährt mit einer vorbestimmten Geschwindigkeit, Kraft, Druck, Beschleunigung in Richtung zum Einweg-Ventil 23b. Bei der Wegumkehr entsteht ein Überdruck in der Dosierkammer 13, welcher das Einweg-Ventil 23a schließt und das Einweg-Ventil 23b öffnet. Dies ermöglicht es, dass die Substanz S über die Verbindungsstelle 4a durch den Nasenaufsatz 4 gedrückt wird. Die Ventilsteuerung kann wie oben beschreiben erfolgen.

Optional werden im Ansaug-Takt mit anderen Werten für Geschwindigkeit, Kraft, Druck und/oder Beschleunigung angelegt oder angestrebt als im Ausbring-Takt.

Die Dosierkammer 13 kann hochpräzise gefertigt sein, um eine minimale Variabilität in der Ausbringmenge zu verursachen, was ebenso für die Bestückungsvorrichtung 15 und die Ausbringvorrichtung 17 gelten kann.

Dies kann realisiert werden, indem ein Kunststoff mit geringem Schwund bzw. geringer Wasseraufnahme verwendet wird. Alternativ oder ergänzend kann, z. B. in Fig. 6, eine Hülse eingepresst sein, welche vorzugsweise sehr enge Toleranzen im Durchmesser aufweist. Dadurch kann das Ausbringvolumen besser kontrolliert werden. Das Ausbringvolumen ergibt sich aus der Formel:
Volumen = Fläche * Hub. Die Fläche ist jene eines Kreises A=d²*π/4.

Kunststoffe mit geringem Schwund bzw. geringer Wasseraufnahme können als "dimensionsstabile Kunststoffe" bezeichnet werden.

Die Dimensionsstabilität bezieht sich auf die Fähigkeit der Polymere, ihre Größe unter verschiedenen Umgebungsbedingungen beizubehalten. Ein dimensionsstabiler Kunststoff neigt daher weniger zur Feuchteaufnahme und zeigt eine geringere Wärmeausdehnung.

Zu den dimensionsstabilen Kunststoffen gehören Polymere wie PEEK, PPS, PSU, PPSU, PEI und PET.

Die Feuchteaufnahme kann nicht nur zur Änderung der Größe führen, sondern auch eine Änderung der Materialeigenschaften herbeiführen. Dies kann sich u. a. auf die mechanische Festigkeit und/oder auf elektrische Eigenschaften, wie die elektrische Leitfähigkeit und den dielektrischen Verlustfaktor, auswirken.

Ein Polymer ohne Wasseraufnahme ist z. B. PTFE. Folgende Polymere sind Kunststoffe mit sehr niedriger Wasseraufnahme: PEEK, PPS, PSU, PPSU, PEI, PVDF, PET, PPE, PP und PE. Eine geringe Wasseraufnahme wird auch bei POM, PA12, PC und ABS verzeichnet.

Die Dosierkammer 13, die Ausbringvorrichtung 17 und/oder die Bestückungsvorrichtung 15 können selbstdichtend sein und/oder eine Dichtung z. B. einen Dichtring 16, Kolbenringe, O-Ringe, Dichtlippen, usw. aufweisen.

Die Dosierkammer 13, und/oder beliebige Elemente, welche mit der Substanz in Berührung kommen wie die Ausbringvorrichtung 17, die Bestückungsvorrichtung 15 und/oder andere, können beschichtet sein. Eine mögliche Beschichtung kann z. B. aus Parylene sein und/oder aus inerten Materialien bestehen wie z. B. Glas und/oder aus technischen Kunststoffen wie z. B. Cycloolefin-Copolymere (COC), Cyclo-Olefin-Polymer (COP), usw.

Hier, wie auch in beliebigen anderen Ausführungsformen können die Dosierkammer 13, die Ausbringvorrichtung 17 und/oder die Bestückungsvorrichtung 15 aus Materialien bestehen oder solche aufweisen, welche keine Schmierung brauchen z. B. POM Homopolymer, Teflon, etc. Es können auch Schmiermittel wie z. B. Silikonöl eingesetzt werden oder Beschichtungen, mit denen die Reibung reduziert wird. In manchen Ausführungsformen können auch Additive zu den Kunststoffen hinzugeben werden, um die Gleiteigenschaften zu verbessern wie z. B. CORDULEN, Montanwachs, Hochmolekulares Polyethylen-Wachs (PE-LD, PE-HD), Fluorelastomere, etc.

In bestimmten Ausführungsformen können die Dosierkammer 13, die Ausbringvorrichtung 17 und/oder die Bestückungsvorrichtung 15 auch aus anderen Materialien wie Kunststoff bestehen oder solche aufweisen.

Wie aus vorstehenden Erläuterungen ersichtlich, ist die Dosierkammer 13 variabel, indem die Bestückungsvorrichtung 15 mal mehr und mal weniger Menge an Substanz S aus dem Substanzreservoir R abtrennt, wobei der Raum, in welchem die so abgetrennte Applikationsdosis vor ihrem Ausbringen vorliegt, als Dosierkammer 13 definiert sein kann.

Das Substanzreservoir R kann in eine Führung eingeklemmt, gedreht, gesteckt, befestigt bzw. im Gehäuse geführt werden oder sein.

Das Substanzreservoir R kann mit der Bestückungsvorrichtung 15, der Ausbringvorrichtung 17, dem Nasenaufsatz 4, dem Abgabedosierer 3 oder der Verbindungsstelle 4a verbunden sein. Die Verbindung kann mittels einer flexiblen oder starren Leitung 22 erfolgen. Die Leitung 22 kann ortsfest sein, oder mit der Ausbringvorrichtung 17 und/oder der Bestückungsvorrichtung 15 beweglich sein.

Das Substanzreservoir R kann mittels z. B. Luer/Lock, Pin, Nadel, Spike mit oder ohne Belüftung, etc. mit der Dosierkammer 13 verbunden sein.

Zum Herstellen einer Fluidverbindung zwischen der Dosierkammer 13 und dem Substanzreservoir R kann beispielsweise eine Nadel 38 vorgesehen sein, welche z. B. ein Septum 39 des Vial durchsticht.

Es kann ein Filter, z. B. ein (z. B. 22 µm) Partikelfilter oder eine poröse Membran (z. B. ein Porex^{®} - Filter), dazwischengeschaltet sein. Dieser Filter kann in manchen Ausführungsformen beim Ausgang der Dosierkammer 13 sein, ergänzend kann dort ein zweiter Filter vorgesehen sein.

Wie aus nachfolgenden Erläuterungen klar wird, kann die Bestückungsvorrichtung 15 identisch mit der Ausbringvorrichtung 17 sein, sie kann allerdings auch getrennt hiervon vorgesehen sein.

Obgleich die Ausbringvorrichtung 17 und/oder die Bestückungsvorrichtung 15 einen Energiespeicher für mechanische Energie oder Federenergie und/oder hydraulische und pneumatische Energie als Energiebeaufschlagungsmechanismus aufweisen kann, ist dies in der Ausführungsform der Fig. 6 nicht der Fall.

Der elektrische Energiespeicher 20 kann in die Ausbringvorrichtung 17 und/oder die Bestückungsvorrichtung 15 integriert sein oder mittels eines externen Anschlusses mit diesen verbunden sein.

Der integrierte Energiespeicher 20 kann für eine Auslösung geladen sein und nach jeder Applikation mittels eines Ladeteils, z. B. eines Qi-Ladepads, oder in einer Gehäuseschale mittels PIN-Anschluss, geladen werden.

Der elektrische Energiespeicher 20 kann ein Kondensator oder Ähnliches sein.

Die Stromversorgung kann per Stromkabel, Netzteil, usw. oder über Ethernet (LAN), englisch Power over Ethernet (PoE), erfolgen. Letzteres bezeichnet ein Verfahren, mit dem netzwerkfähige Geräte über das achtadrige Ethernet-Kabel mit Strom versorgt werden können. Eine solche Versorgung kann hierin vorgesehen sein. In manchen Ausführungsformen können hierfür alternativ Solarzellen oder Mechanismen für ein *Energy Harvesting* vorgesehen sein.

In Fig. 6 sind die Ausbringvorrichtung 17 und/oder die Bestückungsvorrichtung 15 als Elektroantrieb, hier mit Elektromotor 19 mit Spindel 21, welche gekoppelt sind, ausgestaltet.

In anderen Ausführungsformen kann diese Kopplung auch über ein Getriebe erfolgen bzw. mit einer Übersetzung ausgestaltet sein.

Mit Blick auf Fig. 6 sei darauf hingewiesen, dass in einigen Ausführungsformen, wie z. B. jener der Fig. 6, die Ausbringvorrichtung 17 und die Bestückungsvorrichtung 15 durch einen gemeinsamen Mechanismus verwirklicht sein kann, wobei der Mechanismus z. B., wenn er in einer ersten Richtung (etwa weg von der Verbindungsstelle 4a) verfährt, die Dosierkammer 13 als Bestückungsvorrichtung 15 befüllt, und, wenn er in einer zweiten Richtung, welcher zur ersten entgegengesetzt sein kann (etwa auf die Verbindungsstelle 4a zu) verfährt, den Inhalt der Dosierkammer 13 z. B. über das Nasenstück als Ausbringvorrichtung 17 ausbringt. Eine gegensinnige Rotation von Spindel 21 und/oder Motor 19 bei Ansaugen einerseits bzw. Applizieren andererseits kann vorgesehen sein, wie dies auch an anderer Stelle hierin bereits beschrieben ist. Alternativ ist eine gleichsinnige Rotation sowohl für Befüllen als auch für Leeren der Dosierkammer 13 möglich, wie vorstehend z. B. mit Bezug auf den Schubkurbelantrieb ausgeführt. Ebenso sind ein Befüllen und ein Entleeren unter gegenseitiger Rotation bei Einsatz des Schubkurbelantriebs möglich, wie vorstehend beschrieben.

Die Ausbringvorrichtung 17 und/oder die Bestückungsvorrichtung 15 sind mit der Spindel 21 und/oder dem Motor 19 lösbar oder unlösbar verbunden.

Das Substanzreservoir R, die Ausbringvorrichtung 17 und/oder die Bestückungsvorrichtung 15 können als Einwegprodukte ausgelegt sein.

Die Ausbringvorrichtung 17, die Bestückungsvorrichtung 15 kann als Mehrwegprodukt ausgelegt sein.

Beide optional vorgesehenen Gehäuseschalen können als Einwegprodukte ausgelegt sein.

Die elektronische Steuervorrichtung 9, der Motor 19, die Spindel 21 und/oder andere Bauteile können wiederverwendbar sein.

Der Nasenapplikator 100 der Fig. 6 weist ferner einen optionalen Mechanismus zum Verändern des Fassungsvolumens der Dosierkammer 13 auf. Mittels dieses Mechanismus kann das Fassungsvolumen der Dosierkammer 13 für eine Teilmenge der Substanz S variiert werden, wodurch es möglich ist, unterschiedlich große Applikationsdosen zu applizieren.

In manchen Ausführungsformen ist dieser Mechanismus Teil des Abgabedosierers 3, in anderen wie der vorliegenden nicht.

Die elektronische Steuervorrichtung 9, siehe Fig. 1, falls vorgesehen, kann programmiert sein, um die Ausbringvorrichtung 17 mit einer Vielzahl von Geschwindigkeiten, Kräften, Drücken, Beschleunigungen, Flussraten zu verfahren und/oder ausbringen zu lassen.

Mit einer motorgetriebenen, nasalen Vorrichtung kann das Problem gelöst werden, dass beim Auslösen per Hand durch Drücken jeder Nutzer eine andere Geschwindigkeit, Kraft, Druck, Beschleunigung aufbringt. Es ist nicht garantiert, dass jeder Nutzer die gleiche Geschwindigkeit, z. B. Auslösegeschwindigkeit bzw. die ideale Auslösegeschwindigkeit erzeugt. Da die Auslösegeschwindigkeit den größten Einfluss auf die Tröpfchengrößenverteilung, das Spray Pattern und die Plume Geometry hat, ist eine zwischen Nutzern stets identische Auslösung von besonderem Vorteil, da somit im Vergleich zu einer manuellen Auslösung immer ein gleichbleibendes Sprühbild insbesondere die Tröpfchengrößenverteilung, das Spray Pattern und die Plume Geometry und Ausbringvolumen gesichert ist.

Auf diese Weise kann vorteilhaft sichergestellt sein, dass beim Applizieren der nächsten Applikationsdosis stets der hierfür erforderliche, also komplette, Hubweg zurückgelegt wird. Wenn beispielsweise für eine 150 µL Dosis 5mm Weg zurücklegt werden muss, dann kann wie hierin beschrieben sichergestellt sein, dass nicht nur z. B. 4mm zurückgelegt werden, was bei einer manuellen, auf die Druckstärke oder - tiefe abgestellten Lösung möglich sein könnte, sondern, aufgrund der hierin beschriebenen Optionen, immer der komplett benötigte Hubweg zurückgelegt wird.

Ergänzend oder alternativ kann die elektronische Steuervorrichtung 9 programmiert sein, um auf den Mechanismus zum Verändern des Fassungsvolumens der Dosierkammer 13 einzuwirken. Dies kann auch manuell und die Auslösung dennoch automatisch erfolgen.

Die Tröpfchengrößenverteilung, das Spray Pattern, die Plume Geometry kann vorzugsweise angepasst werden, und optional für jede Ausbringmenge unterschiedlich angepasst sein.

Je nach Wirkstoff und dessen Eigenschaften wie z. B. Viskosität, Oberflächenspannung, etc. kann eine unterschiedliche Geschwindigkeit, eine unterschiedliche Kraft, ein unterschiedlicher Druck, eine unterschiedliche Beschleunigung, eine unterschiedliche Flussrate erforderlich sein. Das Gerät kann in manchen Ausführungsformen für jede Dosismenge eine eigene Geschwindigkeit, Kraft, Beschleunigung, Flussrate oder einen eigenen Druck fahren. Diese kann z. B. vorab eingestellt sein oder werden.

In bestimmten Ausführungsformen kann ein Sensor die Lage bzw. Auslöseposition, z. B. senkrecht oder waagrecht (und alles dazwischen), erkennen und dann die Geschwindigkeit, Kraft, Druck, Beschleunigung an diese Lage jeweils anpassen.

In der Ausführungsform der Fig. 6 weist die Bestückungsvorrichtung 15 zum Bestücken der Dosierkammer 13 mit der Applikationsdosis der Substanz S wenigstens ein erstes Einweg-Ventil 23a oder Rückschlagventil aufweist und/oder die Dosierkammer 13 durch wenigstens ein erstes Einweg-Ventil 23a oder Rückschlagventil begrenzt ist. Dieses Ventil kann z. B. in einem Kolben selbst angeordnet sein oder an beliebiger Stelle, z. B. an beliebiger Stelle in der Zuleitung vom Substanzreservoir R zur Dosierkammer 13.

Die Bestückungsvorrichtung 15 weist in der Ausführungsform der Fig. 6 wenigstens ein zweites Einweg-Ventil 23b oder Rückschlagventil auf.

Alternativ ist z. B. das erste Ventil 23a als schaltbares Dreiwegeventil und/oder auf wiederum andere Weise ausgeführt. Damit kann auch das erste Ventil 23a als singuläres Element die Funktion von zwei Ventilen ausführen.

Die Dosierkammer 13 ist hierbei exemplarisch durch wenigstens das zweite Einweg-Ventil 23b oder Rückschlagventil begrenzt. Optional weist das zweite Rückschlagventil 23b eine bezogen auf die Dosierkammer 13 entgegengesetzte Öffnungsrichtung auf als das erste Rückschlagventil 23a.

Optional können die Funktion des ersten Einweg-Ventils 23a und die Funktion des zweiten Einweg-Ventils 23b in ein gemeinsames Bauteil, also z. B. in ein Ventilelement, zusammengelegt sein.

Die Dosierkammer 13 kann optional über ein hier nicht dargestelltes Entlüftungsventil mit der Atmosphäre in Verbindung stehen oder gebracht werden. Es kann vorgesehen sein, über ein solches Entlüftungsventil die anfängliche Entlüftung des Systems zu betreiben und/oder zu einem späteren Zeitpunkt Luftblasen zu eliminieren. Ein Filter kann an geeigneter Stelle vorgesehen sein.

Das Gerät besteht optional aus zwei Gehäuseteilen, nämlich einem Gehäuseoberteil 2b und einem Gehäuseunterteil 2c, welche in Fig. 6 durch eine Gehäusetrennfuge 2d getrennt sind.

Das Gehäuseoberteil 2b beinhaltet in der schematischen Darstellung der Fig. 6 das Substanzreservoir R, die Dosierkammer 13, die Ausbringvorrichtung 17 und/oder die Bestückungsvorrichtung 15. Ventile, Filter, Entlüftung, die Verbindungsstelle 4a für den Nasenaufsatz 4 usw. können ebenfalls im Gehäuseoberteil 2b vorgesehen sein.

Das Gehäuseunterteil 2c weist die elektronische Steuervorrichtung 9, den Motor 19, die Spindel 21, den optionalen Encoder, Controller, die Bremse, den Dämpfer, den Energiespeicher 20, die Abrufvorrichtung 1, etc. auf.

Vorstehend genannte Komponenten sind nur exemplarisch angeordnet wie in Fig. 6 gezeigt. Sie können in jeder beliebigen Kombination angeordnet sein.

Eine oder beide der Gehäuseschalen oder -teile 2b, 2c können Einwegprodukte sein. Der Nasenapplikator 100 kann komplett zusammengebaut ausgeliefert, sterilisiert, umpackt vorliegen bzw. ausgeliefert werden. Alternativ wird er vor Ort, am Patientenbett, vom Patienten, vom Arzt usw. erst komplettiert. So kann z. B. das Substanzreservoir R noch in die Gehäuseoberschale einzulegen sein und Letztere dann mit der Gehäuseunterschale zusammengefügt werden.

Die elektronische Steuervorrichtung 9, der Motor 19, die Spindel 21, der optionale Encoder, der Controller, die Bremse, der Dämpfer, der Energiespeicher 20 und/oder die Abrufvorrichtung 1, können, in beliebiger Kombination miteinander, eine Einheit bilden. Sie können in ein Gehäuseunterteil 2c einlegt werden oder sein, welches dann zusammen mit dem Gehäuseoberteil 2b das Gehäuse 2 bildet. Optional könnte man bei Demontage die Gehäuseschalen (Ober- und Unterteil) 2b, 2c öffnen und entsorgen. Die Elektronik kann aus der Unterschale oder dem Gehäuseunterteil 2c, ohne diese zu berühren, entnommen werden.

Die Gehäuseteile 2b, 2c können allein oder gemeinsam nach Abschluss der Behandlung des Nutzers z. B. auch absichtlich zerstört werden, um die weitere Nutzung des Nasenapplikators zu verhindern. Hierzu kann vorgesehen sein, z. B. einen optionalen Schnapphaken abzubrechen oder eine andere formschlüssige Verbindung irreversibel zu schädigen, abzubrechen, zu verschieben, usw. Man deformiert beispielsweise den Schnapphaken, man verschiebt ein Verbindungsteil oder eine Sperre, usw.

Der Nasenapplikator 100 kann nach Behandlung automatisch und/oder manuell entleert und als Ganzes verworfen werden. Alternativ kann er in Teile zerlegt werden und nur teilweise verworfen bzw. wiederaufbereitet oder weiterverwendet werden, hierzu sind insbesondere z. B. die elektronische Steuervorrichtung 9, der Motor 19, die Spindel 21, usw. geeignet.

Der Nasenapplikator 100 kann einen Deckel umfassen, welcher zum Einlegen des Substanzreservoirs R und/oder der elektronische Steuervorrichtung 9 geöffnet werden kann.

Ein Dichtring 16 kann vorgesehen sein, um die Dichtigkeit zwischen Bestückungsvorrichtung 15 und/oder Ausbringvorrichtung 17 einerseits und umgebender Hülse oder Schaft andererseits zu gewährleisten.

Ein Durchflusssensor 11a kann vorgesehen sein. Er kann Teil der Erfassungsvorrichtung sein oder diese darstellen.

Ein optionaler Stopfen 17a ist zur Begrenzung des Volumens des Subtanzreservoirs R vorgesehen, er kann verschieblich angeordnet sein.

Eine optionale Feder kann vorgesehen sein in dieser oder jeder anderen Ausführungsform, welcher direkt oder indirekt gegen den Stopfen 17a drückt, um ihn aus einer Position, welche er lagerungsbedingt möglicherweise über eine längere Zeitdauer angenommen hat, bei Auslösen des Nasenapplikators 100 herauszubewegen.

Das in Fig. 6 gezeigte Ventil 23a kann sich in beliebigen Ausführungsformen optional bei, an und/oder in der Ausbringvorrichtung 17 und/oder der Bestückungsvorrichtung 15 oder in deren unmittelbaren Nähe befinden.

Optional kann in beliebigen Ausführungsformen die Leitung 22, durch welche die Substanz S in die Dosierkammer 13 geführt wird, durch die Bestückungsvorrichtung 15 und/oder die Ausbringvorrichtung 17 verlaufen.

Optional können in beliebigen Ausführungsformen die Bestückungsvorrichtung 15 und/oder die Ausbringvorrichtung 17 einen Anschluss für die Leitung 22 haben.

Optional können in beliebigen Ausführungsformen die Bestückungsvorrichtung 15 und/oder die Ausbringvorrichtung 17 hohl oder durchgängig sein.

Optional können sich in beliebigen Ausführungsformen die Leitung 22 mit der Bestückungsvorrichtung 15 und/oder der Ausbringvorrichtung 17 mitbewegen. In anderen Ausführungsformen bewegt sich die Leitung 22 nicht mit der Bestückungsvorrichtung 15 und/oder der Ausbringvorrichtung 17.

Optional kann das Substanzreservoir R in beliebigen Ausführungsformen um 180 Grad gegenüber der Darstellung der Fig. 6 gedreht eingebaut werden.

Alle Bauteile können in beliebigen Ausführungsformen optional beliebig positioniert werden, um eine Blindmontage, also ein Einbau ohne Sicht, zu vermeiden, die Gefahr von Verletzungen durch die Nadel 38 verringert wird und dass das Substanzreservoir R, welches das Septum 39 oder einen anderweitigen Anschluss aufweisen kann, einfacher montiert werden kann.

### Bezugszeichen

- 100: Nasenapplikator
- 101: Peripheriegerät
- 1: Abrufvorrichtung
- 2: Gehäuse
- 2b: Gehäuseoberteil
- 2c: Gehäuseunterteil
- 2d: Gehäusetrennfuge
- 3: Abgabedosierer
- 4: Nasenaufsatz
- 4a: Verbindungsstelle
- 5: Sperrvorrichtung
- 7: Dosiserfassungsvorrichtung
- 8: Feedbackvorrichtung
- 9: Elektronische Steuervorrichtung
- 11: Erfassungsvorrichtung
- 11a: Flusssensor (Durchflusssensor)
- 12: Anzeige
- 13: Dosierkammer
- 15: Bestückungsvorrichtung
- 16: Dichtung oder Dichtring
- 17: Ausbringvorrichtung
- 17a: Stopfen
- 19: E-Motor
- 20: Energiespeicher
- 21: Spindel
- 22: Leitung
- 23a: erstes Einwegventil, Rückschlagventil
- 23b: zweites Einwegventil, Rückschlagventil
- 38: Nadel
- 39: Septum
- C_{ED1}: Zielkonzentration, für den gewünschten Effekt/Wirkung erforderliche Konzentration zum Betätigungszeitpunkt tB₁
- C_{ED2}: Zielkonzentration, für den gewünschten Effekt/Wirkung erforderliche Konzentration zum Betätigungszeitpunkt tB₂
- C_{ED3}: Zielkonzentration, für den gewünschten Effekt/Wirkung erforderliche Konzentration zum Betätigungszeitpunkt tB₃
- C_{ED1_+SD}: Erste festgesetzte Zielkonzentration, für den gewünschten Effekt/Wirkung erforderliche Konzentration auf der PK-Kurve PK50_{D0_+SD} zum Betätigungszeitpunkt tB₁
- C_{ED1_M}: Erste festgesetzte Zielkonzentration, für den gewünschten Effekt/Wirkung erforderliche Konzentration auf der PK-Kurve PK50_{D0_M} zum Betätigungszeitpunkt tB₁
- C_{ED1_-SD}: Erste festgesetzte Zielkonzentration, für den gewünschten Effekt/Wirkung erforderliche Konzentration auf der PK-Kurve PK50_{D0_-SD} zum Betätigungszeitpunkt tB₁
- C_{ED2_+SD}: Zweite festgesetzte oder erste geschätzte Zielkonzentration, für den gewünschten Effekt/Wirkung erforderliche Konzentration auf der PK-Kurve PK30_{D1_+SD} zum Betätigungszeitpunkt tB₂
- C_{ED2_M}: Zweite festgesetzte oder erste geschätzte Zielkonzentration, für den gewünschten Effekt/Wirkung erforderliche Konzentration auf der PK-Kurve PK30_{D1_M} zum Betätigungszeitpunkt tB₂
- C_{ED2_-SD}: Zweite festgesetzte oder erste geschätzte Zielkonzentration, für den gewünschten Effekt/Wirkung erforderliche Konzentration auf der PK-Kurve PK30_{D1_-SD} zum Betätigungszeitpunkt tB₂
- C'_{ED1}: Erste vor dem Abgabezeitpunkt tB₁ ermittelte Zielkonzentration, für den gewünschten Effekt/Wirkung erforderliche Konzentration auf der PK-Kurve PK50_{D0}
- C'_{E1_+SD}: Erste vor dem Abgabezeitpunkt tB₁ ermittelte Zielkonzentration, für den gewünschten Effekt/Wirkung erforderliche Konzentration auf der PK-Kurve PK50_{D0_+SD}
- C'_{ED1_M}: Erste vor dem Abgabezeitpunkt tB₁ ermittelte Zielkonzentration, für den gewünschten Effekt/Wirkung erforderliche Konzentration auf der PK-Kurve PK50_{D0_M}
- C'_{ED1_-SD}: Erste vor dem Abgabezeitpunkt tB₁ ermittelte Zielkonzentration, für den gewünschten Effekt/Wirkung erforderliche Konzentration auf der PK-Kurve PK50_{D0_-SD}
- C(t): Konzentration zum Zeitpunkt t
- Cₙₘₐₓ: Konzentrationsmaximum
- C₁ₘₐₓ: Erstes Maximum auf der PK-Kurve PK50_{D0}
- C₂ₘₐₓ: Zweites Maximum auf der PK-Kurve PK25_{D1}
- C₃ₘₐₓ: Drittes Maximum auf der PK-Kurve PK25_{D2}

- C_{1max_+SD}: Erstes Konzentrationsmaximum auf der PK-Kurve PK50_{D0_+SD}
- C_{1max_M}: Erstes Konzentrationsmaximum auf der PK-Kurve PK50_{D0_M}
- C_{1max_-SD}: Erstes Konzentrationsmaximum auf der PK-Kurve PK50_{D0_-SD}

- C_{2max_+SD}: Zweites Konzentrationsmaximum auf der PK-Kurve PK30_{D1_+SD}
- C_{2max_M}: Zweites Konzentrationsmaximum auf der PK-Kurve PK30_{D1_M}
- C_{2max_-SD}: Zweites Konzentrationsmaximum auf der PK-Kurve PK30_{D1_-SD}

- D: Dosis
- D₀: Ausgangsdosis; Initialdosis
- D₁: Erste Applikationsdosis, vorangehende Applikationsdosis
- D₂: Zweite Applikationsdosis, nachfolgende Applikationsdosis
- D₃: Dritte Applikationsdosis
- Dₙ₋₁: Die der nächsten Applikationsdosis vorangehende Applikationsdosis
- Dₙ: Nächste Applikationsdosis
- Dₙ₊₁: Die der nächsten Applikationsdosis nachfolgende oder übernächste Applikationsdosis
- M: Datenspeicher

- PK25: PK-Kurve für 25 µg Fentanyl Einzeldosis
- PK50: PK-Kurve für 50 µg Fentanyl Einzeldosis

- PK25_{Dn}: PK-Kurve für 25 µg Fentanyl Mehrfachdosis
- PK50_{Dn}: PK-Kurve für 50 µg Fentanyl Mehrfachdosis

- PK50_{+SD}: PK-Kurve für 50 µg Fentanyl Einzeldosis, Mittelwert über alle betrachteten Individuen des Kollektivs zuzüglich einer Standardabweichung

- PK50_{M}: PK-Kurve für 50 µg Fentanyl Einzeldosis, Mittelwert über alle betrachteten Individuen des Kollektivs
- PK50_{-SD}: PK-Kurve für 50 µg Fentanyl Einzeldosis, Mittelwert über alle betrachteten Individuen des Kollektivs abzüglich einer Standardabweichung
- PK50_{Dn_+SD}: PK-Kurve für 50 µg Fentanyl Mehrfachdosis, Mittelwert über alle betrachteten Individuen des Kollektivs zuzüglich einer Standardabweichung
- PK50_{Dn_M}: PK-Kurve für 50 µg Fentanyl Mehrfachdosis, Mittelwert über alle betrachteten Individuen des Kollektivs
- PK50_{Dn_-SD}: PK-Kurve für 50 µg Fentanyl Mehrfachdosis, Mittelwert über alle betrachteten Individuen des Kollektivs abzüglich einer Standardabweichung
- PK30_{Dn_+SD}: PK-Kurve für 30 µg Fentanyl Mehrfachdosis, Mittelwert über alle betrachteten Individuen des Kollektivs zuzüglich einer Standardabweichung
- PK30_{Dn_M}: PK-Kurve für 30 µg Fentanyl Mehrfachdosis, Mittelwert über alle betrachteten Individuen des Kollektivs
- PK30_{Dn_-SD}: PK-Kurve für 30 µg Fentanyl Mehrfachdosis, Mittelwert über alle betrachteten Individuen des Kollektivs abzüglich einer Standardabweichung
- PK_{Ind}: Individuelle PK-Kurve
- R: Substanzreservoir
- S: medizinische Substanz

- T_vainₙ: Sperrdauern
- T_vain_{n_S}: Beginn der Sperrdauer
- T_vain_{n_E}: Ende der Sperrdauer

- tA₀: Ausgangszeitpunkt; Abgabezeitpunkt der Ausgangsdosis; Initialdosis

- tA₁,..., tAₙ, tAₙ₊₁: Abgabezeitpunkt 1 bis n+1
- tA_{2_+SD}: Abgabezeitpunkt Mittelwert über alle betrachteten Individuen des Kollektivs zuzüglich einer Standardabweichung
- tA_{2_M}: Abgabezeitpunkt Mittelwert über alle betrachteten Individuen des Kollektivs
- tA_{2_-SD}: Abgabezeitpunkt Mittelwert über alle betrachteten Individuen des Kollektivs abzüglich einer Standardabweichung
- tB₁,..., tBₙ, tBₙ₊₁: Betätigungszeitpunkt 1 bis n+1
- tB_{2_+SD}, tB_{2_M}, tB_{2_-SD}: Berechneter Zielkonzentrationszeitpunkt
- tB_{1_v1}, tB_{2_v1}: Vergebliche Betätigungszeitpunkte während der Sperrdauer T_vain₁
- tB_{1_v2}, tB_{2_v2}: Vergebliche Betätigungszeitpunkte während der Sperrdauer T_vain₂
- tB_{m_vn}: Vergebliche Betätigungszeitpunkte während der Sperrdauer T_vainₙ

- tF_{n_1}: Erster Feedbackzeitpunkt
- tF_{n_2}, tF_{n_3}: Feedbackzeitpunkte
- tF_{n_4}: Letzter Feedbackzeitpunkt

- tV₁,..., tVₙ, tVₙ₊₁: Vorbestimmungszeitpunkte 1 bis n+1

## Patentansprüche

1. Nasenapplikator (100) zum nasalen Verabreichen wenigstens einer medizinischen Substanz (S), insbesondere eines Schmerzmittels oder eines Impfstoffs, mit einem Gehäuse (2), aufweisend oder verbunden jeweils mit
- eine Verbindungsstelle für ein Substanzreservoir (R) zum Vorhalten einer Menge der Substanz (S), oder einem Substanzreservoir (R) zum Vorhalten einer Menge der Substanz (S), und/oder eine Verbindungsstelle für eine unter Druck stehende Gaskartusche, oder eine unter Druck stehende Gaskartusche, und/oder eine Verbindungsstelle (4a) für einen Nasenaufsatz (4), oder einem Nasenaufsatz (4) oder einem Nasenstück;
- einer Abrufvorrichtung (1) zum Betätigen durch den Benutzer mit dem Ziel, eine nächste Applikationsdosis (Dₙ) der Substanz (S) abzurufen;
- einem Abgabedosierer (3) zum Abgeben von Applikationsdosen (D₀, D₁, D₂, ..., Dₙ₋₁, D_{n,} Dₙ₊₁, ...) auf eine Betätigung der Abrufvorrichtung (1) hin zu jeweils einem Abgabezeitpunkt (tA₀, tA₁, tA₂, ..., tAₙ₋₁, tAₙ, tAₙ₊₁, ...); und
- einer Ausbringvorrichtung (17) zum Ausbringen einer Applikationsdosis der Substanz (S) über die Verbindungsstelle (4a) für den Nasenaufsatz (4) oder den Nasenaufsatz (4) oder das Nasenstück und/oder aus dem Nasenapplikator (100) hinaus.

2. Nasenapplikator (100) nach Anspruch 1, weiter aufweisend
- eine Dosierkammer (13) zum vorübergehenden Aufnehmen der Applikationsdosis der in der Substanzreservoir (R) vorgehaltenen Substanz (S) oder zum Aufnehmen von Gas, insbesondere aus der Umgebung oder aus der Gaskartusche; und
- eine Bestückungsvorrichtung (15) zum Bestücken der Dosierkammer (13) mit der Applikationsdosis der Substanz (S) aus dem Substanzreservoir (R) oder mit Gas, beispielsweise nach Ansaugen aus der Umgebung oder aus der Gaskartusche.

3. Nasenapplikator (100) nach einem der vorangegangenen Ansprüche, wobei der Nasenaufsatz (4) ein Nasenaufsatz-Reservoir zum Vorhalten einer Menge der Substanz (S) ist oder aufweist.

4. Nasenapplikator (100) nach einem der vorangegangenen Ansprüche, wobei der Nasenaufsatz (4) ein Einweg-Nasenaufsatz ist.

5. Nasenapplikator (100) nach einem der vorangegangenen Ansprüche, wobei die Ausbringvorrichtung (17) angeordnet ist zum Ausbringen von der in der Dosierkammer (13) oder in dem Substanzreservoir (R) oder in dem Nasenaufsatz (4) vorliegenden Applikationsdosis der Substanz (S), oder von Gas, etwa aus der Umgebung oder aus der Gaskartusche, über die Verbindungsstelle (4a), den Nasenaufsatz (4) oder das Nasenstück und/oder aus dem Nasenapplikator (100) hinaus.

6. Nasenapplikator (100) nach einem der vorangegangenen Ansprüche, wobei das Substanzreservoir (R) oder das Nasenaufsatz-Reservoir einen Impfstoff oder Phagen enthält.

7. Nasenapplikator (100) nach Anspruch 6, wobei der Impfstoff ein DNA-, mRNA- oder Vektorimpfstoff, Phagen, und/oder ein SARS-CoV-2- oder Influenza-Impfstoff ist.

8. Nasenapplikator (100) nach einem der vorangegangenen Ansprüche, weiter aufweisend
- einen Mechanismus zum Verändern des Fassungsvolumens der Dosierkammer (13) für die Applikationsdosis der Substanz (S).

9. Nasenapplikator (100) nach einem der vorangegangenen Ansprüche, weiter aufweisend
- eine elektronische Steuervorrichtung (9).

10. Nasenapplikator (100) nach Anspruch 9, wobei die elektronische Steuervorrichtung (9) programmiert ist, um zum Applizieren einer vorbestimmten Dosismenge x als Applikationsdosis eine Menge, die größer als x ist, aus dem Substanzreservoir (R) und/oder aus der Dosierkammer (13) zu fördern.

11. Nasenapplikator (100) nach einem der vorangegangenen Ansprüche, weiter aufweisend
- eine Einweg-Schutzhülle zum Aufsetzen auf den Nasenaufsatz (4), insbesondere wenn als Nasenaufsatz-Reservoir ausgestaltet.

12. Nasenapplikator (100) nach Anspruch 11, wobei die Einweg-Schutzhülle, vorzugsweise endseitig oder stirnseitig, einen Filter aufweist.

13. System, aufweisend
- wenigstens einen Nasenapplikator (100) nach einem der vorangegangenen Ansprüche; und
- weiter aufweisend wenigstens eine Einweg-Schutzhülle zum Aufsetzen auf den Nasenaufsatz (4) oder wenigstens einen Nasenaufsatz (4) als Einweg-Nasenaufsatz.
